# EUROPEAN PATENT APPLICATION

(11) **EP 1 466 624 A1**
(43) Date of publication of application: **13.10.2004**
(21) Application number: 02790728.6
(22) Date of filing: 12.12.2002
(51) Int. Cl.: A61K 45/00, A61K 38/00, A61P 1/16, A61P 9/00, A61P 11/00, A61P 13/12, A61P 43/00

(54) **DRUG FOR REGENERATING TISSUE AND VESSEL AND METHOD THEREFOR**

(30) Priority: 13.12.2001 JP 2001380158; 04.12.2002 JP 2002352924
(71) Applicant: Japan Tobacco, Inc., Tokyo 105-8422 (JP)
(72) Inventor: NAKAMURA, Motonao, Yokohama-shi, Kanagawa (JP); HIGUCHI, Toshio, Yokohama-shi, Kanagawa (JP); YAMASAKI, Yoshiki, Yokohama-shi, Kanagawa (JP); ORITA, Takuya, Yokohama-shi, Kanagawa (JP)
(74) Representative: VOSSIUS & PARTNER
(86) International application number: PCT/JP2002/013014
(87) International publication number: WO 2003/053467

(57) **Abstract**

The present invention revealed that the functional expression of hepatocyte growth factor (HGF) /HGF receptor is suppressed through the binding of the HGF receptor to a novel mucin-like protein, leading to the inhibition of HGF dependent cell proliferation and regeneration/neogenesis of tissues and blood vessels. Furthermore, inhibition of the binding between the HGF receptor and the mucin-like protein was found to enable treatment of various diseases including liver diseases.

SEQUENCE LISTING

<110> JAPAN TOBACCO, INC.

<120> PHARMACEUTICAL AGENTS AND METHODS FOR TISSUE AND VASCULAR
REGENERATION

<130> J1-A0205P

<140> PCT/JP02/13014
<141> 2002-12-12

<150> JP 2001-380158
<151> 2001-12-13

<150> JP 2002-352924
<151> 2002-12-04

<160> 57

<170> PatentIn Ver. 2.1

<210> 1
<211> 4173
<212> DNA
<213> Homo sapiens

<220>

<221> CDS

<222> (1)..(4173)

<220>

<221> sig_peptide

<222> (1)..(72)

<220>

<221> mat_peptide

<222> (73)..(4170)

<400> 1
atg aag gcc ccc gct gtg ctt gca cct ggc atc ctc gtg ctc ctg ttt 48
acc ttg gtg cag agg agc aat ggg gag tgt aaa gag gca cta gca aag 96
tcc gag atg aat gtg aat atg aag tat cag ctt ccc aac ttc acc gcg 144
gaa aca ccc atc cag aat gtc att cta cat gag cat cac att ttc ctt 192
ggt gcc act aac tac att tat gtt tta aat gag gaa gac ctt cag aag 240
gtt gct gag tac aag act ggg cct gtg ctg gaa cac cca gat tgt ttc 288
cca tgt cag gac tgc agc agc aaa gcc aat tta tca gga ggt gtt tgg 336
aaa gat aac atc aac atg gct cta gtt gtc gac acc tac tat gat gat 384
caa ctc att agc tgt ggc agc gtc aac aga ggg acc tgc cag cga cat 432
gtc ttt ccc cac aat cat act gct gac ata cag tcg gag gtt cac tgc 480
ata ttc tcc cca cag ata gaa gag ccc agc cag tgt cct gac tgt gtg 528
gtg agc gcc ctg gga gcc aaa gtc ctt tca tct gta aag gac cgg ttc 576
atc aac ttc ttt gta ggc aat acc ata aat tct tct tat ttc cca gat 624
cat cca ttg cat tcg ata tca gtg aga agg cta aag gaa acg aaa gat 672
ggt ttt atg ttt ttg acg gac cag tcc tac att gat gtt tta cct gag 720
ttc aga gat tct tac ccc att aag tat gtc cat gcc ttt gaa agc aac 768
aat ttt att tac ttc ttg acg gtc caa agg gaa act cta gat gct cag 816
act ttt cac aca aga ata atc agg ttc tgt tcc ata aac tct gga ttg 864
cat tcc tac atg gaa atg cct ctg gag tgt att ctc aca gaa aag aga 912
aaa aag aga tcc aca aag aag gaa gtg ttt aat ata ctt cag gct gcg 960
tat gtc agc aag cct ggg gcc cag ctt gct aga caa ata gga gcc agc 1008
ctg aat gat gac att ctt ttc ggg gtg ttc gca caa agc aag cca gat 1056
tct gcc gaa cca atg gat cga tct gcc atg tgt gca ttc cct atc aaa 1104
tat gtc aac gac ttc ttc aac aag atc gtc aac aaa aac aat gtg aga 1152
tgt ctc cag cat ttt tac gga ccc aat cat gag cac tgc ttt aat agg 1200
aca ctt ctg aga aat tca tca ggc tgt gaa gcg cgc cgt gat gaa tat 1248
cga aca gag ttt acc aca gct ttg cag cgc gtt gac tta ttc atg ggt 1296
caa ttc agc gaa gtc ctc tta aca tct ata tcc acc ttc att aaa gga 1344
gac ctc acc ata gct aat ctt ggg aca tca gag ggt cgc ttc atg cag 1392
gtt gtg gtt tct cga tca gga cca tca acc cct cat gtg aat ttt ctc 1440
ctg gac tcc cat cca gtg tct cca gaa gtg att gtg gag cat aca tta 1488
aac caa aat ggc tac aca ctg gtt atc act ggg aag aag atc acg aag 1536
atc cca ttg aat ggc ttg ggc tgc aga cat ttc cag tcc tgc agt caa 1584
tgc ctc tct gcc cca ccc ttt gtt cag tgt ggc tgg tgc cac gac aaa 1632
tgt gtg cga tcg gag gaa tgc ctg agc ggg aca tgg act caa cag atc 1680
tgt ctg cct gca atc tac aag gtt ttc cca aat agt gca ccc ctt gaa 1728
gga ggg aca agg ctg acc ata tgt ggc tgg gac ttt gga ttt cgg agg 1776
aat aat aaa ttt gat tta aag aaa act aga gtt ctc ctt gga aat gag 1824
agc tgc acc ttg act tta agt gag agc acg atg aat aca ttg aaa tgc 1872
aca gtt ggt cct gcc atg aat aag cat ttc aat atg tcc ata att att 1920
tca aat ggc cac ggg aca aca caa tac agt aca ttc tcc tat gtg gat 1968
cct gta ata aca agt att tcg ccg aaa tac ggt cct atg gct ggt ggc 2016
act tta ctt act tta act gga aat tac cta aac agt ggg aat tct aga 2064
cac att tca att ggt gga aaa aca tgt act tta aaa agt gtg tca aac 2112
agt att ctt gaa tgt tat acc cca gcc caa acc att tca act gag ttt 2160
gct gtt aaa ttg aaa att gac tta gcc aac cga gag aca agc atc ttc 2208
agt tac cgt gaa gat ccc att gtc tat gaa att cat cca acc aaa tct 2256
ttt att agt ggt ggg agc aca ata aca ggt gtt ggg aaa aac ctg aat 2304
tca gtt agt gtc ccg aga atg gtc ata aat gtg cat gaa gca gga agg 2352
aac ttt aca gtg gca tgt caa cat cgc tct aat tca gag ata atc tgt    2400
tgt acc act cct tcc ctg caa cag ctg aat ctg caa ctc ccc ctg aaa    2448
acc aaa gcc ttt ttc atg tta gat ggg atc ctt tcc aaa tac ttt gat    2496
ctc att tat gta cat aat cct gtg ttt aag cct ttt gaa aag cca gtg    2544
atg atc tca atg ggc aat gaa aat gta ctg gaa att aag gga aat gat    2592
att gac cct gaa gca gtt aaa ggt gaa gtg tta aaa gtt gga aat aag    2640
agc tgt gag aat ata cac tta cat tct gaa gcc gtt tta tgc acg gtc    2688
ccc aat gac ctg ctg aaa ttg aac agc gag cta aat ata gag tgg aag    2736
caa gca att tct tca acc gtc ctt gga aaa gta ata gtt caa cca gat    2784
cag aat ttc aca gga ttg att gct ggt gtt gtc tca ata tca aca gca    2832
ctg tta tta cta ctt ggg ttt ttc ctg tgg ctg aaa aag aga aag caa    2880
att aaa gat ctg ggc agt gaa tta gtt cgc tac gat gca aga gta cac    2928
act cct cat ttg gat agg ctt gta agt gcc cga agt gta agc cca act    2976
aca gaa atg gtt tca aat gaa tct gta gac tac cga gct act ttt cca 3024
gaa gat cag ttt cct aat tca tct cag aac ggt tca tgc cga caa gtg 3072
cag tat cct ctg aca gac atg tcc ccc atc cta act agt ggg gac tct 3120
gat ata tcc agt cca tta ctg caa aat act gtc cac att gac ctc agt 3168
gct cta aat cca gag ctg gtc cag gca gtg cag cat gta gtg att ggg 3216
ccc agt agc ctg att gtg cat ttc aat gaa gtc ata gga aga ggg cat 3264
ttt ggt tgt gta tat cat ggg act ttg ttg gac aat gat ggc aag aaa 3312
att cac tgt gct gtg aaa tcc ttg aac aga atc act gac ata gga gaa 3360
gtt tcc caa ttt ctg acc gag gga atc atc atg aaa gat ttt agt cat 3408
ccc aat gtc ctc tcg ctc ctg gga atc tgc ctg cga agt gaa ggg tct 3456
ccg ctg gtg gtc cta cca tac atg aaa cat gga gat ctt cga aat ttc 3504
att cga aat gag act cat aat cca act gta aaa gat ctt att ggc ttt 3552
ggt ctt caa gta gcc aaa ggc atg aaa tat ctt gca agc aaa aag ttt 3600
gtc cac aga gac ttg gct gca aga aac tgt atg ctg gat gaa aaa ttc    3648
aca gtc aag gtt gct gat ttt ggt ctt gcc aga gac atg tat gat aaa    3696
gaa tac tat agt gta cac aac aaa aca ggt gca aag ctg cca gtg aag    3744
tgg atg gct ttg gaa agt ctg caa act caa aag ttt acc acc aag tca    3792
gat gtg tgg tcc ttt ggc gtc gtc ctc tgg gag ctg atg aca aga gga    3840
gcc cca cct tat cct gac gta aac acc ttt gat ata act gtt tac ttg    3888
ttg caa ggg aga aga ctc cta caa ccc gaa tac tgc cca gac ccc tta 3936
tat gaa gta atg cta aaa tgc tgg cac cct aaa gcc gaa atg cgc cca 3984
tcc ttt tct gaa ctg gtg tcc cgg ata tca gcg atc ttc tct act ttc 4032
att ggg gag cac tat gtc cat gtg aac gct act tat gtg aac gta aaa 4080
tgt gtc gct ccg tat cct tct ctg ttg tca tca gaa gat aac gct gat 4128
gat gag gtg gac aca cga cca gcc tcc ttc tgg gag aca tca tag 4173

<210> 2

<211> 1390

<212> PRT

<213> Homo sapiens

<400> 2

<210> 3

<211> 3252

<212> DNA

<213> Homo sapiens

<220>

<221> CDS

<222> (1)..(3252)

<400> 3

tcc aca aag aag gaa gtg ttt aat ata ctt cag gct gcg tat gtc agc 48

Ser Thr Lys Lys Glu Val Phe Asn Ile Leu Gln Ala Ala Tyr Val Ser

1               5                   10                  15

aag cct ggg gcc cag ctt gct aga caa ata gga gcc agc ctg aat gat 96

Lys Pro Gly Ala Gln Leu Ala Arg Gln Ile Gly Ala Ser Leu Asn Asp

            20                  25                  30

gac att ctt ttc ggg gtg ttc gca caa agc aag cca gat tct gcc gaa 144

Asp Ile Leu Phe Gly Val Phe Ala Gln Ser Lys Pro Asp Ser Ala Glu

        35                  40                  45

cca atg gat cga tct gcc atg tgt gca ttc cct atc aaa tat gtc aac 192

Pro Met Asp Arg Ser Ala Met Cys Ala Phe Pro Ile Lys Tyr Val Asn

    50                  55                  60

gac ttc ttc aac aag atc gtc aac aaa aac aat gtg aga tgt ctc cag 240
Asp Phe Phe Asn Lys Ile Val Asn Lys Asn Asn Val Arg Cys Leu Gln
65                  70                  75                  80

cat ttt tac gga ccc aat cat gag cac tgc ttt aat agg aca ctt ctg 288
His Phe Tyr Gly Pro Asn His Glu His Cys Phe Asn Arg Thr Leu Leu
                85                  90                  95

aga aat tca tca ggc tgt gaa gcg cgc cgt gat gaa tat cga aca gag 336
Arg Asn Ser Ser Gly Cys Glu Ala Arg Arg Asp Glu Tyr Arg Thr Glu
            100                 105                 110

ttt acc aca gct ttg cag cgc gtt gac tta ttc atg ggt caa ttc agc 384
Phe Thr Thr Ala Leu Gln Arg Val Asp Leu Phe Met Gly Gln Phe Ser
        115                 120                 125

gaa gtc ctc tta aca tct ata tcc acc ttc att aaa gga gac ctc acc 432
Glu Val Leu Leu Thr Ser Ile Ser Thr Phe Ile Lys Gly Asp Leu Thr
    130                 135                 140

ata gct aat ctt ggg aca tca gag ggt cgc ttc atg cag gtt gtg gtt 480
Ile Ala Asn Leu Gly Thr Ser Glu Gly Arg Phe Met Gln Val Val Val
145                 150                 155                 160

tct cga tca gga cca tca acc cct cat gtg aat ttt ctc ctg gac tcc 528
Ser Arg Ser Gly Pro Ser Thr Pro His Val Asn Phe Leu Leu Asp Ser

                165                 170                 175

cat cca gtg tct cca gaa gtg att gtg gag cat aca tta aac caa aat 576
His Pro Val Ser Pro Glu Val Ile Val Glu His Thr Leu Asn Gln Asn
            180                 185                 190

ggc tac aca ctg gtt atc act ggg aag aag atc acg aag atc cca ttg 624
Gly Tyr Thr Leu Val Ile Thr Gly Lys Lys Ile Thr Lys Ile Pro Leu
        195                 200                 205

aat ggc ttg ggc tgc aga cat ttc cag tcc tgc agt caa tgc ctc tct 672
Asn Gly Leu Gly Cys Arg His Phe Gln Ser Cys Ser Gln Cys Leu Ser
    210                 215                 220

gcc cca ccc ttt gtt cag tgt ggc tgg tgc cac gac aaa tgt gtg cga 720
Ala Pro Pro Phe Val Gln Cys Gly Trp Cys His Asp Lys Cys Val Arg
225                 230                 235                 240

tcg gag gaa tgc ctg agc ggg aca tgg act caa cag atc tgt ctg cct 768
Ser Glu Glu Cys Leu Ser Gly Thr Trp Thr Gln Gln Ile Cys Leu Pro
                245                 250                 255

gca atc tac aag gtt ttc cca aat agt gca ccc ctt gaa gga ggg aca 816
Ala Ile Tyr Lys Val Phe Pro Asn Ser Ala Pro Leu Glu Gly Gly Thr
            260                 265                 270

agg ctg acc ata tgt ggc tgg gac ttt gga ttt cgg agg aat aat aaa 864
Arg Leu Thr Ile Cys Gly Trp Asp Phe Gly Phe Arg Arg Asn Asn Lys
        275                 280                 285

ttt gat tta aag aaa act aga gtt ctc ctt gga aat gag agc tgc acc 912
Phe Asp Leu Lys Lys Thr Arg Val Leu Leu Gly Asn Glu Ser Cys Thr
    290                 295                 300

ttg act tta agt gag agc acg atg aat aca ttg aaa tgc aca gtt ggt 960
Leu Thr Leu Ser Glu Ser Thr Met Asn Thr Leu Lys Cys Thr Val Gly
305                 310                 315                 320

cct gcc atg aat aag cat ttc aat atg tcc ata att att tca aat ggc 1008
Pro Ala Met Asn Lys His Phe Asn Met Ser Ile Ile Ile Ser Asn Gly
                325                 330                 335

cac ggg aca aca caa tac agt aca ttc tcc tat gtg gat cct gta ata 1056
His Gly Thr Thr Gln Tyr Ser Thr Phe Ser Tyr Val Asp Pro Val Ile
            340                 345                 350

aca agt att tcg ccg aaa tac ggt cct atg gct ggt ggc act tta ctt 1104
Thr Ser Ile Ser Pro Lys Tyr Gly Pro Met Ala Gly Gly Thr Leu Leu
        355                 360                 365

act tta act gga aat tac cta aac agt ggg aat tct aga cac att tca 1152
Thr Leu Thr Gly Asn Tyr Leu Asn Ser Gly Asn Ser Arg His Ile Ser

370                 375                 380

att ggt gga aaa aca tgt act tta aaa agt gtg tca aac agt att ctt    1200
Ile Gly Gly Lys Thr Cys Thr Leu Lys Ser Val Ser Asn Ser Ile Leu
385                 390                 395                 400

gaa tgt tat acc cca gcc caa acc att tca act gag ttt gct gtt aaa    1248
Glu Cys Tyr Thr Pro Ala Gln Thr Ile Ser Thr Glu Phe Ala Val Lys
                405                 410                 415

ttg aaa att gac tta gcc aac cga gag aca agc atc ttc agt tac cgt    1296
Leu Lys Ile Asp Leu Ala Asn Arg Glu Thr Ser Ile Phe Ser Tyr Arg
            420                 425                 430

gaa gat ccc att gtc tat gaa att cat cca acc aaa tct ttt att agt    1344
Glu Asp Pro Ile Val Tyr Glu Ile His Pro Thr Lys Ser Phe Ile Ser
        435                 440                 445

ggt ggg agc aca ata aca ggt gtt ggg aaa aac ctg aat tca gtt agt    1392
Gly Gly Ser Thr Ile Thr Gly Val Gly Lys Asn Leu Asn Ser Val Ser
    450                 455                 460

gtc ccg aga atg gtc ata aat gtg cat gaa gca gga agg aac ttt aca    1440
Val Pro Arg Met Val Ile Asn Val His Glu Ala Gly Arg Asn Phe Thr
465                 470                 475                 480

gtg gca tgt caa cat cgc tct aat tca gag ata atc tgt tgt acc act 1488
Val Ala Cys Gln His Arg Ser Asn Ser Glu Ile Ile Cys Cys Thr Thr
                485                 490                 495

cct tcc ctg caa cag ctg aat ctg caa ctc ccc ctg aaa acc aaa gcc 1536
Pro Ser Leu Gln Gln Leu Asn Leu Gln Leu Pro Leu Lys Thr Lys Ala
            500                 505                 510

ttt ttc atg tta gat ggg atc ctt tcc aaa tac ttt gat ctc att tat 1584
Phe Phe Met Leu Asp Gly Ile Leu Ser Lys Tyr Phe Asp Leu Ile Tyr
        515                 520                 525

gta cat aat cct gtg ttt aag cct ttt gaa aag cca gtg atg atc tca 1632
Val His Asn Pro Val Phe Lys Pro Phe Glu Lys Pro Val Met Ile Ser
    530                 535                 540

atg ggc aat gaa aat gta ctg gaa att aag gga aat gat att gac cct 1680
Met Gly Asn Glu Asn Val Leu Glu Ile Lys Gly Asn Asp Ile Asp Pro
545                 550                 555                 560

gaa gca gtt aaa ggt gaa gtg tta aaa gtt gga aat aag agc tgt gag 1728
Glu Ala Val Lys Gly Glu Val Leu Lys Val Gly Asn Lys Ser Cys Glu
                565                 570                 575

aat ata cac tta cat tct gaa gcc gtt tta tgc acg gtc ccc aat gac 1776
Asn Ile His Leu His Ser Glu Ala Val Leu Cys Thr Val Pro Asn Asp

            580                 585                 590

ctg ctg aaa ttg aac agc gag cta aat ata gag tgg aag caa gca att 1824
Leu Leu Lys Leu Asn Ser Glu Leu Asn Ile Glu Trp Lys Gln Ala Ile
        595                 600                 605

tct tca acc gtc ctt gga aaa gta ata gtt caa cca gat cag aat ttc 1872
Ser Ser Thr Val Leu Gly Lys Val Ile Val Gln Pro Asp Gln Asn Phe
    610                 615                 620

aca gga ttg att gct ggt gtt gtc tca ata tca aca gca ctg tta tta 1920
Thr Gly Leu Ile Ala Gly Val Val Ser Ile Ser Thr Ala Leu Leu Leu
625                 630                 635                 640

cta ctt ggg ttt ttc ctg tgg ctg aaa aag aga aag caa att aaa gat 1968
Leu Leu Gly Phe Phe Leu Trp Leu Lys Lys Arg Lys Gln Ile Lys Asp
                645                 650                 655

ctg ggc agt gaa tta gtt cgc tac gat gca aga gta cac act cct cat 2016
Leu Gly Ser Glu Leu Val Arg Tyr Asp Ala Arg Val His Thr Pro His
            660                 665                 670

ttg gat agg ctt gta agt gcc cga agt gta agc cca act aca gaa atg 2064
Leu Asp Arg Leu Val Ser Ala Arg Ser Val Ser Pro Thr Thr Glu Met
        675                 680                 685

gtt tca aat gaa tct gta gac tac cga gct act ttt cca gaa gat cag    2112
Val Ser Asn Glu Ser Val Asp Tyr Arg Ala Thr Phe Pro Glu Asp Gln
    690                 695                 700

ttt cct aat tca tct cag aac ggt tca tgc cga caa gtg cag tat cct    2160
Phe Pro Asn Ser Ser Gln Asn Gly Ser Cys Arg Gln Val Gln Tyr Pro
705                 710                 715                 720

ctg aca gac atg tcc ccc atc cta act agt ggg gac tct gat ata tcc    2208
Leu Thr Asp Met Ser Pro Ile Leu Thr Ser Gly Asp Ser Asp Ile Ser
                725                 730                 735

agt cca tta ctg caa aat act gtc cac att gac ctc agt gct cta aat    2256
Ser Pro Leu Leu Gln Asn Thr Val His Ile Asp Leu Ser Ala Leu Asn
            740                 745                 750

cca gag ctg gtc cag gca gtg cag cat gta gtg att ggg ccc agt agc    2304
Pro Glu Leu Val Gln Ala Val Gln His Val Val Ile Gly Pro Ser Ser
        755                 760                 765

ctg att gtg cat ttc aat gaa gtc ata gga aga ggg cat ttt ggt tgt    2352
Leu Ile Val His Phe Asn Glu Val Ile Gly Arg Gly His Phe Gly Cys
    770                 775                 780

gta tat cat ggg act ttg ttg gac aat gat ggc aag aaa att cac tgt    2400
Val Tyr His Gly Thr Leu Leu Asp Asn Asp Gly Lys Lys Ile His Cys

785                 790                 795                 800

gct gtg aaa tcc ttg aac aga atc act gac ata gga gaa gtt tcc caa 2448
Ala Val Lys Ser Leu Asn Arg Ile Thr Asp Ile Gly Glu Val Ser Gln
                805                 810                 815

ttt ctg acc gag gga atc atc atg aaa gat ttt agt cat ccc aat gtc 2496
Phe Leu Thr Glu Gly Ile Ile Met Lys Asp Phe Ser His Pro Asn Val
            820                 825                 830

ctc tcg ctc ctg gga atc tgc ctg cga agt gaa ggg tct ccg ctg gtg 2544
Leu Ser Leu Leu Gly Ile Cys Leu Arg Ser Glu Gly Ser Pro Leu Val
        835                 840                 845

gtc cta cca tac atg aaa cat gga gat ctt cga aat ttc att cga aat 2592
Val Leu Pro Tyr Met Lys His Gly Asp Leu Arg Asn Phe Ile Arg Asn
    850                 855                 860

gag act cat aat cca act gta aaa gat ctt att ggc ttt ggt ctt caa 2640
Glu Thr His Asn Pro Thr Val Lys Asp Leu Ile Gly Phe Gly Leu Gln
865                 870                 875                 880

gta gcc aaa ggc atg aaa tat ctt gca agc aaa aag ttt gtc cac aga 2688
Val Ala Lys Gly Met Lys Tyr Leu Ala Ser Lys Lys Phe Val His Arg
                885                 890                 895

gac ttg gct gca aga aac tgt atg ctg gat gaa aaa ttc aca gtc aag 2736
Asp Leu Ala Ala Arg Asn Cys Met Leu Asp Glu Lys Phe Thr Val Lys
            900                 905                 910

gtt gct gat ttt ggt ctt gcc aga gac atg tat gat aaa gaa tac tat 2784
Val Ala Asp Phe Gly Leu Ala Arg Asp Met Tyr Asp Lys Glu Tyr Tyr
        915                 920                 925

agt gta cac aac aaa aca ggt gca aag ctg cca gtg aag tgg atg gct 2832
Ser Val His Asn Lys Thr Gly Ala Lys Leu Pro Val Lys Trp Met Ala
    930                 935                 940

ttg gaa agt ctg caa act caa aag ttt acc acc aag tca gat gtg tgg 2880
Leu Glu Ser Leu Gln Thr Gln Lys Phe Thr Thr Lys Ser Asp Val Trp
945                 950                 955                 960

tcc ttt ggc gtc gtc ctc tgg gag ctg atg aca aga gga gcc cca cct 2928
Ser Phe Gly Val Val Leu Trp Glu Leu Met Thr Arg Gly Ala Pro Pro
                965                 970                 975

tat cct gac gta aac acc ttt gat ata act gtt tac ttg ttg caa ggg 2976
Tyr Pro Asp Val Asn Thr Phe Asp Ile Thr Val Tyr Leu Leu Gln Gly
            980                 985                 990

aga aga ctc cta caa ccc gaa tac tgc cca gac ccc tta tat gaa gta 3024
Arg Arg Leu Leu Gln Pro Glu Tyr Cys Pro Asp Pro Leu Tyr Glu Val

995                 1000                1005

atg cta aaa tgc tgg cac cct aaa gcc gaa atg cgc cca tcc ttt tct    3072
Met Leu Lys Cys Trp His Pro Lys Ala Glu Met Arg Pro Ser Phe Ser
    1010                1015                1020

gaa ctg gtg tcc cgg ata tca gcg atc ttc tct act ttc att ggg gag    3120
Glu Leu Val Ser Arg Ile Ser Ala Ile Phe Ser Thr Phe Ile Gly Glu
1025                1030                1035                1040

cac tat gtc cat gtg aac gct act tat gtg aac gta aaa tgt gtc gct    3168
His Tyr Val His Val Asn Ala Thr Tyr Val Asn Val Lys Cys Val Ala
                1045                1050                1055

ccg tat cct tct ctg ttg tca tca gaa gat aac gct gat gat gag gtg    3216
Pro Tyr Pro Ser Leu Leu Ser Ser Glu Asp Asn Ala Asp Asp Glu Val
            1060                1065                1070

gac aca cga cca gcc tcc ttc tgg gag aca tca tag                    3252
Asp Thr Arg Pro Ala Ser Phe Trp Glu Thr Ser
        1075                1080

<210> 4
<211> 1083
<212> PRT

<213> Homo sapiens

<400> 4

Ser Thr Lys Lys Glu Val Phe Asn Ile Leu Gln Ala Ala Tyr Val Ser
1               5                   10                  15

Lys Pro Gly Ala Gln Leu Ala Arg Gln Ile Gly Ala Ser Leu Asn Asp
            20                  25                  30

Asp Ile Leu Phe Gly Val Phe Ala Gln Ser Lys Pro Asp Ser Ala Glu
        35                  40                  45

Pro Met Asp Arg Ser Ala Met Cys Ala Phe Pro Ile Lys Tyr Val Asn
    50                  55                  60

Asp Phe Phe Asn Lys Ile Val Asn Lys Asn Asn Val Arg Cys Leu Gln
65                  70                  75                  80

His Phe Tyr Gly Pro Asn His Glu His Cys Phe Asn Arg Thr Leu Leu
                85                  90                  95

Arg Asn Ser Ser Gly Cys Glu Ala Arg Arg Asp Glu Tyr Arg Thr Glu
            100                 105                 110

Phe Thr Thr Ala Leu Gln Arg Val Asp Leu Phe Met Gly Gln Phe Ser
        115                 120                 125

Glu Val Leu Leu Thr Ser Ile Ser Thr Phe Ile Lys Gly Asp Leu Thr
    130                 135                 140

Ile Ala Asn Leu Gly Thr Ser Glu Gly Arg Phe Met Gln Val Val Val
145                 150                 155                 160

Ser Arg Ser Gly Pro Ser Thr Pro His Val Asn Phe Leu Leu Asp Ser
                165                 170                 175

His Pro Val Ser Pro Glu Val Ile Val Glu His Thr Leu Asn Gln Asn

            180                 185                 190
Gly Tyr Thr Leu Val Ile Thr Gly Lys Lys Ile Thr Lys Ile Pro Leu
        195                 200                 205
Asn Gly Leu Gly Cys Arg His Phe Gln Ser Cys Ser Gln Cys Leu Ser
    210                 215                 220
Ala Pro Pro Phe Val Gln Cys Gly Trp Cys His Asp Lys Cys Val Arg
225                 230                 235                 240
Ser Glu Glu Cys Leu Ser Gly Thr Trp Thr Gln Gln Ile Cys Leu Pro
                245                 250                 255
Ala Ile Tyr Lys Val Phe Pro Asn Ser Ala Pro Leu Glu Gly Gly Thr
            260                 265                 270
Arg Leu Thr Ile Cys Gly Trp Asp Phe Gly Phe Arg Arg Asn Asn Lys
        275                 280                 285
Phe Asp Leu Lys Lys Thr Arg Val Leu Leu Gly Asn Glu Ser Cys Thr
    290                 295                 300
Leu Thr Leu Ser Glu Ser Thr Met Asn Thr Leu Lys Cys Thr Val Gly
305                 310                 315                 320
Pro Ala Met Asn Lys His Phe Asn Met Ser Ile Ile Ile Ser Asn Gly
                325                 330                 335
His Gly Thr Thr Gln Tyr Ser Thr Phe Ser Tyr Val Asp Pro Val Ile
            340                 345                 350
Thr Ser Ile Ser Pro Lys Tyr Gly Pro Met Ala Gly Gly Thr Leu Leu
        355                 360                 365
Thr Leu Thr Gly Asn Tyr Leu Asn Ser Gly Asn Ser Arg His Ile Ser
    370                 375                 380
Ile Gly Gly Lys Thr Cys Thr Leu Lys Ser Val Ser Asn Ser Ile Leu

385                 390                 395                 400

Glu Cys Tyr Thr Pro Ala Gln Thr Ile Ser Thr Glu Phe Ala Val Lys

                405                 410                 415

Leu Lys Ile Asp Leu Ala Asn Arg Glu Thr Ser Ile Phe Ser Tyr Arg

            420                 425                 430

Glu Asp Pro Ile Val Tyr Glu Ile His Pro Thr Lys Ser Phe Ile Ser

        435                 440                 445

Gly Gly Ser Thr Ile Thr Gly Val Gly Lys Asn Leu Asn Ser Val Ser

    450                 455                 460

Val Pro Arg Met Val Ile Asn Val His Glu Ala Gly Arg Asn Phe Thr

465                 470                 475                 480

Val Ala Cys Gln His Arg Ser Asn Ser Glu Ile Ile Cys Cys Thr Thr

                485                 490                 495

Pro Ser Leu Gln Gln Leu Asn Leu Gln Leu Pro Leu Lys Thr Lys Ala

            500                 505                 510

Phe Phe Met Leu Asp Gly Ile Leu Ser Lys Tyr Phe Asp Leu Ile Tyr

        515                 520                 525

Val His Asn Pro Val Phe Lys Pro Phe Glu Lys Pro Val Met Ile Ser

    530                 535                 540

Met Gly Asn Glu Asn Val Leu Glu Ile Lys Gly Asn Asp Ile Asp Pro

545                 550                 555                 560

Glu Ala Val Lys Gly Glu Val Leu Lys Val Gly Asn Lys Ser Cys Glu

                565                 570                 575

Asn Ile His Leu His Ser Glu Ala Val Leu Cys Thr Val Pro Asn Asp

            580                 585                 590

Leu Leu Lys Leu Asn Ser Glu Leu Asn Ile Glu Trp Lys Gln Ala Ile

        595                 600                 605
Ser Ser Thr Val Leu Gly Lys Val Ile Val Gln Pro Asp Gln Asn Phe
    610                 615                 620
Thr Gly Leu Ile Ala Gly Val Val Ser Ile Ser Thr Ala Leu Leu Leu
625                 630                 635                 640
Leu Leu Gly Phe Phe Leu Trp Leu Lys Lys Arg Lys Gln Ile Lys Asp
                645                 650                 655
Leu Gly Ser Glu Leu Val Arg Tyr Asp Ala Arg Val His Thr Pro His
            660                 665                 670
Leu Asp Arg Leu Val Ser Ala Arg Ser Val Ser Pro Thr Thr Glu Met
        675                 680                 685
Val Ser Asn Glu Ser Val Asp Tyr Arg Ala Thr Phe Pro Glu Asp Gln
    690                 695                 700
Phe Pro Asn Ser Ser Gln Asn Gly Ser Cys Arg Gln Val Gln Tyr Pro
705                 710                 715                 720
Leu Thr Asp Met Ser Pro Ile Leu Thr Ser Gly Asp Ser Asp Ile Ser
                725                 730                 735
Ser Pro Leu Leu Gln Asn Thr Val His Ile Asp Leu Ser Ala Leu Asn
            740                 745                 750
Pro Glu Leu Val Gln Ala Val Gln His Val Val Ile Gly Pro Ser Ser
        755                 760                 765
Leu Ile Val His Phe Asn Glu Val Ile Gly Arg Gly His Phe Gly Cys
    770                 775                 780
Val Tyr His Gly Thr Leu Leu Asp Asn Asp Gly Lys Lys Ile His Cys
785                 790                 795                 800
Ala Val Lys Ser Leu Asn Arg Ile Thr Asp Ile Gly Glu Val Ser Gln

                805                 810                 815
Phe Leu Thr Glu Gly Ile Ile Met Lys Asp Phe Ser His Pro Asn Val
            820                 825                 830
Leu Ser Leu Leu Gly Ile Cys Leu Arg Ser Glu Gly Ser Pro Leu Val
        835                 840                 845
Val Leu Pro Tyr Met Lys His Gly Asp Leu Arg Asn Phe Ile Arg Asn
    850                 855                 860
Glu Thr His Asn Pro Thr Val Lys Asp Leu Ile Gly Phe Gly Leu Gln
865                 870                 875                 880
Val Ala Lys Gly Met Lys Tyr Leu Ala Ser Lys Lys Phe Val His Arg
                885                 890                 895
Asp Leu Ala Ala Arg Asn Cys Met Leu Asp Glu Lys Phe Thr Val Lys
            900                 905                 910
Val Ala Asp Phe Gly Leu Ala Arg Asp Met Tyr Asp Lys Glu Tyr Tyr
        915                 920                 925
Ser Val His Asn Lys Thr Gly Ala Lys Leu Pro Val Lys Trp Met Ala
    930                 935                 940
Leu Glu Ser Leu Gln Thr Gln Lys Phe Thr Thr Lys Ser Asp Val Trp
945                 950                 955                 960
Ser Phe Gly Val Val Leu Trp Glu Leu Met Thr Arg Gly Ala Pro Pro
                965                 970                 975
Tyr Pro Asp Val Asn Thr Phe Asp Ile Thr Val Tyr Leu Leu Gln Gly
            980                 985                 990
Arg Arg Leu Leu Gln Pro Glu Tyr Cys Pro Asp Pro Leu Tyr Glu Val
        995                 1000                1005
Met Leu Lys Cys Trp His Pro Lys Ala Glu Met Arg Pro Ser Phe Ser

1010                1015                1020

Glu Leu Val Ser Arg Ile Ser Ala Ile Phe Ser Thr Phe Ile Gly Glu

1025                1030                1035                1040

His Tyr Val His Val Asn Ala Thr Tyr Val Asn Val Lys Cys Val Ala

                1045                1050                1055

Pro Tyr Pro Ser Leu Leu Ser Ser Glu Asp Asn Ala Asp Asp Glu Val

            1060                1065                1070

Asp Thr Arg Pro Ala Ser Phe Trp Glu Thr Ser

        1075                1080

<210> 5

<211> 40

<212> PRT

<213> Homo sapiens

<400> 5

Trp His Pro Lys Ala Glu Met Arg Pro Ser Phe Ser Glu Leu Val Ser

1               5                   10                  15

Arg Ile Ser Ala Ile Phe Ser Thr Phe Ile Gly Glu His Tyr Val His

            20                  25                  30

Val Asn Ala Thr Tyr Val Asn Val

        35                  40

<210> 6

<211> 2187

<212> DNA

<213> Homo sapiens

<220>

<221> CDS

<222> (1)..(2187)

<400> 6

atg tgg gtg acc aaa ctc ctg cca gcc ctg ctg ctg cag cat gtc ctc 48

Met Trp Val Thr Lys Leu Leu Pro Ala Leu Leu Leu Gln His Val Leu

1               5                   10                  15

ctg cat ctc ctc ctg ctc ccc atc gcc atc ccc tat gca gag gga caa 96

Leu His Leu Leu Leu Leu Pro Ile Ala Ile Pro Tyr Ala Glu Gly Gln

            20                  25                  30

agg aaa aga aga aat aca att cat gaa ttc aaa aaa tca gca aag act 144

Arg Lys Arg Arg Asn Thr Ile His Glu Phe Lys Lys Ser Ala Lys Thr

        35                  40                  45

acc cta atc aaa ata gat cca gca ctg aag ata aaa acc aaa aaa gtg 192

Thr Leu Ile Lys Ile Asp Pro Ala Leu Lys Ile Lys Thr Lys Lys Val

    50                  55                  60

aat act gca gac caa tgt gct aat aga tgt act agg aat aaa gga ctt 240
Asn Thr Ala Asp Gln Cys Ala Asn Arg Cys Thr Arg Asn Lys Gly Leu
65                  70                  75                  80

cca ttc act tgc aag gct ttt gtt ttt gat aaa gca aga aaa caa tgc 288
Pro Phe Thr Cys Lys Ala Phe Val Phe Asp Lys Ala Arg Lys Gln Cys
                85                  90                  95

ctc tgg ttc ccc ttc aat agc atg tca agt gga gtg aaa aaa gaa ttt 336
Leu Trp Phe Pro Phe Asn Ser Met Ser Ser Gly Val Lys Lys Glu Phe
            100                 105                 110

ggc cat gaa ttt gac ctc tat gaa aac aaa gac tac att aga aac tgc 384
Gly His Glu Phe Asp Leu Tyr Glu Asn Lys Asp Tyr Ile Arg Asn Cys
        115                 120                 125

atc att ggt aaa gga cgc agc tac aag gga aca gta tct atc act aag 432
Ile Ile Gly Lys Gly Arg Ser Tyr Lys Gly Thr Val Ser Ile Thr Lys
    130                 135                 140

agt ggc atc aaa tgt cag ccc tgg agt tcc atg ata cca cac gaa cac 480
Ser Gly Ile Lys Cys Gln Pro Trp Ser Ser Met Ile Pro His Glu His
145                 150                 155                 160

agc ttt ttg cct tcg agc tat cgg ggt aaa gac cta cag gaa aac tac 528
Ser Phe Leu Pro Ser Ser Tyr Arg Gly Lys Asp Leu Gln Glu Asn Tyr
                165                 170                 175

tgt cga aat cct cga ggg gaa gaa ggg gga ccc tgg tgt ttc aca agc 576
Cys Arg Asn Pro Arg Gly Glu Glu Gly Gly Pro Trp Cys Phe Thr Ser
            180                 185                 190

aat cca gag gta cgc tac gaa gtc tgt gac att cct cag tgt tca gaa 624
Asn Pro Glu Val Arg Tyr Glu Val Cys Asp Ile Pro Gln Cys Ser Glu
        195                 200                 205

gtt gaa tgc atg acc tgc aat ggg gag agt tat cga ggt ctc atg gat 672
Val Glu Cys Met Thr Cys Asn Gly Glu Ser Tyr Arg Gly Leu Met Asp
    210                 215                 220

cat aca gaa tca ggc aag att tgt cag cgc tgg gat cat cag aca cca 720
His Thr Glu Ser Gly Lys Ile Cys Gln Arg Trp Asp His Gln Thr Pro
225                 230                 235                 240

cac cgg cac aaa ttc ttg cct gaa aga tat ccc gac aag ggc ttt gat 768
His Arg His Lys Phe Leu Pro Glu Arg Tyr Pro Asp Lys Gly Phe Asp
                245                 250                 255

gat aat tat tgc cgc aat ccc gat ggc cag ccg agg cca tgg tgc tat 816
Asp Asn Tyr Cys Arg Asn Pro Asp Gly Gln Pro Arg Pro Trp Cys Tyr

            260                 265                 270

act ctt gac cct cac acc cgc tgg gag tac tgt gca att aaa aca tgc 864
Thr Leu Asp Pro His Thr Arg Trp Glu Tyr Cys Ala Ile Lys Thr Cys
        275                 280                 285

gct gac aat act atg aat gac act gat gtt cct ttg gaa aca act gaa 912
Ala Asp Asn Thr Met Asn Asp Thr Asp Val Pro Leu Glu Thr Thr Glu
    290                 295                 300

tgc atc caa ggt caa gga gaa ggc tac agg ggc act gtc aat acc att 960
Cys Ile Gln Gly Gln Gly Glu Gly Tyr Arg Gly Thr Val Asn Thr Ile
305                 310                 315                 320

tgg aat gga att cca tgt cag cgt tgg gat tct cag tat cct cac gag 1008
Trp Asn Gly Ile Pro Cys Gln Arg Trp Asp Ser Gln Tyr Pro His Glu
                325                 330                 335

cat gac atg act cct gaa aat ttc aag tgc aag gac cta cga gaa aat 1056
His Asp Met Thr Pro Glu Asn Phe Lys Cys Lys Asp Leu Arg Glu Asn
            340                 345                 350

tac tgc cga aat cca gat ggg tct gaa tca ccc tgg tgt ttt acc act 1104
Tyr Cys Arg Asn Pro Asp Gly Ser Glu Ser Pro Trp Cys Phe Thr Thr
        355                 360                 365

gat cca aac atc cga gtt ggc tac tgc tcc caa att cca aac tgt gat 1152
Asp Pro Asn Ile Arg Val Gly Tyr Cys Ser Gln Ile Pro Asn Cys Asp
    370                 375                 380

atg tca cat gga caa gat tgt tat cgt ggg aat ggc aaa aat tat atg 1200
Met Ser His Gly Gln Asp Cys Tyr Arg Gly Asn Gly Lys Asn Tyr Met
385                 390                 395                 400

ggc aac tta tcc caa aca aga tct gga cta aca tgt tca atg tgg gac 1248
Gly Asn Leu Ser Gln Thr Arg Ser Gly Leu Thr Cys Ser Met Trp Asp
                405                 410                 415

aag aac atg gaa gac tta cat cgt cat atc ttc tgg gaa cca gat gca 1296
Lys Asn Met Glu Asp Leu His Arg His Ile Phe Trp Glu Pro Asp Ala
            420                 425                 430

agt aag ctg aat gag aat tac tgc cga aat cca gat gat gat gct cat 1344
Ser Lys Leu Asn Glu Asn Tyr Cys Arg Asn Pro Asp Asp Asp Ala His
        435                 440                 445

gga ccc tgg tgc tac acg gga aat cca ctc att cct tgg gat tat tgc 1392
Gly Pro Trp Cys Tyr Thr Gly Asn Pro Leu Ile Pro Trp Asp Tyr Cys
    450                 455                 460

cct att tct cgt tgt gaa ggt gat acc aca cct aca ata gtc aat tta 1440
Pro Ile Ser Arg Cys Glu Gly Asp Thr Thr Pro Thr Ile Val Asn Leu

465                 470                 475                 480

gac cat ccc gta ata tct tgt gcc aaa acg aaa caa ttg cga gtt gta 1488
Asp His Pro Val Ile Ser Cys Ala Lys Thr Lys Gln Leu Arg Val Val
                485                 490                 495

aat ggg att cca aca cga aca aac ata gga tgg atg gtt agt ttg aga 1536
Asn Gly Ile Pro Thr Arg Thr Asn Ile Gly Trp Met Val Ser Leu Arg
            500                 505                 510

tac aga aat aaa cat atc tgc gga gga tca ttg ata aag gag agt tgg 1584
Tyr Arg Asn Lys His Ile Cys Gly Gly Ser Leu Ile Lys Glu Ser Trp
        515                 520                 525

gtt ctt act gca cga cag tgt ttc cct tct cga gac ttg aaa gat tat 1632
Val Leu Thr Ala Arg Gln Cys Phe Pro Ser Arg Asp Leu Lys Asp Tyr
    530                 535                 540

gaa gct tgg ctt gga att cat gat gtc cac gga aga gga gat gag aaa 1680
Glu Ala Trp Leu Gly Ile His Asp Val His Gly Arg Gly Asp Glu Lys
545                 550                 555                 560

tgc aaa cag gtt ctc aat gtt tcc cag ctg gta tat ggc cct gaa gga 1728
Cys Lys Gln Val Leu Asn Val Ser Gln Leu Val Tyr Gly Pro Glu Gly
                565                 570                 575

tca gat ctg gtt tta atg aag ctt gcc agg cct gct gtc ctg gat gat 1776
Ser Asp Leu Val Leu Met Lys Leu Ala Arg Pro Ala Val Leu Asp Asp
            580                 585                 590

ttt gtt agt acg att gat tta cct aat tat gga tgc aca att cct gaa 1824
Phe Val Ser Thr Ile Asp Leu Pro Asn Tyr Gly Cys Thr Ile Pro Glu
        595                 600                 605

aag acc agt tgc agt gtt tat ggc tgg ggc tac act gga ttg atc aac 1872
Lys Thr Ser Cys Ser Val Tyr Gly Trp Gly Tyr Thr Gly Leu Ile Asn
    610                 615                 620

tat gat ggc cta tta cga gtg gca cat ctc tat ata atg gga aat gag 1920
Tyr Asp Gly Leu Leu Arg Val Ala His Leu Tyr Ile Met Gly Asn Glu
625                 630                 635                 640

aaa tgc agc cag cat cat cga ggg aag gtg act ctg aat gag tct gaa 1968
Lys Cys Ser Gln His His Arg Gly Lys Val Thr Leu Asn Glu Ser Glu
                645                 650                 655

ata tgt gct ggg gct gaa aag att gga tca gga cca tgt gag ggg gat 2016
Ile Cys Ala Gly Ala Glu Lys Ile Gly Ser Gly Pro Cys Glu Gly Asp
            660                 665                 670

tat ggt ggc cca ctt gtt tgt gag caa cat aaa atg aga atg gtt ctt 2064
Tyr Gly Gly Pro Leu Val Cys Glu Gln His Lys Met Arg Met Val Leu

        675                 680                 685

ggt gtc att gtt cct ggt cgt gga tgt gcc att cca aat cgt cct ggt 2112
Gly Val Ile Val Pro Gly Arg Gly Cys Ala Ile Pro Asn Arg Pro Gly
    690                 695                 700

att ttt gtc cga gta gca tat tat gca aaa tgg ata cac aaa att att 2160
Ile Phe Val Arg Val Ala Tyr Tyr Ala Lys Trp Ile His Lys Ile Ile
705                 710                 715                 720

tta aca tat aag gta cca cag tca tag 2187
Leu Thr Tyr Lys Val Pro Gln Ser
                725

<210> 7
<211> 728
<212> PRT
<213> Homo sapiens

<400> 7
Met Trp Val Thr Lys Leu Leu Pro Ala Leu Leu Leu Gln His Val Leu
1               5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr

35                  40                  45

Thr Leu Ile Lys Ile Asp Pro Ala Leu Lys Ile Lys Thr Lys Lys Val

    50                  55                  60

Asn Thr Ala Asp Gln Cys Ala Asn Arg Cys Thr Arg Asn Lys Gly Leu

65                  70                  75                  80

Pro Phe Thr Cys Lys Ala Phe Val Phe Asp Lys Ala Arg Lys Gln Cys

                85                  90                  95

Leu Trp Phe Pro Phe Asn Ser Met Ser Ser Gly Val Lys Lys Glu Phe

            100                 105                 110

Gly His Glu Phe Asp Leu Tyr Glu Asn Lys Asp Tyr Ile Arg Asn Cys

        115                 120                 125

Ile Ile Gly Lys Gly Arg Ser Tyr Lys Gly Thr Val Ser Ile Thr Lys

    130                 135                 140

Ser Gly Ile Lys Cys Gln Pro Trp Ser Ser Met Ile Pro His Glu His

145                 150                 155                 160

Ser Phe Leu Pro Ser Ser Tyr Arg Gly Lys Asp Leu Gln Glu Asn Tyr

                165                 170                 175

Cys Arg Asn Pro Arg Gly Glu Glu Gly Gly Pro Trp Cys Phe Thr Ser

            180                 185                 190

Asn Pro Glu Val Arg Tyr Glu Val Cys Asp Ile Pro Gln Cys Ser Glu

        195                 200                 205

Val Glu Cys Met Thr Cys Asn Gly Glu Ser Tyr Arg Gly Leu Met Asp

    210                 215                 220

His Thr Glu Ser Gly Lys Ile Cys Gln Arg Trp Asp His Gln Thr Pro

225                 230                 235                 240

His Arg His Lys Phe Leu Pro Glu Arg Tyr Pro Asp Lys Gly Phe Asp

                245                 250                 255
Asp Asn Tyr Cys Arg Asn Pro Asp Gly Gln Pro Arg Pro Trp Cys Tyr
            260                 265                 270
Thr Leu Asp Pro His Thr Arg Trp Glu Tyr Cys Ala Ile Lys Thr Cys
        275                 280                 285
Ala Asp Asn Thr Met Asn Asp Thr Asp Val Pro Leu Glu Thr Thr Glu
    290                 295                 300
Cys Ile Gln Gly Gln Gly Glu Gly Tyr Arg Gly Thr Val Asn Thr Ile
305                 310                 315                 320
Trp Asn Gly Ile Pro Cys Gln Arg Trp Asp Ser Gln Tyr Pro His Glu
                325                 330                 335
His Asp Met Thr Pro Glu Asn Phe Lys Cys Lys Asp Leu Arg Glu Asn
            340                 345                 350
Tyr Cys Arg Asn Pro Asp Gly Ser Glu Ser Pro Trp Cys Phe Thr Thr
        355                 360                 365
Asp Pro Asn Ile Arg Val Gly Tyr Cys Ser Gln Ile Pro Asn Cys Asp
    370                 375                 380
Met Ser His Gly Gln Asp Cys Tyr Arg Gly Asn Gly Lys Asn Tyr Met
385                 390                 395                 400
Gly Asn Leu Ser Gln Thr Arg Ser Gly Leu Thr Cys Ser Met Trp Asp
                405                 410                 415
Lys Asn Met Glu Asp Leu His Arg His Ile Phe Trp Glu Pro Asp Ala
            420                 425                 430
Ser Lys Leu Asn Glu Asn Tyr Cys Arg Asn Pro Asp Asp Asp Ala His
        435                 440                 445
Gly Pro Trp Cys Tyr Thr Gly Asn Pro Leu Ile Pro Trp Asp Tyr Cys

    450                 455                 460
Pro Ile Ser Arg Cys Glu Gly Asp Thr Thr Pro Thr Ile Val Asn Leu
465                 470                 475                 480
Asp His Pro Val Ile Ser Cys Ala Lys Thr Lys Gln Leu Arg Val Val
                485                 490                 495
Asn Gly Ile Pro Thr Arg Thr Asn Ile Gly Trp Met Val Ser Leu Arg
            500                 505                 510
Tyr Arg Asn Lys His Ile Cys Gly Gly Ser Leu Ile Lys Glu Ser Trp
        515                 520                 525
Val Leu Thr Ala Arg Gln Cys Phe Pro Ser Arg Asp Leu Lys Asp Tyr
    530                 535                 540
Glu Ala Trp Leu Gly Ile His Asp Val His Gly Arg Gly Asp Glu Lys
545                 550                 555                 560
Cys Lys Gln Val Leu Asn Val Ser Gln Leu Val Tyr Gly Pro Glu Gly
                565                 570                 575
Ser Asp Leu Val Leu Met Lys Leu Ala Arg Pro Ala Val Leu Asp Asp
            580                 585                 590
Phe Val Ser Thr Ile Asp Leu Pro Asn Tyr Gly Cys Thr Ile Pro Glu
        595                 600                 605
Lys Thr Ser Cys Ser Val Tyr Gly Trp Gly Tyr Thr Gly Leu Ile Asn
    610                 615                 620
Tyr Asp Gly Leu Leu Arg Val Ala His Leu Tyr Ile Met Gly Asn Glu
625                 630                 635                 640
Lys Cys Ser Gln His His Arg Gly Lys Val Thr Leu Asn Glu Ser Glu
                645                 650                 655
Ile Cys Ala Gly Ala Glu Lys Ile Gly Ser Gly Pro Cys Glu Gly Asp

660                 665                 670

Tyr Gly Gly Pro Leu Val Cys Glu Gln His Lys Met Arg Met Val Leu

        675                 680                 685

Gly Val Ile Val Pro Gly Arg Gly Cys Ala Ile Pro Asn Arg Pro Gly

    690                 695                 700

Ile Phe Val Arg Val Ala Tyr Tyr Ala Lys Trp Ile His Lys Ile Ile

705                 710                 715                 720

Leu Thr Tyr Lys Val Pro Gln Ser

                725

<210> 8

<211> 19

<212> PRT

<213> Homo sapiens

<220>

<221> UNSURE

<222> (1)

<223> Xaa means Ser, Ala or other amino acid.

<220>

<221> UNSURE

<222> (10)

<223> Xaa means Ser, Gly or other amino acid.

<220>

<221> UNSURE

<222> (11)

<223> Xaa means Pro, Leu or other amino acd.

<220>

<221> UNSURE

<222> (19)

<223> Xaa means Arg, Trp or other amino acid.

<400> 8

Xaa Ser Glu Ser Ser Ala Ser Ser Asp Xaa Xaa His Pro Val Ile Thr

1               5                   10                  15

Pro Ser Xaa

<210> 9

<211> 1512

<212> DNA

<213> Homo sapiens

<220>

<221> CDS

<222> (1)..(1512)

<400> 9

atg aca acg gac gac aca gaa gtg ccc gct atg act cta gca ccg ggc 48

Met Thr Thr Asp Asp Thr Glu Val Pro Ala Met Thr Leu Ala Pro Gly

1               5                   10                  15

cac gcc gct ctg gaa act caa acg ctg agc gct gag acc tct tct agg 96

His Ala Ala Leu Glu Thr Gln Thr Leu Ser Ala Glu Thr Ser Ser Arg

            20                  25                  30

gcc tca acc cca gcc ggc ccc att cca gaa gca gag acc agg gga gcc 144

Ala Ser Thr Pro Ala Gly Pro Ile Pro Glu Ala Glu Thr Arg Gly Ala

        35                  40                  45

aag aga att tcc cct gca aga gag acc agg agt ttc aca aaa aca tct 192

Lys Arg Ile Ser Pro Ala Arg Glu Thr Arg Ser Phe Thr Lys Thr Ser

    50                  55                  60

ccc aac ttc atg gtg ctg atc gcc acc tcc gtg gag aca tca gcc gcc 240

Pro Asn Phe Met Val Leu Ile Ala Thr Ser Val Glu Thr Ser Ala Ala

65                  70                  75                  80

agt ggc agc ccc gag gga gct gga atg acc aca gtt cag acc atc aca 288

Ser Gly Ser Pro Glu Gly Ala Gly Met Thr Thr Val Gln Thr Ile Thr

                85                  90                  95

ggc agt gat ccc gag gaa gcc atc ttt gac acc ctt tgc acc gat gac 336
Gly Ser Asp Pro Glu Glu Ala Ile Phe Asp Thr Leu Cys Thr Asp Asp
            100                 105                 110

agc tct gaa gag gca aag aca ctc aca atg gac ata ttg aca ttg gct 384
Ser Ser Glu Glu Ala Lys Thr Leu Thr Met Asp Ile Leu Thr Leu Ala
        115                 120                 125

cac acc tcc aca gaa gct aag ggc ctg tcc tca gag agc agt gcc tct 432
His Thr Ser Thr Glu Ala Lys Gly Leu Ser Ser Glu Ser Ser Ala Ser
    130                 135                 140

tcc gac ggc ccc cat cca gtc atc acc ccg tca cgg gcc tca gag agc 480
Ser Asp Gly Pro His Pro Val Ile Thr Pro Ser Arg Ala Ser Glu Ser
145                 150                 155                 160

agc gcc tct tcc gac ggc ccc cat cca gtc atc acc ccg tca cgg gcc 528
Ser Ala Ser Ser Asp Gly Pro His Pro Val Ile Thr Pro Ser Arg Ala
                165                 170                 175

tca gag agc agc gcc tct tcc gac ggc ccc cat cca gtc atc acc ccc 576
Ser Glu Ser Ser Ala Ser Ser Asp Gly Pro His Pro Val Ile Thr Pro
            180                 185                 190

tca tgg tcc ccg gga tct gat gtc act ctc ctc gct gaa gcc ctg gtg 624

Ser Trp Ser Pro Gly Ser Asp Val Thr Leu Leu Ala Glu Ala Leu Val
        195                 200                 205

act gtc aca aac atc gag gtt att aat tgc agc atc aca gaa ata gaa 672
Thr Val Thr Asn Ile Glu Val Ile Asn Cys Ser Ile Thr Glu Ile Glu
    210                 215                 220

aca aca act tcc agc atc cct ggg gcc tca gac ata gat ctc atc ccc 720
Thr Thr Thr Ser Ser Ile Pro Gly Ala Ser Asp Ile Asp Leu Ile Pro
225                 230                 235                 240

acg gaa ggg gtg aag gcc tcg tcc acc tcc gat cca cca gct ctg cct 768
Thr Glu Gly Val Lys Ala Ser Ser Thr Ser Asp Pro Pro Ala Leu Pro
                245                 250                 255

gac tcc act gaa gca aaa cca cac atc act gag gtc aca gcc tct gcc 816
Asp Ser Thr Glu Ala Lys Pro His Ile Thr Glu Val Thr Ala Ser Ala
            260                 265                 270

gag acc ctg tcc aca gcc ggc acc aca gag tca gct gca cct cat gcc 864
Glu Thr Leu Ser Thr Ala Gly Thr Thr Glu Ser Ala Ala Pro His Ala
        275                 280                 285

acg gtt ggg acc cca ctc ccc act aac agc gcc aca gaa aga gaa gtg 912
Thr Val Gly Thr Pro Leu Pro Thr Asn Ser Ala Thr Glu Arg Glu Val
    290                 295                 300

aca gca ccc ggg gcc acg acc ctc agt gga gct ctg gtc aca gtt agc 960
Thr Ala Pro Gly Ala Thr Thr Leu Ser Gly Ala Leu Val Thr Val Ser
305                 310                 315                 320

agg aat ccc ctg gaa gaa acc tca gcc ctc tct gtt gag aca cca agt 1008
Arg Asn Pro Leu Glu Glu Thr Ser Ala Leu Ser Val Glu Thr Pro Ser
                325                 330                 335

tac gtc aaa gtc tca gga gca gct ccg gtc tcc ata gag gct ggg tca 1056
Tyr Val Lys Val Ser Gly Ala Ala Pro Val Ser Ile Glu Ala Gly Ser
            340                 345                 350

gca gtg ggc aaa aca act tcc ttt gct ggg agc tct gct tcc tcc tac 1104
Ala Val Gly Lys Thr Thr Ser Phe Ala Gly Ser Ser Ala Ser Ser Tyr
        355                 360                 365

agc ccc tcg gaa gcc gcc ctc aag aac ttc acc cct tca gag aca ccg 1152
Ser Pro Ser Glu Ala Ala Leu Lys Asn Phe Thr Pro Ser Glu Thr Pro
    370                 375                 380

acc atg gac atc gca acc aag ggg ccc ttc ccc acc agc agg gac cct 1200
Thr Met Asp Ile Ala Thr Lys Gly Pro Phe Pro Thr Ser Arg Asp Pro
385                 390                 395                 400

ctt cct tct gtc cct ccg act aca acc aac agc agc cga ggg acg aac 1248

Leu Pro Ser Val Pro Pro Thr Thr Thr Asn Ser Ser Arg Gly Thr Asn
                405                 410                 415

agc acc tta gcc aag atc aca acc tca gcg aag acc acg atg aag ccc 1296
Ser Thr Leu Ala Lys Ile Thr Thr Ser Ala Lys Thr Thr Met Lys Pro
            420                 425                 430

cca aca gcc acg ccc acg act gcc cgg acg agg ccg acc aca gac gtg 1344
Pro Thr Ala Thr Pro Thr Thr Ala Arg Thr Arg Pro Thr Thr Asp Val
        435                 440                 445

agt gca ggt gaa aat gga ggt ttc ctc ctc ctg cgg ctg agt gtg gct 1392
Ser Ala Gly Glu Asn Gly Gly Phe Leu Leu Leu Arg Leu Ser Val Ala
    450                 455                 460

tcc ccg gaa gac ctc act gac ccc aga gtg gca gaa agg ctg atg cag 1440
Ser Pro Glu Asp Leu Thr Asp Pro Arg Val Ala Glu Arg Leu Met Gln
465                 470                 475                 480

cag ctc cac cgg gaa ctc cac gcc cac gcg cct cac ttc cag gtc tcc 1488
Gln Leu His Arg Glu Leu His Ala His Ala Pro His Phe Gln Val Ser
                485                 490                 495

tta ctg cgt gtc agg aga ggc taa 1512
Leu Leu Arg Val Arg Arg Gly
            500

<210> 10

<211> 503

<212> PRT

<213> Homo sapiens

<400> 10

Met Thr Thr Asp Asp Thr Glu Val Pro Ala Met Thr Leu Ala Pro Gly

1               5                   10                  15

His Ala Ala Leu Glu Thr Gln Thr Leu Ser Ala Glu Thr Ser Ser Arg

            20                  25                  30

Ala Ser Thr Pro Ala Gly Pro Ile Pro Glu Ala Glu Thr Arg Gly Ala

        35                  40                  45

Lys Arg Ile Ser Pro Ala Arg Glu Thr Arg Ser Phe Thr Lys Thr Ser

    50                  55                  60

Pro Asn Phe Met Val Leu Ile Ala Thr Ser Val Glu Thr Ser Ala Ala

65                  70                  75                  80

Ser Gly Ser Pro Glu Gly Ala Gly Met Thr Thr Val Gln Thr Ile Thr

                85                  90                  95

Gly Ser Asp Pro Glu Glu Ala Ile Phe Asp Thr Leu Cys Thr Asp Asp

            100                 105                 110

Ser Ser Glu Glu Ala Lys Thr Leu Thr Met Asp Ile Leu Thr Leu Ala

        115                 120                 125

His Thr Ser Thr Glu Ala Lys Gly Leu Ser Ser Glu Ser Ser Ala Ser

    130                 135                 140

Ser Asp Gly Pro His Pro Val Ile Thr Pro Ser Arg Ala Ser Glu Ser
145                 150                 155                 160
Ser Ala Ser Ser Asp Gly Pro His Pro Val Ile Thr Pro Ser Arg Ala
                165                 170                 175
Ser Glu Ser Ser Ala Ser Ser Asp Gly Pro His Pro Val Ile Thr Pro
            180                 185                 190
Ser Trp Ser Pro Gly Ser Asp Val Thr Leu Leu Ala Glu Ala Leu Val
        195                 200                 205
Thr Val Thr Asn Ile Glu Val Ile Asn Cys Ser Ile Thr Glu Ile Glu
    210                 215                 220
Thr Thr Thr Ser Ser Ile Pro Gly Ala Ser Asp Ile Asp Leu Ile Pro
225                 230                 235                 240
Thr Glu Gly Val Lys Ala Ser Ser Thr Ser Asp Pro Pro Ala Leu Pro
                245                 250                 255
Asp Ser Thr Glu Ala Lys Pro His Ile Thr Glu Val Thr Ala Ser Ala
            260                 265                 270
Glu Thr Leu Ser Thr Ala Gly Thr Thr Glu Ser Ala Ala Pro His Ala
        275                 280                 285
Thr Val Gly Thr Pro Leu Pro Thr Asn Ser Ala Thr Glu Arg Glu Val
    290                 295                 300
Thr Ala Pro Gly Ala Thr Thr Leu Ser Gly Ala Leu Val Thr Val Ser
305                 310                 315                 320
Arg Asn Pro Leu Glu Glu Thr Ser Ala Leu Ser Val Glu Thr Pro Ser
                325                 330                 335
Tyr Val Lys Val Ser Gly Ala Ala Pro Val Ser Ile Glu Ala Gly Ser
            340                 345                 350

Ala Val Gly Lys Thr Thr Ser Phe Ala Gly Ser Ser Ala Ser Ser Tyr
        355                 360                 365
Ser Pro Ser Glu Ala Ala Leu Lys Asn Phe Thr Pro Ser Glu Thr Pro
    370                 375                 380
Thr Met Asp Ile Ala Thr Lys Gly Pro Phe Pro Thr Ser Arg Asp Pro
385                 390                 395                 400
Leu Pro Ser Val Pro Pro Thr Thr Thr Asn Ser Ser Arg Gly Thr Asn
                405                 410                 415
Ser Thr Leu Ala Lys Ile Thr Thr Ser Ala Lys Thr Thr Met Lys Pro
            420                 425                 430
Pro Thr Ala Thr Pro Thr Thr Ala Arg Thr Arg Pro Thr Thr Asp Val
        435                 440                 445
Ser Ala Gly Glu Asn Gly Gly Phe Leu Leu Leu Arg Leu Ser Val Ala
    450                 455                 460
Ser Pro Glu Asp Leu Thr Asp Pro Arg Val Ala Glu Arg Leu Met Gln
465                 470                 475                 480
Gln Leu His Arg Glu Leu His Ala His Ala Pro His Phe Gln Val Ser
                485                 490                 495
Leu Leu Arg Val Arg Arg Gly
            500

<210> 11
<211> 1971
<212> DNA

<213> Mus musculus

<220>
<221> CDS
<222> (1)..(1971)

<400> 11
atg ggc tct gtc tgg ggc ctg gct gtg ccc ctc ctt gtc ttc tgc tgg 48
Met Gly Ser Val Trp Gly Leu Ala Val Pro Leu Leu Val Phe Cys Trp
1               5                   10                  15

aag gtt ggc gtc agt gtg agc tct cca ggt ctc gac att agc aga tca 96
Lys Val Gly Val Ser Val Ser Ser Pro Gly Leu Asp Ile Ser Arg Ser
            20                  25                  30

gtt cct ttg gtg aca acc aac aac atg gaa gtg tcc acc ttc act caa 144
Val Pro Leu Val Thr Thr Asn Asn Met Glu Val Ser Thr Phe Thr Gln
        35                  40                  45

agg gac agg ccc agc tca gag aga gct ttc cag acc acc aac ctg att 192
Arg Asp Arg Pro Ser Ser Glu Arg Ala Phe Gln Thr Thr Asn Leu Ile
    50                  55                  60

cag tat gta cct cta gac aca caa act ctc agc act gaa tct gct tct 240
Gln Tyr Val Pro Leu Asp Thr Gln Thr Leu Ser Thr Glu Ser Ala Ser
65                  70                  75                  80

aat gct tta agt gca act agt atc agt tca gag gtc aat tcc aga gac 288
Asn Ala Leu Ser Ala Thr Ser Ile Ser Ser Glu Val Asn Ser Arg Asp
                85                  90                  95

act cag gcc acc tct ttt gta aca aag acc agg aag aca cat act acc 336
Thr Gln Ala Thr Ser Phe Val Thr Lys Thr Arg Lys Thr His Thr Thr
            100                 105                 110

act cct gca gca tca tcc cta gag gcc cag acc acc tct cct aat ttg 384
Thr Pro Ala Ala Ser Ser Leu Glu Ala Gln Thr Thr Ser Pro Asn Leu
        115                 120                 125

tca acc ctg aat att cag att acc tcc cca ata gca tcg tcc ctg gat 432
Ser Thr Leu Asn Ile Gln Ile Thr Ser Pro Ile Ala Ser Ser Leu Asp
    130                 135                 140

gct cag acc att ttt cct gta tca tta tcc ctg agt acc cag acc act 480
Ala Gln Thr Ile Phe Pro Val Ser Leu Ser Leu Ser Thr Gln Thr Thr
145                 150                 155                 160

tct cct gca cca tca ttc ctg gat acc caa acc act tcc cct gaa cca 528
Ser Pro Ala Pro Ser Phe Leu Asp Thr Gln Thr Thr Ser Pro Glu Pro
                165                 170                 175

tca tct ctg acc act tcc cct gca cca tca tct ctg atc act tct cct 576

Ser Ser Leu Thr Thr Ser Pro Ala Pro Ser Ser Leu Ile Thr Ser Pro
            180                 185                 190

act cca tca tct cta act ata tcc cct gca ccg tca ttc ctg gat acc 624
Thr Pro Ser Ser Leu Thr Ile Ser Pro Ala Pro Ser Phe Leu Asp Thr
        195                 200                 205

cag acc act tcc cct gca cta tca tct ctg gct act tcc cct gca cca 672
Gln Thr Thr Ser Pro Ala Leu Ser Ser Leu Ala Thr Ser Pro Ala Pro
    210                 215                 220

tca tcc cta gat acc cag acc atc tcg cct att gaa ttg acc cta aag 720
Ser Ser Leu Asp Thr Gln Thr Ile Ser Pro Ile Glu Leu Thr Leu Lys
225                 230                 235                 240

acc caa acc att tcc act gta acg gag acc aga acg gtt tca ata aga 768
Thr Gln Thr Ile Ser Thr Val Thr Glu Thr Arg Thr Val Ser Ile Arg
                245                 250                 255

ata cct tct gac ctc aag gtt atg cac acc atc cct aca gag aca ttg 816
Ile Pro Ser Asp Leu Lys Val Met His Thr Ile Pro Thr Glu Thr Leu
            260                 265                 270

aca cca agc aac agc ccc aga act ggg atg agc act gtc caa act ggc 864
Thr Pro Ser Asn Ser Pro Arg Thr Gly Met Ser Thr Val Gln Thr Gly
        275                 280                 285

aca gtg tgg gac ccc ata gaa gcc atc ttt gac acc ctt tgt acc gat 912
Thr Val Trp Asp Pro Ile Glu Ala Ile Phe Asp Thr Leu Cys Thr Asp
    290                 295                 300

gac agc tct gaa gag gca agg aag atc aca gtt gac ctc ctg acg ttg 960
Asp Ser Ser Glu Glu Ala Arg Lys Ile Thr Val Asp Leu Leu Thr Leu
305                 310                 315                 320

gca cac acc tcc acg gaa gtg gag cac ctg tct tca gag agc agc tct 1008
Ala His Thr Ser Thr Glu Val Glu His Leu Ser Ser Glu Ser Ser Ser
                325                 330                 335

tcc tct gac agt tca gct ggg gtc ctc agc agc tca cga gtc ctg gga 1056
Ser Ser Asp Ser Ser Ala Gly Val Leu Ser Ser Ser Arg Val Leu Gly
            340                 345                 350

ccc gac agt gca act cca gcc aag ggg ttg gtt gcc ttc aac atc act 1104
Pro Asp Ser Ala Thr Pro Ala Lys Gly Leu Val Ala Phe Asn Ile Thr
        355                 360                 365

cat att aaa ctc agc acc tgc atc aca gaa ata gaa aca acc atc acc 1152
His Ile Lys Leu Ser Thr Cys Ile Thr Glu Ile Glu Thr Thr Ile Thr
    370                 375                 380

atc tct ggg gcc cca ggc gtg agc ctc agc cct aca gaa gcg acg gca 1200

Ile Ser Gly Ala Pro Gly Val Ser Leu Ser Pro Thr Glu Ala Thr Ala
385                 390                 395                 400

gct ttg ttc act tct gag ata tta act ttg cct cca ccc act gag gca     1248
Ala Leu Phe Thr Ser Glu Ile Leu Thr Leu Pro Pro Pro Thr Glu Ala
                405                 410                 415

aaa cca att ttc ccc gag acc aca agc tta tct ggg atc ctg tca act     1296
Lys Pro Ile Phe Pro Glu Thr Thr Ser Leu Ser Gly Ile Leu Ser Thr
            420                 425                 430

gcc ggc acc ccg gcc ctt gcc acc acc ctt gag ggg aca gtc tcc act     1344
Ala Gly Thr Pro Ala Leu Ala Thr Thr Leu Glu Gly Thr Val Ser Thr
        435                 440                 445

agt gca atc aca gaa agc gaa aca gca gtg gcc cag act ctt acc tct     1392
Ser Ala Ile Thr Glu Ser Glu Thr Ala Val Ala Gln Thr Leu Thr Ser
    450                 455                 460

gtt gga act tcg gtg aca gtt aga agg aac ccc ctg gaa aac act tca     1440
Val Gly Thr Ser Val Thr Val Arg Arg Asn Pro Leu Glu Asn Thr Ser
465                 470                 475                 480

aca ctc tct att gag aca caa agc cac act gag gtc ttg ggc acc atc     1488
Thr Leu Ser Ile Glu Thr Gln Ser His Thr Glu Val Leu Gly Thr Ile
                485                 490                 495

aca gtc ccc atg gtt gct gga tca aca atg gga gaa aca gcc tcc ttc 1536
Thr Val Pro Met Val Ala Gly Ser Thr Met Gly Glu Thr Ala Ser Phe
            500                 505                 510

gtg agt ttc acg gct ctc gac agc agc agt ctc tca gtg gtg gtc acc 1584
Val Ser Phe Thr Ala Leu Asp Ser Ser Ser Leu Ser Val Val Val Thr
        515                 520                 525

acc gag agc tct gct acc tca gag act tta acc aca ggc aat aca acc 1632
Thr Glu Ser Ser Ala Thr Ser Glu Thr Leu Thr Thr Gly Asn Thr Thr
    530                 535                 540

aac agc tcc ttc ctc aca gaa agc cac cct cct ttt tct atc tat tca 1680
Asn Ser Ser Phe Leu Thr Glu Ser His Pro Pro Phe Ser Ile Tyr Ser
545                 550                 555                 560

act aca gct agc acc agc aaa aac cca aac atc acc tta acc aag acc 1728
Thr Thr Ala Ser Thr Ser Lys Asn Pro Asn Ile Thr Leu Thr Lys Thr
                565                 570                 575

aca gcc tca cca aag ccc ccg acg cat cct aca aca tct gca tcc act 1776
Thr Ala Ser Pro Lys Pro Pro Thr His Pro Thr Thr Ser Ala Ser Thr
            580                 585                 590

gct tgg ata agg aaa acc aca aaa cat gat cca ggt gaa gat gga ggc 1824

Ala Trp Ile Arg Lys Thr Thr Lys His Asp Pro Gly Glu Asp Gly Gly
        595                 600                 605

ttc ctc ctt gta cgg ctg acc gtg gcc tcc cct aag gac ctc act gag 1872
Phe Leu Leu Val Arg Leu Thr Val Ala Ser Pro Lys Asp Leu Thr Glu
    610                 615                 620

cac aac gcc aga gag aag ctg atg aac cag ctc cgc cgt gaa ctg cac 1920
His Asn Ala Arg Glu Lys Leu Met Asn Gln Leu Arg Arg Glu Leu His
625                 630                 635                 640

gcc cgc atg cca ctt gtc cac atg tcc ttc ctg agc atc agg agg ggt 1968
Ala Arg Met Pro Leu Val His Met Ser Phe Leu Ser Ile Arg Arg Gly
                645                 650                 655

tga 1971

<210> 12
<211> 656
<212> PRT
<213> Mus musculus

<400> 12
Met Gly Ser Val Trp Gly Leu Ala Val Pro Leu Leu Val Phe Cys Trp

1                   5                   10                  15

Lys Val Gly Val Ser Val Ser Ser Pro Gly Leu Asp Ile Ser Arg Ser

                20                  25                  30

Val Pro Leu Val Thr Thr Asn Asn Met Glu Val Ser Thr Phe Thr Gln

            35                  40                  45

Arg Asp Arg Pro Ser Ser Glu Arg Ala Phe Gln Thr Thr Asn Leu Ile

        50                  55                  60

Gln Tyr Val Pro Leu Asp Thr Gln Thr Leu Ser Thr Glu Ser Ala Ser

65                  70                  75                  80

Asn Ala Leu Ser Ala Thr Ser Ile Ser Ser Glu Val Asn Ser Arg Asp

                85                  90                  95

Thr Gln Ala Thr Ser Phe Val Thr Lys Thr Arg Lys Thr His Thr Thr

            100                 105                 110

Thr Pro Ala Ala Ser Ser Leu Glu Ala Gln Thr Thr Ser Pro Asn Leu

        115                 120                 125

Ser Thr Leu Asn Ile Gln Ile Thr Ser Pro Ile Ala Ser Ser Leu Asp

    130                 135                 140

Ala Gln Thr Ile Phe Pro Val Ser Leu Ser Leu Ser Thr Gln Thr Thr

145                 150                 155                 160

Ser Pro Ala Pro Ser Phe Leu Asp Thr Gln Thr Thr Ser Pro Glu Pro

                165                 170                 175

Ser Ser Leu Thr Thr Ser Pro Ala Pro Ser Ser Leu Ile Thr Ser Pro

            180                 185                 190

Thr Pro Ser Ser Leu Thr Ile Ser Pro Ala Pro Ser Phe Leu Asp Thr

        195                 200                 205

Gln Thr Thr Ser Pro Ala Leu Ser Ser Leu Ala Thr Ser Pro Ala Pro

    210                 215                 220
Ser Ser Leu Asp Thr Gln Thr Ile Ser Pro Ile Glu Leu Thr Leu Lys
225                 230                 235                 240
Thr Gln Thr Ile Ser Thr Val Thr Glu Thr Arg Thr Val Ser Ile Arg
                245                 250                 255
Ile Pro Ser Asp Leu Lys Val Met His Thr Ile Pro Thr Glu Thr Leu
            260                 265                 270
Thr Pro Ser Asn Ser Pro Arg Thr Gly Met Ser Thr Val Gln Thr Gly
        275                 280                 285
Thr Val Trp Asp Pro Ile Glu Ala Ile Phe Asp Thr Leu Cys Thr Asp
    290                 295                 300
Asp Ser Ser Glu Glu Ala Arg Lys Ile Thr Val Asp Leu Leu Thr Leu
305                 310                 315                 320
Ala His Thr Ser Thr Glu Val Glu His Leu Ser Ser Glu Ser Ser Ser
                325                 330                 335
Ser Ser Asp Ser Ser Ala Gly Val Leu Ser Ser Ser Arg Val Leu Gly
            340                 345                 350
Pro Asp Ser Ala Thr Pro Ala Lys Gly Leu Val Ala Phe Asn Ile Thr
        355                 360                 365
His Ile Lys Leu Ser Thr Cys Ile Thr Glu Ile Glu Thr Thr Ile Thr
    370                 375                 380
Ile Ser Gly Ala Pro Gly Val Ser Leu Ser Pro Thr Glu Ala Thr Ala
385                 390                 395                 400
Ala Leu Phe Thr Ser Glu Ile Leu Thr Leu Pro Pro Pro Thr Glu Ala
                405                 410                 415
Lys Pro Ile Phe Pro Glu Thr Thr Ser Leu Ser Gly Ile Leu Ser Thr

            420                 425                 430
Ala Gly Thr Pro Ala Leu Ala Thr Thr Leu Glu Gly Thr Val Ser Thr
        435                 440                 445
Ser Ala Ile Thr Glu Ser Glu Thr Ala Val Ala Gln Thr Leu Thr Ser
    450                 455                 460
Val Gly Thr Ser Val Thr Val Arg Arg Asn Pro Leu Glu Asn Thr Ser
465                 470                 475                 480
Thr Leu Ser Ile Glu Thr Gln Ser His Thr Glu Val Leu Gly Thr Ile
                485                 490                 495
Thr Val Pro Met Val Ala Gly Ser Thr Met Gly Glu Thr Ala Ser Phe
            500                 505                 510
Val Ser Phe Thr Ala Leu Asp Ser Ser Ser Leu Ser Val Val Val Thr
        515                 520                 525
Thr Glu Ser Ser Ala Thr Ser Glu Thr Leu Thr Thr Gly Asn Thr Thr
    530                 535                 540
Asn Ser Ser Phe Leu Thr Glu Ser His Pro Pro Phe Ser Ile Tyr Ser
545                 550                 555                 560
Thr Thr Ala Ser Thr Ser Lys Asn Pro Asn Ile Thr Leu Thr Lys Thr
                565                 570                 575
Thr Ala Ser Pro Lys Pro Pro Thr His Pro Thr Thr Ser Ala Ser Thr
            580                 585                 590
Ala Trp Ile Arg Lys Thr Thr Lys His Asp Pro Gly Glu Asp Gly Gly
        595                 600                 605
Phe Leu Leu Val Arg Leu Thr Val Ala Ser Pro Lys Asp Leu Thr Glu
    610                 615                 620
His Asn Ala Arg Glu Lys Leu Met Asn Gln Leu Arg Arg Glu Leu His

625                 630                 635                 640

Ala Arg Met Pro Leu Val His Met Ser Phe Leu Ser Ile Arg Arg Gly

                645                 650                 655

<210> 13

<211> 491

<212> DNA

<213> Rattus norvegicus

<220>

<221> CDS

<222> (3)..(491)

<400> 13

ct gag gtt ttt ggt acc atc aca gtc ccc acg gtt gct gga tca aca     47

   Glu Val Phe Gly Thr Ile Thr Val Pro Thr Val Ala Gly Ser Thr

   1               5                   10                  15

gta gga gaa gca acc tct ctt gtg agt tcc aca gct cta gac agc agt     95

Val Gly Glu Ala Thr Ser Leu Val Ser Ser Thr Ala Leu Asp Ser Ser

                20                  25                  30

ctc tca gca gtg gtc acc acc aag ggc tct gct tcc tca gag act tta     143

Leu Ser Ala Val Val Thr Thr Lys Gly Ser Ala Ser Ser Glu Thr Leu

            35                  40                  45

acc aca gac aat aca acc aac agc tcc ttc ctc aca ggg agt cgc cct 191
Thr Thr Asp Asn Thr Thr Asn Ser Ser Phe Leu Thr Gly Ser Arg Pro
        50                  55                  60

cct tct tta atc tat tcg aca aca gct agc acc agt gaa aga aca aac 239
Pro Ser Leu Ile Tyr Ser Thr Thr Ala Ser Thr Ser Glu Arg Thr Asn
    65                  70                  75

gtc act tta acc aag acc aca gcc tca ccg aag acc ccg atg aat ccc 287
Val Thr Leu Thr Lys Thr Thr Ala Ser Pro Lys Thr Pro Met Asn Pro
80                  85                  90                  95

gca ccc act gct tgg aca agg aaa acc aca gag cac gat cca ggt ata 335
Ala Pro Thr Ala Trp Thr Arg Lys Thr Thr Glu His Asp Pro Gly Ile
                100                 105                 110

aat gga ggc ttc ctc ctt gta cgg ctg acc gtg gcc tcc cct aag gac 383
Asn Gly Gly Phe Leu Leu Val Arg Leu Thr Val Ala Ser Pro Lys Asp
            115                 120                 125

ctc act gag cac gtc acc aga gag aag ctg atg cac cag ctc cgc cgt 431
Leu Thr Glu His Val Thr Arg Glu Lys Leu Met His Gln Leu Arg Arg
        130                 135                 140

gaa ctg cac acc cat atg cca ctt gtc caa gtg tcc tta ctg agc atc 479
Glu Leu His Thr His Met Pro Leu Val Gln Val Ser Leu Leu Ser Ile
    145                 150                 155

agg agg agc tga 491
Arg Arg Ser
160

<210> 14
<211> 162
<212> PRT
<213> Rattus norvegicus

<400> 14
Glu Val Phe Gly Thr Ile Thr Val Pro Thr Val Ala Gly Ser Thr Val
1               5                   10                  15
Gly Glu Ala Thr Ser Leu Val Ser Ser Thr Ala Leu Asp Ser Ser Leu
            20                  25                  30
Ser Ala Val Val Thr Thr Lys Gly Ser Ala Ser Ser Glu Thr Leu Thr
        35                  40                  45
Thr Asp Asn Thr Thr Asn Ser Ser Phe Leu Thr Gly Ser Arg Pro Pro
    50                  55                  60
Ser Leu Ile Tyr Ser Thr Thr Ala Ser Thr Ser Glu Arg Thr Asn Val
65                  70                  75                  80
Thr Leu Thr Lys Thr Thr Ala Ser Pro Lys Thr Pro Met Asn Pro Ala

                85                  90                  95

Pro Thr Ala Trp Thr Arg Lys Thr Thr Glu His Asp Pro Gly Ile Asn

            100                 105                 110

Gly Gly Phe Leu Leu Val Arg Leu Thr Val Ala Ser Pro Lys Asp Leu

        115                 120                 125

Thr Glu His Val Thr Arg Glu Lys Leu Met His Gln Leu Arg Arg Glu

    130                 135                 140

Leu His Thr His Met Pro Leu Val Gln Val Ser Leu Leu Ser Ile Arg

145                 150                 155                 160

Arg Ser

<210> 15

<211> 53

<212> PRT

<213> Homo sapiens

<400> 15

Gly Glu Asn Gly Gly Phe Leu Leu Leu Arg Leu Ser Val Ala Ser Pro

1               5                   10                  15

Glu Asp Leu Thr Asp Pro Arg Val Ala Glu Arg Leu Met Gln Gln Leu

            20                  25                  30

His Arg Glu Leu His Ala His Ala Pro His Phe Gln Val Ser Leu Leu

        35                  40                  45

Arg Val Arg Arg Gly

    50

<210> 16

<211> 993

<212> PRT

<213> Escherichia coli

<400> 16

Met Thr Met Ile Thr Asp Ser Leu Ala Val Val Ala Arg Thr Asp Arg

1               5                   10                  15

Pro Ser Gln Gln Leu Arg Ser Leu Asn Gly Glu Trp Arg Phe Ala Trp

            20                  25                  30

Phe Pro Ala Pro Glu Ala Val Pro Glu Ser Trp Leu Glu Cys Asp Leu

        35                  40                  45

Pro Glu Ala Asp Thr Val Val Val Pro Ser Asn Trp Gln Met His Gly

    50                  55                  60

Tyr Asp Ala Pro Ile Tyr Thr Asn Val Thr Tyr Pro Ile Thr Val Asn

65                  70                  75                  80

Pro Pro Phe Val Pro Thr Glu Asn Pro Thr Gly Cys Tyr Ser Leu Thr
                85                  90                  95
Phe Asn Val Asp Glu Ser Trp Leu Gln Glu Gly Gln Thr Arg Ile Ile
            100                 105                 110
Phe Asp Gly Val Asn Ser Ala Phe His Leu Trp Cys Asn Gly Arg Trp
        115                 120                 125
Val Gly Tyr Gly Gln Asp Ser Arg Leu Pro Ser Glu Phe Asp Leu Ser
    130                 135                 140
Ala Phe Leu Arg Ala Gly Glu Asn Arg Leu Ala Val Met Val Leu Arg
145                 150                 155                 160
Trp Ser Asp Gly Ser Tyr Leu Glu Asp Gln Asp Met Trp Arg Met Ser
                165                 170                 175
Gly Ile Phe Arg Asp Val Ser Leu Leu His Lys Pro Thr Thr Gln Ile
            180                 185                 190
Ser Asp Phe His Val Ala Thr Arg Phe Asn Asp Asp Phe Ser Arg Ala
        195                 200                 205
Val Leu Glu Ala Glu Val Gln Met Cys Gly Glu Leu Arg Asp Tyr Leu

    210                 215                 220

Arg Val Thr Val Ser Leu Trp Gln Gly Glu Thr Gln Val Ala Ser Gly
225                 230                 235                 240

Thr Ala Pro Phe Gly Gly Glu Ile Ile Asp Glu Arg Gly Gly Tyr Ala
                245                 250                 255

Asp Arg Val Thr Leu Arg Leu Asn Val Glu Asn Pro Lys Leu Trp Ser
            260                 265                 270

Ala Glu Ile Pro Asn Leu Tyr Arg Ala Val Val Glu Leu His Thr Ala
        275                 280                 285

Asp Gly Thr Leu Ile Glu Ala Glu Ala Cys Asp Val Gly Phe Arg Glu
    290                 295                 300

Val Arg Ile Glu Asn Gly Leu Leu Leu Leu Asn Gly Lys Pro Leu Leu
305                 310                 315                 320

Ile Arg Gly Val Asn Arg His Glu His His Pro Leu His Gly Gln Val
                325                 330                 335

Met Asp Glu Gln Thr Met Val Gln Asp Ile Leu Leu Met Lys Gln Asn
            340                 345                 350

Asn Phe Asn Ala Val Arg Cys Ser His Tyr Pro Asn His Pro Leu Trp
        355                 360                 365

Tyr Thr Leu Cys Asp Arg Tyr Gly Leu Tyr Val Val Asp Glu Ala Asn
    370                 375                 380

Ile Glu Thr His Gly Met Val Pro Met Asn Arg Leu Thr Asp Asp Pro
385                 390                 395                 400

Arg Trp Leu Pro Ala Met Ser Glu Arg Val Thr Arg Met Val Gln Arg
                405                 410                 415

Asp Arg Asn His Pro Ser Val Ile Ile Trp Ser Leu Gly Asn Glu Ser
            420                 425                 430

Gly His Gly Ala Asn His Asp Ala Leu Tyr Arg Trp Ile Lys Ser Val
        435                 440                 445

Asp Pro Ser Arg Pro Val Gln Tyr Glu Gly Gly Gly Ala Asp Thr Thr
    450                 455                 460

Ala Thr Asp Ile Ile Cys Pro Met Tyr Ala Arg Val Asp Glu Asp Gln
465                 470                 475                 480

Pro Phe Pro Ala Val Pro Lys Trp Ser Ile Lys Lys Trp Leu Ser Leu
                485                 490                 495

Pro Gly Glu Thr Arg Pro Leu Ile Leu Cys Glu Tyr Ala His Ala Met
            500                 505                 510
Gly Asn Ser Leu Gly Gly Phe Ala Lys Tyr Trp Gln Ala Phe Arg Gln
        515                 520                 525
Tyr Pro Arg Leu Gln Gly Gly Phe Val Trp Asp Trp Val Asp Gln Ser
    530                 535                 540
Leu Ile Lys Tyr Asp Glu Asn Gly Asn Pro Trp Ser Ala Tyr Gly Gly
545                 550                 555                 560
Asp Phe Gly Asp Thr Pro Asn Asp Arg Gln Phe Cys Met Asn Gly Leu
                565                 570                 575
Val Phe Ala Asp Arg Thr Pro His Pro Ala Leu Thr Glu Ala Lys His
            580                 585                 590
Gln Gln Gln Phe Phe Gln Phe Arg Leu Ser Gly Gln Thr Ile Glu Val
        595                 600                 605
Thr Ser Glu Tyr Leu Phe Arg His Ser Asp Asn Glu Leu Leu His Trp
    610                 615                 620
Met Val Ala Leu Asp Gly Lys Pro Leu Ala Ser Gly Glu Val Pro Leu

625                 630                 635                 640

Asp Val Ala Pro Gln Gly Lys Gln Leu Ile Glu Leu Pro Glu Leu Pro

                645                 650                 655

Gln Pro Glu Ser Ala Gly Gln Leu Trp Leu Thr Val Arg Val Val Gln

            660                 665                 670

Pro Asn Ala Thr Ala Trp Ser Glu Ala Gly His Ile Ser Ala Trp Gln

        675                 680                 685

Gln Trp Arg Leu Ala Glu Asn Leu Ser Val Thr Leu Pro Ala Ala Ser

    690                 695                 700

His Ala Ile Pro His Leu Thr Thr Ser Glu Met Asp Phe Cys Ile Glu

705                 710                 715                 720

Leu Gly Asn Lys Arg Trp Gln Phe Asn Arg Gln Ser Gly Phe Leu Ser

                725                 730                 735

Gln Met Trp Ile Gly Asp Lys Lys Gln Leu Leu Thr Pro Leu Arg Asp

            740                 745                 750

Gln Phe Thr Arg Ala Pro Leu Asp Asn Asp Ile Gly Val Ser Glu Ala

        755                 760                 765

Thr Arg Ile Asp Pro Asn Ala Trp Val Glu Arg Trp Lys Ala Ala Gly
    770                 775                 780

His Tyr Gln Ala Glu Ala Ala Leu Leu Gln Cys Thr Ala Asp Thr Leu
785                 790                 795                 800

Ala Asp Ala Val Leu Ile Thr Thr Ala His Ala Trp Gln His Gln Gly
                805                 810                 815

Lys Thr Leu Phe Ile Ser Arg Lys Thr Tyr Arg Ile Asp Gly Ser Gly
            820                 825                 830

Gln Met Ala Ile Thr Val Asp Val Glu Val Ala Ser Asp Thr Pro His
        835                 840                 845

Pro Ala Arg Ile Gly Leu Asn Cys Gln Leu Ala Gln Val Ala Glu Arg
    850                 855                 860

Val Asn Trp Leu Gly Leu Gly Pro Gln Glu Asn Tyr Pro Asp Arg Leu
865                 870                 875                 880

Thr Ala Ala Cys Phe Asp Arg Trp Asp Leu Pro Leu Ser Asp Met Tyr
                885                 890                 895

Thr Pro Tyr Val Phe Pro Ser Glu Asn Gly Leu Arg Cys Gly Thr Arg
            900                 905                 910

Glu Leu Asn Tyr Gly Pro His Gln Trp Arg Gly Asp Phe Gln Phe Asn
        915                 920                 925
Ile Ser Arg Tyr Ser Gln Gln Gln Leu Met Glu Thr Ser His Arg His
    930                 935                 940
Leu Leu His Ala Glu Glu Gly Thr Trp Leu Asn Ile Asp Gly Phe His
945                 950                 955                 960
Met Gly Ile Gly Gly Asp Asp Ser Trp Ser Pro Ser Val Ser Ala Glu
                965                 970                 975
Phe Gln Leu Ser Ala Gly Arg Tyr His Tyr Gln Leu Val Trp Cys Gln
            980                 985                 990
Lys

<210> 17
<211> 789
<212> PRT
<213> Escherichia coli

<400> 17

Met Thr Met Ile Thr Asp Ser Leu Ala Val Val Leu Gln Arg Arg Asp
1               5                   10                  15

Trp Glu Asn Pro Gly Val Thr Gln Leu Asn Arg Leu Ala Ala His Pro
            20                  25                  30

Pro Phe Ala Ser Trp Arg Asn Ser Glu Glu Ala Arg Thr Asp Arg Pro
        35                  40                  45

Ser Gln Gln Leu Arg Ser Leu Asn Gly Glu Trp Arg Phe Ala Trp Phe
    50                  55                  60

Pro Ala Pro Glu Ala Val Pro Glu Ser Trp Leu Glu Cys Asp Leu Pro
65                  70                  75                  80

Glu Ala Asp Thr Val Val Val Pro Ser Asn Trp Gln Met His Gly Tyr
                85                  90                  95

Asp Ala Pro Ile Tyr Thr Asn Val Thr Tyr Pro Ile Thr Val Asn Pro
            100                 105                 110

Pro Phe Val Pro Thr Glu Asn Pro Thr Gly Cys Tyr Ser Leu Thr Phe
        115                 120                 125

Asn Val Asp Glu Ser Trp Leu Gln Glu Gly Gln Thr Arg Ile Ile Phe
    130                 135                 140

Asp Gly Val Asn Ser Ala Phe His Leu Trp Cys Asn Gly Arg Trp Val
145                 150                 155                 160
Gly Tyr Gly Gln Asp Ser Arg Leu Pro Ser Glu Phe Asp Leu Ser Ala
                165                 170                 175
Phe Leu Arg Ala Gly Glu Asn Arg Leu Ala Val Met Val Leu Arg Trp
            180                 185                 190
Ser Asp Gly Ser Tyr Leu Glu Asp Gln Asp Met Trp Arg Met Ser Gly
        195                 200                 205
Ile Phe Arg Asp Val Ser Leu Leu His Lys Pro Thr Thr Gln Ile Ser
    210                 215                 220
Asp Phe His Val Ala Thr Arg Phe Asn Asp Asp Phe Ser Arg Ala Val
225                 230                 235                 240
Leu Glu Ala Glu Val Gln Met Cys Gly Glu Leu Arg Asp Tyr Leu Arg
                245                 250                 255
Val Thr Val Ser Leu Trp Gln Gly Glu Thr Gln Val Ala Ser Gly Thr
            260                 265                 270
Ala Pro Phe Gly Gly Glu Ile Ile Asp Glu Arg Gly Gly Tyr Ala Asp

        275                 280                 285

Arg Val Thr Leu Arg Leu Asn Val Glu Asn Pro Lys Leu Trp Ser Ala

    290                 295                 300

Glu Ile Pro Asn Leu Tyr Arg Ala Val Val Glu Leu His Thr Ala Asp

305                 310                 315                 320

Gly Thr Leu Ile Glu Ala Glu Ala Cys Asp Val Gly Phe Arg Glu Val

                325                 330                 335

Arg Ile Glu Asn Gly Leu Leu Leu Leu Asn Gly Lys Pro Leu Leu Ile

            340                 345                 350

Arg Gly Val Asn Arg His Glu His His Pro Leu His Gly Gln Val Met

        355                 360                 365

Asp Glu Gln Thr Met Val Gln Asp Ile Leu Leu Met Lys Gln Asn Asn

    370                 375                 380

Phe Asn Ala Val Arg Cys Ser His Tyr Pro Asn His Pro Leu Trp Tyr

385                 390                 395                 400

Thr Leu Cys Asp Arg Tyr Gly Leu Tyr Val Val Asp Glu Ala Asn Ile

                405                 410                 415

Glu Thr His Gly Met Val Pro Met Asn Arg Leu Thr Asp Asp Pro Arg
            420                 425                 430

Trp Leu Pro Ala Met Ser Glu Arg Val Thr Arg Met Val Gln Arg Asp
        435                 440                 445

Arg Asn His Pro Ser Val Ile Ile Trp Ser Leu Gly Asn Glu Ser Gly
    450                 455                 460

His Gly Ala Asn His Asp Ala Leu Tyr Arg Trp Ile Lys Ser Val Asp
465                 470                 475                 480

Pro Ser Arg Pro Val Gln Tyr Glu Gly Gly Gly Ala Asp Thr Thr Ala
                485                 490                 495

Thr Asp Ile Ile Cys Pro Met Tyr Ala Arg Val Asp Glu Asp Gln Pro
            500                 505                 510

Phe Pro Ala Val Pro Lys Trp Ser Ile Lys Lys Trp Leu Ser Leu Pro
        515                 520                 525

Gly Glu Thr Arg Pro Leu Ile Leu Cys Glu Tyr Ala His Ala Met Gly
    530                 535                 540

Asn Ser Leu Gly Gly Phe Ala Lys Tyr Trp Gln Ala Phe Arg Gln Tyr
545                 550                 555                 560

Pro Arg Leu Gln Gly Gly Phe Val Trp Asp Trp Val Asp Gln Ser Leu
                565                 570                 575
Ile Lys Tyr Asp Glu Asn Gly Asn Pro Trp Ser Ala Tyr Gly Gly Asp
            580                 585                 590
Phe Gly Asp Thr Pro Asn Asp Arg Gln Phe Cys Met Asn Gly Leu Val
        595                 600                 605
Phe Ala Asp Arg Thr Pro His Pro Ala Leu Thr Glu Ala Lys His Gln
    610                 615                 620
Gln Gln Phe Phe Gln Phe Arg Leu Ser Gly Gln Thr Ile Glu Val Thr
625                 630                 635                 640
Ser Glu Tyr Leu Phe Arg His Ser Asp Asn Glu Leu Leu His Trp Met
                645                 650                 655
Val Ala Leu Asp Gly Lys Pro Leu Ala Ser Gly Glu Val Pro Leu Asp
            660                 665                 670
Val Ala Pro Gln Gly Lys Gln Leu Ile Glu Leu Pro Glu Leu Pro Gln
        675                 680                 685
Pro Glu Ser Ala Gly Gln Leu Trp Leu Thr Val Arg Val Val Gln Pro

    690                 695                 700

Asn Ala Thr Ala Trp Ser Glu Ala Gly His Ile Ser Ala Trp Gln Gln
705                 710                 715                 720

Trp Arg Leu Ala Glu Asn Leu Ser Val Thr Leu Pro Ala Ala Ser His
                725                 730                 735

Ala Ile Pro His Leu Thr Thr Ser Glu Met Asp Phe Cys Ile Glu Leu
            740                 745                 750

Gly Asn Lys Arg Trp Gln Phe Asn Arg Gln Ser Gly Phe Leu Ser Gln
        755                 760                 765

Met Trp Ile Gly Asp Lys Lys Gln Leu Leu Thr Pro Leu Arg Asp Gln
    770                 775                 780

Phe Thr Arg Ala Pro
785

<210> 18
<211> 31
<212> DNA
<213> Artificial Sequence

<220>

<221> primer_bind

<222> (1)..(31)

<220>

<223> Description of Artificial Sequence: PCR primer

sequence

<400> 18

ttcagaattc tcagagacac cgaccatgga c 31

<210> 19

<211> 28

<212> DNA

<213> Artificial Sequence

<220>

<223> Description of Artificial Sequence: PCR primer

sequence

<220>

<221> primer_bind

<222> (1)..(28)

<400> 19

cacggaattc actgcccgga cgaggccg 28

<210> 20

<211> 31

<212> DNA

<213> Artificial Sequence

<220>

<223> Description of Artificial Sequence: PCR primer

sequence

<220>

<221> primer_bind

<222> (1)..(31)

<400> 20

gggggtcgac ttagcctctc ctgacacgca g 31

<210> 21

<211> 17

<212> PRT

<213> Homo sapiens

<400> 21

Arg Gly Ala Lys Arg Ile Ser Pro Ala Arg Glu Thr Arg Ser Phe Thr
1               5                   10                  15

Lys

<210> 22
<211> 32
<212> DNA
<213> Artificial Sequence

<220>
<223> Description of Artificial Sequence: PCR primer
      sequence

<220>
<221> primer_bind
<222> (1)..(32)

<400> 22
ggggaagctt atgacaacgg acgacacaga ag                                32

<210> 23
<211> 60

<212> DNA
<213> Artificial Sequence

<220>
<223> Description of Artificial Sequence: PCR primer
      sequence

<220>
<221> primer_bind
<222> (1)..(60)

<400> 23
aaaactcgag tcacttgtcg tcatcgtctt tgtagtcgcc tctcctgaca cgcagtaagg 60

<210> 24
<211> 54
<212> DNA
<213> Artificial Sequence

<220>
<223> Description of Artificial Sequence: PCR primer
      sequence

<220>
<221> primer_bind

<222> (1)..(54)

<400> 24
aaaactcgag tcaatggtga tggtgatgat ggcctctcct gacacgcagt aagg          54

<210> 25
<211> 21
<212> DNA
<213> Artificial Sequence

<220>
<223> Description of Artificial Sequence: PCR primer
      sequence

<220>
<221> primer_bind
<222> (1)..(21)

<400> 25
cagagtggca gaaaggctga t                                              21

<210> 26
<211> 20
<212> DNA

<213> Artificial Sequence

<220>
<223> Description of Artificial Sequence: PCR primer
      sequence

<220>
<221> primer_bind
<222> (1)..(20)

<400> 26
ggaagcacac agatgggtga                                                    20

<210> 27
<211> 30
<212> DNA
<213> Artificial Sequence

<220>
<223> Description of Artificial Sequence: PCR primer
      sequence

<220>
<221> primer_bind
<222> (1)..(30)

<400> 27

aaaagaattc gtgaaggcct cgtccacctc                                        30

<210> 28

<211> 31

<212> DNA

<213> Artificial Sequence

<220>

<223> Description of Artificial Sequence: PCR primer

      sequence

<220>

<221> primer_bind

<222> (1)..(31)

<400> 28

aaaagtcgac ttagcctctc ctgacacgca g                                      31

<210> 29

<211> 30

<212> DNA

<213> Artificial Sequence

<220>

<223> Description of Artificial Sequence: PCR primer

sequence

<220>

<221> primer_bind

<222> (1)..(30)

<400> 29

aaaagaattc gtgaaggcct cgtccacctc 30

<210> 30

<211> 33

<212> DNA

<213> Artificial Sequence

<220>

<223> Description of Artificial Sequence: PCR primer

sequence

<220>

<221> primer_bind

<222> (1)..(33)

<400> 30
ctccgtcgac ttatgcactc acgtctgtgg tcg                                    33

<210> 31
<211> 32
<212> DNA
<213> Artificial Sequence

<220>
<223> Description of Artificial Sequence: PCR primer
      sequence

<220>
<221> primer_bind
<222> (1)..(32)

<400> 31
aaaagaattc ggtgaaaatg gaggtttcct cc                                     32

<210> 32
<211> 31
<212> DNA
<213> Artificial Sequence

<220>

<223> Description of Artificial Sequence: PCR primer

sequence

<220>

<221> primer_bind

<222> (1)..(31)

<400> 32

aaaagtcgac ttagcctctc ctgacacgca g 31

<210> 33

<211> 37

<212> DNA

<213> Artificial Sequence

<220>

<223> Description of Artificial Sequence: PCR primer

sequence

<220>

<221> primer_bind

<222> (1)..(37)

<400> 33

ggggctcgag atgaccatga ttacggattc actggcc                                37

<210> 34
<211> 35
<212> DNA
<213> Artificial Sequence

<220>
<223> Description of Artificial Sequence: PCR primer
      sequence

<220>
<221> primer_bind
<222> (1)..(35)

<400> 34
gggggtcgac ttatttttga caccagacca actgg                                  35

<210> 35
<211> 37
<212> DNA
<213> Artificial Sequence

<220>

<223> Description of Artificial Sequence: PCR primer
      sequence

<220>
<221> primer_bind
<222> (1)..(37)

<400> 35
ggggctcgag atgaccatga ttacggattc actggcc                                37

<210> 36
<211> 35
<212> DNA
<213> Artificial Sequence

<220>
<223> Description of Artificial Sequence: PCR primer
      sequence

<220>
<221> primer_bind
<222> (1)..(35)

<400> 36
gggggtcgac ttacggtgca cgggtgaact gatcg                                  35

<210> 37

<211> 32

<212> DNA

<213> Artificial Sequence

<220>

<223> Description of Artificial Sequence: PCR primer

sequence

<220>

<221> primer_bind

<222> (1)..(32)

<400> 37

ggggaagctt atgacaacgg acgacacaga ag 32

<210> 38

<211> 33

<212> DNA

<213> Artificial Sequence

<220>

<223> Description of Artificial Sequence: PCR primer

sequence

<220>
<221> primer_bind
<222> (1)..(33)

<400> 38
aaaactcgag gcctctcctg acacgcagta agg                                    33

<210> 39
<211> 32
<212> DNA
<213> Artificial Sequence

<220>
<223> Description of Artificial Sequence: PCR primer
      sequence

<220>
<221> primer_bind
<222> (1)..(32)

<400> 39
ggggaagctt atgacaacgg acgacacaga ag                                     32

<210> 40

<211> 31

<212> DNA

<213> Artificial Sequence

<220>

<223> Description of Artificial Sequence: PCR primer

sequence

<220>

<221> primer_bind

<222> (1)..(31)

<400> 40

cattctcgag tgcactcacg tctgtggtcg g 31

<210> 41

<211> 37

<212> DNA

<213> Artificial Sequence

<220>

<223> Description of Artificial Sequence: PCR primer

sequence

<220>

<221> primer_bind

<222> (1)..(37)

<400> 41

gtttaagctt atgagaagac tcctacaacc cgaatac                                37

<210> 42

<211> 31

<212> DNA

<213> Artificial Sequence

<220>

<223> Description of Artificial Sequence: PCR primer

      sequence

<220>

<221> primer_bind

<222> (1)..(31)

<400> 42

actactcgag tgatgtctcc cagaaggagg c                                      31

<210> 43

<211> 25

<212> DNA

<213> Artificial Sequence

<220>

<223> Description of Artificial Sequence: PCR primer

sequence

<220>

<221> primer_bind

<222> (1)..(25)

<400> 43

catccaagcc atatatggac gttcc 25

<210> 44

<211> 25

<212> DNA

<213> Artificial Sequence

<220>

<223> Description of Artificial Sequence: PCR primer

sequence

<220>

<221> primer_bind

<222> (1)..(25)

<400> 44

tctggagagt caaaattctc ttcgt                                          25

<210> 45

<211> 23

<212> DNA

<213> Artificial Sequence

<220>

<223> Description of Artificial Sequence: PCR primer

      sequence

<220>

<221> primer_bind

<222> (1)..(23)

<400> 45

ggcatccggc acctctatgg tcc                                            23

<210> 46

<211> 24

<212> DNA

<213> Artificial Sequence

<220>

<223> Description of Artificial Sequence: PCR primer

sequence

<220>

<221> primer_bind

<222> (1)..(24)

<400> 46

gccacttgtc ggcgataagg aagg 24

<210> 47

<211> 26

<212> DNA

<213> Artificial Sequence

<220>

<223> Description of Artificial Sequence: PCR primer

sequence

<220>

<221> primer_bind
<222> (1)..(26)

<400> 47
tgaaggtcgg agtcaacgga tttggt                                          26

<210> 48
<211> 24
<212> DNA
<213> Artificial Sequence

<220>
<223> Description of Artificial Sequence: PCR primer
      sequence

<220>
<221> primer_bind
<222> (1)..(24)

<400> 48
catgtgggcc atgaggtcca ccac                                            24

<210> 49
<211> 21

<212> DNA
<213> Artificial Sequence

<220>
<223> Description of Artificial Sequence: PCR primer
      sequence

<220>
<221> primer_bind
<222> (1)..(21)

<400> 49
atgctgtgga tctgggctgt c                                                21

<210> 50
<211> 21
<212> DNA
<213> Artificial Sequence

<220>
<223> Description of Artificial Sequence: PCR primer
      sequence

<220>
<221> primer_bind

<222> (1)..(21)

<400> 50
ggttctagat tcagggtcat c                                                21

<210> 51
<211> 29
<212> DNA
<213> Artificial Sequence

<220>
<223> Description of Artificial Sequence: PCR primer
      sequence

<220>
<221> primer_bind
<222> (1)..(29)

<400> 51
gcatctagaa tcgaggttat taattgcag                                        29

<210> 52
<211> 21
<212> DNA

<213> Artificial Sequence

<220>
<223> Description of Artificial Sequence: PCR primer
      sequence

<220>
<221> primer_bind
<222> (1)..(21)

<400> 52
ttagcctctc ctgacacgca g                                                21

<210> 53
<211> 39
<212> DNA
<213> Artificial Sequence

<220>
<223> Description of Artificial Sequence: PCR primer
      sequence

<220>
<221> primer_bind
<222> (1)..(39)

<400> 53
aaaaaagctt ggcaccatgc tgaaggcctc gtccacctc                                39

<210> 54
<211> 31
<212> DNA
<213> Artificial Sequence

<220>
<223> Description of Artificial Sequence: PCR primer
      sequence

<220>
<221> primer_bind
<222> (1)..(31)

<400> 54
aaaactcgag ttacggagct gctcctgaga c                                        31

<210> 55
<211> 33
<212> DNA
<213> Artificial Sequence

<220>

<223> Description of Artificial Sequence: PCR primer

      sequence

<220>

<221> primer_bind

<222> (1)..(33)

<400> 55

aaaactcgag ttaaggggtg aagttcttga ggg                                    33

<210> 56

<211> 32

<212> DNA

<213> Artificial Sequence

<220>

<223> Description of Artificial Sequence: PCR primer

      sequence

<220>

<221> primer_bind

<222> (1)..(32)

<400> 56

aaaactcgag ttacgtccct cggctgctgt tg                                    32

<210> 57

<211> 32

<212> DNA

<213> Artificial Sequence

<220>

<223> Description of Artificial Sequence: PCR primer

      sequence

<220>

<221> primer_bind

<222> (1)..(32)

<400> 57

aaaactcgag ttatgcactc acgtctgtgg tc                                    32

## Description

### Technical Field

The present invention relates to pharmaceutical compositions comprising a substance having activity to inhibit the biological activity of c-Met Regulatory Mucin (MERMUC). Specifically, the present invention relates to a substance having activity to inhibit the binding between MERMUC and c-Met or the expression of MERMUC.

More specifically, the invention relates to pharmaceutical compositions comprising a substance having activity to inhibit the binding between MERMUC and c-Met or the expression of MERMUC yet used for maintaining or enhancing cell proliferation or formation, repair, regeneration or neogenesis of tissues, organs, blood vessels, mucosa, skeleton or nerves due to hepatocyte growth factor (HGF).

### Background Art

The living body of mammals and other organisms has regenerative ability to quickly repair or newly generate damaged or defective tissues and organs in vivo so that they function normally. In other words, this regenerative ability is indispensable for treating and recovering a living body from a diseased state.

Hepatocyte growth factor (HGF) has been extensively studied as one of the factors greatly contributing to this regenerative ability of tissues and organs. Furthermore, in the field of regenerative medicine therapy, HGF is greatly expected to enable treatment of various intractable diseases, and research and development for clinical applications is now being extensively pursued.

In 1984, HGF was identified as a potent growth factor of primary cultured hepatocytes partially purified from rat blood by Nakamura *et al*. (Biochem. Biophys. Res. Commun. , 1984, Vol.122, p.1450-1459). Five years later in 1989, cDNAs encoding human and rat HGF were cloned and the primary structure of HGF was elucidated (Nature, 1989, Vol.342, p.440-443).

Human HGF is synthesized as a single-stranded inactive prepro form comprising 728 amino acid residues. Then, it is processed by a serine protease called HGF converting enzyme or HGF activator into an active heterodimer consisting of an α chain (molecular weight 69 kDa) and a β chain (molecular weight 34 kDa). This active heterodimer binds to the HGF receptor to exhibit various biological activities of HGF.

On the other hand, HGF was found to be the ligand for receptor-type tyrosine kinase c-Met that is a product of the c-met gene originally identified as an oncogene from human osteosarcoma cells. Thus, c-Met was identified to be the receptor for HGF (Science, 1991, Vol.251, p.801-804; Oncogene, 1991, Vol.6, p.501-504).

c-Met, which is an HGF receptor, is a heterodimer of disulfide-bonded α chain (molecular weight 50 kDa) and β chain (molecular weight 145 kDa). The β chain of c-Met comprises an extracellular region, transmembrane region and cytoplasmic region, and a tyrosine kinase domain exists in the cytoplasmic region. When HGF binds to receptor c-Met, the receptor dimerizes, and then autophosphorylation of the tyrosine residues existing in the cytoplasmic region is induced.

Furthermore, a site consisting of 3 phosphorylated tyrosine residues called multifunctional docking site exists on the C-terminal side of the c-Met β chain. Through the binding of various signal transduction molecules comprising an SH2 domain, such as PI3 kinase, PLC-γ, SHC, Grb-1, Grb-2 and pp60^{c-SRC}, to this site, transmission of signals involved in various biological activities of HGF is induced (for example, Cell, 1994, Vol.77, p.261-271, 1994; Jikken Igaku (Experimental Medicine), 1997, Vol.15, p.1033-1039).

Previous studies have revealed that HGF exhibits various kinds of biological activities, and therapeutic and ameliorative effects against disorders, as described below, through signal transduction into cells via the binding to its receptor, c-Met:
(1) HGF functions as a growth-promoting factor of not only hepatocytes but also most epithelial cells (renal tubule epithelial cells, skin keratinocytes, melanocytes, alveolar type II epithelial cells, gastric mucosal epithelial cells, etc.) and some mesenchymal cells (vascular endothelial cells, chondrocytes, osteoclasts, muscular satellite cells, etc.) (for example, J. Biochem., 1996, Vol.119, p.591-600; Biochem. Biophys. Res. Commun., 1997, Vol.239, p.639-644);
(2) HGF has the function as a mediator molecule for the interaction between epithelial cells and mesenchymal tissues in the developmental process of mammalian and amphibian organisms, and induces the formation of internal organs, such as liver, kidney and lung, and various tissue organs, such as limb bud and somite (J. Biochem., 1996, Vol.119, p.591-600; Biochem. Biophys. Res. Commun., 1997, Vol.239, p.639-644; J. Cell Biol., 1993, Vol.123, p.223-236; Development; 1998, Vol.125, p.1315-1324; Nature, 1995, vol.373, p.699-702; Nature, 1995, Vol.373, p.702-705; Nature, 1995, Vol.376, p.768-771; Biochem. Biophys. Res. Commun., 1997, Vol.234, p.8-14);
(3) On the other hand, in a mature organism, HGF functions as a factor that promotes repair, regeneration and neogenesis of organs, such as liver, kidney and lung, and tissues thereof by functioning paracrine and endocrine (for example, J. Biochem., 1996, Vol.119, p.591-600; Biochem. Biophys. Res. Commun., 1997, Vol.239, p.639-644; J. Cell Biol., 1993, Vol.123, p.223-236; Biochem. Biophys. Res. Commun., 1990, Vol.173, p.42-47; Biochem. Biophys. Res. Commun., 1991, Vol.177, p.330-335; Hepatology, 1992, Vol.16, p.1227-1235; J. Biol. Chem., 1991, Vol.266, p.22781-22784; Am. J. Physiol., 1993, Vol.265, p.61-69; Proc. Natl. Acad. Sci. USA., 1994, Vol.91, p.4357-4361; Am. J. Physiol., 1996, Vol.270, p.1031-1039; Protein Nucleic Acid and Enzyme, 2000, Vol.45, No.13, p.2100-2108);
(4) Apart from the role in the proliferation of the above-mentioned epithelial cells and some mesenchymal cells (vascular endothelial cells, muscular cells, etc.), and the repair and regeneration of damages in organs such as liver, kidneys and lungs, HGF strongly promotes proliferation and repair of other cells, such as gastric mucosal epithelial cells, myocardial cells, skeletal muscle cells and nerve cells, and takes part in the prevention of disruption as well as regeneration and repair of gastric mucosa, heart, skeletal tissue and nervous tissue (for example, Protein Nucleic Acid and Enzyme, 2000, Vol.45, No.13, p.2100-2108);
(5) HGF suppresses apoptosis of vascular endothelial cells due to hyperglycemia, ischemia, etc. (for example, Diabetologia, 1997, Vol.40, p.1053-1061; Diabetes, 1997, Vol.46, p.138-142);
(6) Since the expression of uPA, MMP-1, MMP-9 and such are induced by stimulating myocardial fibroblasts with HGF, HGF is suggested to have an anti-fibrosis effect on various tissues (for example, Nihon Rinsho (Japanese Journal of Clinical Medicine) , 2000, Vol.58, Suppl. p.168-172);
(7) Vascular neogenesis due to HGF has been confirmed by administering HGF or HGF gene to rabbits and rats (for example, Nihon Rinsho (Japanese Journal of Clinical Medicine, 2000, Vol.58, Suppl. p.168-172; Hypertension, 1999, Vol.33, p.1379-1384); and
(8) Animal testing has shown that HGF exhibits dramatic therapeutic effects towards intractable organ failure for which ultimate therapy still do not exist, such as hepatic fibrosis, hepatic cirrhosis, fulminant hepatitis, viral hepatitis, acute hepatitis, chronic renal failure, renal fibrosis and pulmonary fibrosis, through the wide variety of physiological activities of HGF described above (for example, Experimental Medicine, 1997, Vol.15, p.90-97; J. Biochem., 1995, Vol.118, p.643-649; Nature Med., 1999, Vol.5, p.226-230; Hepatology, 1997, Vol.26, p.81-89; Hepatology, 1999, Vol.30, p.151-159; Biochem. Biophys. Res. Commun., 1998, Vol.244, p.683-690; J. Clin. Invest., 1999, Vol.103, p.313-320; Kidney Int., 2000, Vol.57, p.937-948; J. Clin. Invest., 1998, Vol.101, p.1827-1834; Am. J. Repair. Crit. Care Med., 1997, Vol.156, p.1937-1944).

Furthermore, recently, treatment of arteriosclerosis obliterans by gene therapy using the HGF gene has been attempted.

While various biological activities and therapeutic effects of HGF that are effective for the treatment of various kinds of intractable diseases as mentioned above are elucidated, the possibility of carcinogenesis attributable to excessive provision of HGF into the patient's body is a matter of concern in the treatment of diseases using HGF or the HGF gene. Therefore, there is a demand for the development of novel methods that enable treatment of such diseases without depending only on the administration of HGF or HGF gene to the living body.

As an approach for developing such novel therapeutic methods, a promising method is to identify a novel molecule that modulates the HGF/c-Met signal transduction by interacting with c-Met, the HGF receptor, and modulate the interaction of the novel molecule with c-Met or the function of the novel molecule.

### Disclosure of the Invention

Specifically, the objectives of the present invention are: (1) identifying a novel molecule that modulates the HGF/c-Met signal transduction by interacting with the HGF receptor c-Met; (2) providing methods, substances and pharmaceutical compositions that modulate the HGF/c-Met signal transduction by regulating the interaction of the novel molecule with c-Met or the function of the novel molecule; and (3) using these methods, substances and pharmaceutical compositions, to treat various diseases to which HGF drugs (HGF protein drugs, HGF gene therapy, etc.) are applied for treatment; in particular, intractable organ diseases such as hepatic fibrosis, hepatic cirrhosis, fulminant hepatitis, viral hepatitis, acute hepatitis, chronic renal failure, renal fibrosis and pulmonary fibrosis.

The present inventors extensively studied a recently reported mucin-like protein (International Application No. WO 01/05803) cloned from the renal tissue of a hepatitis patient (further described below) . Specifically, molecules that bind to the mucin-like protein were studied.

As a result, the present inventors discovered that: (1) the HGF receptor c-Met binds to the mucin-like protein; (2) the binding of the two molecules suppressively regulates (down-regulates) the biological activity of HGF exhibited via the signal transduction mediated by HGF/c-Met; and (3) the binding affinity of the mucin-like protein to c-Met is increased by multimerization (self-association) of the mucin-like protein. Finally, the present invention was accomplished and will be described in detail below.

The present inventors dubbed this molecule "c-Met Regulatory Mucin (MERMUC)" based on these characteristics of the mucin-like protein.

The finding that the expression of the biological activity of HGF is down-regulated by the binding of its receptor c-Met with MERMUC (including self-associated MERMUC molecules), is obviously an unprecedented and completely novel finding. Furthermore, most importantly, based on this completely novel finding, the present invention that is extremely useful in medicine and pharmaceutical industries as described below was accomplished.

Specifically, this invention enables repair, regeneration or neogenesis of tissues and organs required for the prevention and treatment of various diseases using the minimum required amount of HGF drugs (HGF protein, HGF gene, etc.) or without such drugs. More specifically, the present invention enables treatment of various intractable diseases that are still considered difficult to treat (for example, hepatic fibrosis, hepatic cirrhosis, fulminant hepatitis, viral hepatitis, acute hepatitis, chronic renal failure, renal fibrosis and pulmonary fibrosis).

This is because the potential biological activity possessed by endogenous HGF or HGF exogenously supplied (as HGF protein drug or by HGF gene therapy) to the patient can be maximally drawn out through the elimination of HGF activity down-regulation that occurs due to the binding between c-Met and MERMUC using a pharmaceutical composition of the present invention. Such pharmaceutical compositions, specifically include: (1) those comprising a substance that has activity to inhibit the binding between MERMUC and the HGF receptor c-Met; (2) those comprising a substance that has activity to inhibit multimerization (self-association) of MERMUC molecules; and (3) those comprising a substance that suppresses or inhibits the expression of MERMUC.

Therefore, in the treatment of diseases using HGF drugs, the dose of the HGF drug administered to a patient, frequency of the administration and duration of the administration can be drastically decreased by combining a pharmaceutical composition of the present invention such as those described above. Furthermore, thereby, mental and physical pain of the patient can be reduced, and simultaneously, various costs (costs borne by each of patients, medical institutes, drug makers and government agencies) will be saved.

In addition to the above-mentioned pharmaceutical compositions, the present invention provides methods for identifying the active ingredients of the pharmaceutical compositions: (1) substances having activity to inhibit the binding between c-Met and MERMUC; (2) substances having activity to inhibit multimerization (self-association) of MERMUC; and (3) substances that suppress or inhibit the expression of MERMUC. Furthermore, the present invention provides methods of testing a given substance for various activities, such as: (1) activity to inhibit the binding between c-Met and MERMUC; and (2) activity to inhibit multimerization (self-association) of MERMUC. Moreover, the present invention provides various tools (various polypeptides, fusion polypeptides, etc.) used for these methods. The above-described pharmaceutical compositions of the present invention can be provided through these inventions of methods and tools.

More specifically, the present invention relates to:
(1) a pharmaceutical composition used for maintaining or enhancing cell proliferation, or formation, repair, regeneration or neogenesis of tissues, organs, blood vessels, mucosa, skeleton or nerves, which comprises a substance having activity to inhibit the binding between c-Met and MERMUC or multimerization of MERMUC, and a pharmaceutically acceptable carrier;
(2) a pharmaceutical composition used for maintaining or enhancing cell proliferation, or formation, repair, regeneration or neogenesis of tissues, organs, blood vessels, mucosa, skeleton or nerves due to HGF, which comprises a substance having activity to inhibit the binding between c-Met and MERMUC or multimerization of MERMUC, and a pharmaceutically acceptable carrier;
(3) the pharmaceutical composition of (2) , wherein said HGF is an endogenous HGF of an organism;
(4) the pharmaceutical composition of (2) , wherein said HGF is an HGF drug that exogenously provides HGF to an organism;
(5) the pharmaceutical composition of (4) , wherein the HGF drug is a protein drug comprising an HGF protein;
(6) the pharmaceutical composition of (4) , wherein the HGF drug is a DNA drug comprising DNA encoding an HGF protein;
(7) the pharmaceutical composition of (1) or (2) , which is used for preventing or treating fibrosis;
(8) the pharmaceutical composition of (7) , wherein the fibrosis is hepatic, renal or pulmonary fibrosis;
(9) the pharmaceutical composition of (1) or (2), which is used for preventing or treating hepatic cirrhosis, hepatic fibrosis or hepatitis;
(10) the pharmaceutical composition of (1) or (2), wherein hepatitis is used for preventing or treating fulminant hepatitis, acute hepatitis or viral hepatitis;
(11) the pharmaceutical composition of (1) or (2) , which is used for preventing or treating chronic renal failure;
(12) the pharmaceutical composition of (1) or (2) , which is used for preventing or treating arteriosclerosis obliterans;
(13) the pharmaceutical composition of (1) or (2) , wherein the substance has activity to inhibit the binding between c-Met and MERMUC;
(14) the pharmaceutical composition of (13), wherein the substance has activity to inhibit the binding between the C-terminal region of the c-Met β chain and the C-terminal region of MERMUC;
(15) the pharmaceutical composition of (1) or (2) , wherein the substance has activity to inhibit multimerization of MERMUC;
(16) the pharmaceutical composition of (1) or (2), wherein the substance binds to MERMUC;
(17) a pharmaceutical composition used for maintaining or enhancing cell proliferation, or formation, repair, regeneration or neogenesis of tissues, organs, blood vessels, mucosa, skeleton or nerves, which comprises a substance that suppresses or inhibits the expression of MERUMUC and a pharmaceutically acceptable carrier;
(18) the pharmaceutical composition of (17), wherein the substance inhibits the transcription of a gene encoding MERMUC to mRNA;
(19) the pharmaceutical composition of (17), wherein the substance inhibits the translation of an mRNA encoding MERMUC to protein;
(20) a pharmaceutical composition used for maintaining or enhancing cell proliferation, or formation, repair, regeneration or neogenesis of tissues, organs, blood vessels, mucosa, skeleton or nerves due to HGF, which comprises a substance that suppresses or inhibits the expression of MERUMUC and a pharmaceutically acceptable carrier;
(21) the pharmaceutical composition of (20), wherein the substance inhibits the transcription of a gene encoding MERMUC to mRNA;
(22) the pharmaceutical composition of (20), wherein the substance inhibits the translation of an mRNA encoding MERMUC to protein;
(23) the pharmaceutical composition of (20) , wherein said HGF is an endogenous HGF of an organism;
(24) the pharmaceutical composition of (20) , wherein said HGF is an HGF drug that can exogenously provide HGF to an organism;
(25) the pharmaceutical composition of (24), wherein the HGF drug is a protein drug comprising an HGF protein;
(26) the pharmaceutical composition of (24), wherein the HGF drug is a DNA drug comprising DNA encoding an HGF protein;
(27) the pharmaceutical composition of (17) or (20), which is used for preventing or treating fibrosis;
(28) the pharmaceutical composition of (27), wherein the fibrosis is hepatic, renal or pulmonary fibrosis;
(29) the pharmaceutical composition of (17) or (20), which is used for preventing or treating hepatic cirrhosis, hepatic fibrosis or hepatitis;
(30) the pharmaceutical composition of (17) or (20), wherein hepatitis is used for preventing or treating fulminant hepatitis, acute hepatitis or viral hepatitis;
(31) the pharmaceutical composition of (17) or (20), which is used for preventing or treating chronic renal failure;
(32) the pharmaceutical composition of (17) or (20), which is used for preventing or treating arteriosclerosis obliterans;
(33) a polypeptide comprising a partial amino acid sequence of the full-length amino acid sequence of MERMUC, and having activity to bind c-Met;
(34) the polypeptide of (33), wherein said partial amino acid sequence corresponds to whole or a portion of a cytoplasmic region of MERMUC;
(35) the polypeptide of (35) , wherein said whole or a portion of a cytoplasmic region comprises whole or a portion of a leucine repeat region in the C-terminal region of MERMUC;
(36) the polypeptide of (33), wherein said MERMUC is human MERMUC;
(37) the polypeptide of (33), which comprises the amino acid sequence of SEQ ID NO: 15;
(38) a polypeptide comprising a partial amino acid sequence of the full-length amino acid sequence of c-Met, and having activity to bind MERMUC;
(39) the polypeptide of (38), wherein said partial amino acid sequence corresponds to whole or a portion of a cytoplasmic region of the c-Metβ chain;
(40) the polypeptide of (39), wherein said whole or a portion of a cytoplasmic region comprises whole or a portion of a C-terminal region of c-Met comprising one or more tyrosine residues that undergo self-phosphorylation;
(41) the polypeptide of (38) , wherein said c-Met is human c-Met;
(42) the polypeptide of (38), which comprises the amino acid sequence of SEQ ID NO: 5;
(43) a polypeptide comprising a partial amino acid sequence of the full-length amino acid sequence of MERMUC, and comprising the activity to bind MERMUC;
(44) the polypeptide of (43) , wherein said partial amino acid sequence corresponds to whole or a portion of a cytoplasmic region of MERMUC;
(45) the polypeptide of (33), wherein said MERMUC is human MERMUC;
(46) a fusion polypeptide represented by the general formula W-X-Y (wherein, W is absent, or the full-length amino acid sequence or a portion of a protein other than MERMUC; X is the full-length amino acid sequence of MERMUC or a portion thereof; and Y is the full-length amino acid sequence or a portion of a protein other than MERMUC) ;
(47) the fusion polypeptide of (46) , wherein the protein Y is β-galΔα or β-galΔω;
(48) the fusion polypeptide of (47), wherein said β-galΔα comprises the amino acid sequence of SEQ ID NO: 16;
(49) the fusion polypeptide of (47), wherein said β-galΔω comprises the amino acid sequence of SEQ ID NO: 17;
(50) the fusion polypeptide of (46), wherein said portion of the full-length amino acid sequence of MERMUC has activity to bind c-Met;
(51) the fusion polypeptide of (46), wherein said portion of the full-length amino acid sequence of MERMUC comprises whole or a portion of a cytoplasmic region of MERMUC;
(52) the fusion polypeptide of (51) , wherein said portion of the full-length amino acid sequence of MERMUC comprises whole or a portion of a leucine repeat region of the C-terminal region of MERMUC;
(53) the fusion polypeptide of (46), wherein said MERMUC is human MERMUC;
(54) the polypeptide of (46) , wherein said X comprises the amino acid sequence of SEQ ID NO: 15;
(55) the polypeptide of (46), wherein the protein W comprises the extracellular region of Fas;
(56) a fusion polypeptide represented by the general formula Z-Y (wherein Z is the full-length amino acid sequence of c-Met or a portion thereof; and Y is the full-length amino acid sequence or a portion of a protein other than c-Met);
(57) the fusion polypeptide of (52), wherein said protein is β-galΔα or β-galΔω;
(58) the fusion polypeptide of (57), wherein said β-galΔα comprises the amino acid sequence of SEQ ID NO: 16;
(59) the fusion polypeptide of (57), wherein said β-galΔω comprises the amino acid sequence of SEQ ID NO: 17;
(60) the fusion polypeptide of (56), wherein said portion of the full-length amino acid sequence of c-Met has activity to bind MERMUC;
(61) the fusion polypeptide of (56), wherein said portion of the full-length amino acid sequence of c-Met comprises whole or a portion of a cytoplasmic region of the c-Met β chain;
(62) the fusion polypeptide of (56), wherein said portion of the full-length amino acid sequence of c-Met comprises whole or a portion of a C-terminal region of c-Met comprising one or more tyrosine residues that undergo self-phosphorylation;
(63) the fusion polypeptide of (56) , wherein said c-Met is human c-Met;
(64) the fusion polypeptide of (56) , wherein said Z comprises the amino acid sequence of SEQ ID NO: 5;
(65) a method of testing a substance for the presence or extent of activity to inhibit the binding between c-Met and MERMUC, which comprises the step of comparing the presence or extent of the binding between c-Met and MERMUC in the presence of a test substance to the extent of the binding between c-Met and MERMUC in the absence of the test substance;
(66) a method of identifying a substance that has activity to inhibit the binding between c-Met and MERMUC, which comprises the step of comparing the presence or extent of the binding between c-Met and MERMUC in the presence of a test substance to the extent of the binding between c-Met and MERMUC in the absence of the test substance;
(67) a method of testing for the presence or extent of activity of a substance to inhibit the binding between c-Met and MERMUC, which comprises the steps of:
   (a) preparing a cell that is designed to coexpress c-Met and MERMUC, and allow detection of the binding between said c-Met and MERMUC;
   (b) culturing the cell obtained in step (a) in the presence of HGF while in the absence of test substance, and then measuring the extent of the binding between c-Met and MERMUC in the cell;
   (c) culturing the cell obtained in step (b) in the presence of HGF and a test substance, and then measuring the extent of the binding between c-Met and MERMUC in the cell; and
   (d) comparing the measurement result of step (b) with that of step (c);
(68) the method of (67), wherein said MERMUC is the fusion polypeptide of any one of (46) to (54);
(69) the method of (67), wherein said MERMUC is the fusion polypeptide of any one of (47) to (49);
(70) the method of (67), wherein said c-Met is the fusion polypeptide of any one of (56) to (64);
(71) the method of (67), wherein said c-Met is the fusion polypeptide of any one of (57) to (59);
(72) a method of identifying a substance that has activity to inhibit the binding between c-Met and MERMUC, which comprises the steps of:
   (a) preparing a cell that is designed to coexpress c-Met and MERMUC, and allow detection of the binding between said c-Met and MERMUC bind;
   (b) culturing the cell obtained in step (a) in the presence of HGF while in the absence of test substance, and then measuring the extent of the binding between c-Met and MERMUC in the cell;
   (c) culturing the cell obtained in step (b) in the presence of HGF and a test substance, and then measuring the extent of the binding between c-Met and MERMUC in the cell; and
   (d) comparing the measurement result of step (b) with that of step (c);
(73) the method of (72), wherein said MERMUC is the fusion polypeptide of any one of (46) to (54);
(74) the method of (72), wherein said MERMUC is the fusion polypeptide of any one of (47) to (49);
(75) the method of (72), wherein said c-Met is the fusion polypeptide of any one of (56) to (64);
(76) the method of (72), wherein said c-Met is the fusion polypeptide of any one of (57) to (59);
(77) a method of testing a substance for the presence or extent of activity to inhibit multimerization of MERMUC, which comprises the step of comparing the presence or extent of multimerization of MERMUC in the presence of a test substance to the extent of multimerization of MERMUC in the absence of the test substance; and
(78) a method for identifying a substance having activity to inhibit multimerization of MERMUC, which comprises the step of comparing the presence or extent of multimerization of MERMUC in the presence of a test compound to the extent of multimerization of MERMUC in the absence of the test substance.

The present invention is specifically described by defining the meaning of the terms used in this invention and methods of producing the substances, as follows.

In the present invention, the term "mammal" refers to humans, cattle, goats, rabbits, mice, rats, hamsters, guinea pigs and such, preferably humans, cattle, rats, mice or hamsters, and particularly preferably humans.

Herein, the term "c-Met" refers to the c-Met of the above-mentioned mammals, preferably human, mouse or rat c-Met, and particularly preferred is human c-Met. c-Met is a receptor of hepatocyte growth factor (HGF) defined below, and c-Met has the structure and biological function as previously reported.

c-Met is a heterodimer of disulfide-bonded α chain (molecular weight: approximately 50 kDa) consisting of only the extracellular region, and β chain (molecular weight: approximately 145 kDa) which is a transmembrane protein.

Human c-Met is a preferred embodiment of the present invention, and isomers of c-Met (isomer 1, isomer 2, etc.) exist for human c-Met. The human c-Met of this invention includes all of these isomers. Isomer 1 and isomer 2 are considered splice variants to each other.

The primary structures (the precursor of the heterodimer) of human c-Met reported as isomer 1 and isomer 2 comprise 1390 amino acid residues (SEQ ID NO: 2; GenBank Accession Nos. P08581, TVHUME and CAB56793; the corresponding cDNA nucleotide sequence is shown in SEQ ID NO: 1) and 1408 amino acid residues (GenBank Accession No. 4557747; corresponding), respectively.

The only difference between isomer 1 and isomer 2 is the 18 amino acid residues present between amino acids 754 (Ile) and 755 (Ser) of isomer 1, and the rest of the amino acid sequence is identical.

Hereinafter, c-Met will be described and defined using isomer 1 as an example; however, it should be construed that isomer 2 which has a similar structure is also encompassed by the present invention.

Human c-Met consists of signal peptide (amino acids 1-24 of SEQ ID NO: 2), α chain (amino acids 25-303 of SEQ ID NO: 2) and β chain (amino acids 308-1390 of SEQ ID NO: 2; identical to the full-length sequence of SEQ ID NO: 4; SEQ ID NO: 3 is the corresponding cDNA sequence) (Proc. Natl. Acad. Sci. USA., 1987, Vol.84, No.8, p.6379-6383; Oncogene, 1987, Vol.1, No.2, p.229-233; Oncogene, 1993, Vol.8, No.12, p.3403-3410; Nature, 1985, Vol.318, No.6044, p.385-388; Science, 1991, Vol.251, No.4995, p.802-804; J. Biol. Chem., 1991, Vol.266, No.29, p.19558-19564; Nature Genetics, 1997, Vol.16, No.1, p.68-73; Mol. Cell. Biol., 1987, Vol.7, No.2, p.921-924; J. Biol. Chem., 1994, Vol.269, No.17, p.12852-12857).

The structural and biological characteristics of both mouse and rat c-Met are similar to human c-Met ([mouse c-Met] GenBank Accession Nos. S01254, NP 032617, P16056, and CAA68680; Oncogene, 1988, Vol.2, No.6, p.593-599; Gene, 1989, Vol.85, No.1, p.67-74; J. Cell Biol., 1993, Vol.121, No.1, p.145-154. [rat] GenBankAccessionNos. NP 113705 and AAB19189; Am. J. Physiol. 1996, Vol.271, No.3 Pt 2, p.F679-F688) .

The β chain of c-Met (in humans, amino acids 308-1390 of SEQ ID NO: 2; same as the full-length sequence of SEQ ID NO: 4) is composed of an extracellular region (in humans, amino acids 308-932 of SEQ ID NO: 2; same as amino acids 1-625 of SEQ ID NO: 4), transmembrane region (in humans, amino acids 933-955 of SEQ ID NO: 2; same as amino acids 626-648 of SEQ ID NO: 4) and cytoplasmic region (in humans, amino acids 956-1390 of SEQ ID NO: 2; same as amino acids 649-1083 of SEQ ID NO: 4). The cytoplasmic region comprises a tyrosine kinase domain (in humans, amino acids 1078-1345 of SEQ ID NO: 2; same as amino acids 771-1038 of SEQ ID NO: 4). When HGF binds to the receptor c-Met, dimerization of the receptor takes place, and subsequently induces self-phosphorylation of tyrosine residues that exist in the cytoplasmic region (in humans, the tyrosine residues of amino acids 1349, 1356 and 1365 of SEQ ID NO: 2; same as amino acids 1042, 1049 and 1058 of SEQ ID NO: 4).

Furthermore, a site comprising 3 phosphorylated tyrosine residues (in humans, amino acids 1349, 1356 and 1365 of SEQ ID NO: 2; same as amino acids 1042, 1049 and 1058 of SEQ ID NO: 4) called multifunctional docking site exists on the C-terminal side of the c-Met β chain. The binding of various signal transduction molecules comprising an SH2 domain, such as PI3 kinase, PLC-γ, SHC, Grb-1, Grb-2 and pp60^{c-SRC}, to this site, in particular, causes transmission of signals responsible in various biological activities of HGF (Cell, 1994, Vol.77, p.261-271; Experimental Medicine, 1997, Vol.15, p.1033-1039).

The phrase "cytoplasmic region of the c-Met β chain" in this invention preferably refers to the cytoplasmic region of the c-Met β chain of human, mouse or rat, and particularly preferred is the cytoplasmic region of the human c-Met β chain (amino acids 956-1390 of SEQ ID NO: 2; same as amino acids 649-1083 of SEQ ID NO: 4).

The phrase "a portion of the cytoplasmic region of the c-Met β chain" in this invention refers to any arbitrary portion of the above-mentioned cytoplasmic region. However, the C-terminal region of the β chain is preferred, and a portion binding to the C-terminal region MERMUC, defined *infra*, which comprises one or more tyrosine residues that undergo self-phosphorylation (in humans, amino acids 1349, 1356 and 1365 of SEQ ID NO: 2; the same as amino acids 1042, 1049 and 1058 of SEQ ID NO: 4) is particularly preferred. More specifically, it refers to a region comprising the shortest amino acid sequence required in the binding with MERMUC (SEQ ID NO: 5), which sequence is indicated in the Examples described below. However, this shortest amino acid sequence refers to the sequence based only on limited experimental results disclosed in the Examples described below. Thus, the term "smallest unit" in the present invention also refers to shorter amino acid sequences than this sequence of SEQ ID NO: 5.

The human c-Met of this invention includes isomers of human c-Met having the primary structures described above (one of the isomers, isomer 2 comprises 1408 amino acids; Proc. Natl. Acad. Sci. USA., 1987, Vol.84, No.18, p.6379-6383; Nature, 1985, Vol.318, No.6044, p.385-388; Mol. Cell. Biol., 1987, Vol.7, No.2, p.921-924; Science, 1991, Vol.251, No.4995, p.802-804; Cytogenet. Cell Genet., 1992, Vol.60, No.2, p.114-116; Cell. Mol. Biol. Res., 1994, Vol.40, No.4, p.337-350; Cell Mol. Biol. Res., 1994, Vol.40, No.7-8, p.7-7) . The primary structure of the two isomers differ only in a portion of the amino acid sequence within the extracellular region, and the amino acid sequences from the C-terminal side of the extracellular region through the cytoplasmic region to the C-terminus are identical. This means that the structure of the region required for the binding to MERMUC, demonstrated in the following Examples, is conserved in both forms of the human c-Met β chains.

In the present invention, the term "HGF" refers to a mammal-derived hepatocyte growth factor (HGF), which is a ligand of c-Met defined above. Preferred HGF includes those from human, mouse and rat, and human HGF is particularly preferred.

HGF is synthesized as single-stranded inactive prepro form. Then, it is processed by a serine protease called HGF converting enzyme or HGF activator to form the active heterodimer consisting of an α chain (molecular weight of approximately 69 kDa) and a β chain (molecular weight of approximately 34 kDa). This active heterodimer exhibits various biological activities of HGF through the binding to an HGF receptor (see supra).

The prepro form of human HGF comprises 728 amino acids (amino acid sequence: SEQ ID NO: 7; corresponding cDNA sequence: SEQ ID NO: 6) (GenBank Accession Nos. JH0579, P14210, XP 052257, XP 052258, XP 052260, AAA52648, BAA14348, AAA52650 and AAA64239; Biochem. Biophys. Res. Commun., 1989, Vol.163 No.2, p.967-973; Nature, 1989, Vol.342, No.6248, p.440-443; Biochem. Biophys. Res. Commun., 1990, Vol.172, No.1, p.321-327; J. Cell Biol., 1990, Vol.111, No.5, Pt.1, p.2097-2108; Proc. Natl. Acad. Sci. USA., 1991, Vol.88, No.2, p.415-419; Biochem. Biophys. Res. Commun., 1991, Vol.175, No.2, p.660-667; Eur. J. Biochem. , 1991, Vol.197, No.1, p.15-22; Gene, 1991, Vol.102, No.2, p.213-219; Proc. Natl. Acad. Sci. USA., 1991, Vol.88, No.16, p.7001-7005; Biochem. Biophys. Res. Commun., 1991, Vol.180, No.2, p.1151-1158; Proc. Natl. Acad. Sci. USA., 1992, Vol.89, No.23, p.11574-11578; Biochem. Biophys. Res. Commun., 1992, Vol.189, No.3, p.1329-1335; EMBO J., 1992, Vol.11, No.7, p.2503-2510; Structure, 1998, Vol.6, No.1, p.109-116; Structure, 1998, Vol.6, No.11, p.1383-1393).

Similar to human HGF, the prepro forms of rat HGF and mouse HGF are also composed of 728 amino acids, and have structural and biological characteristics as reported previously ([rat] GenBank Accession Nos. NP 058713, P17945, A35644, BAA14133 andCAA38266; Proc. Natl. Acad. Sci. USA., 1990, Vol.87, No.8, p.3200-3204; Eur. J. Biochem., 1990, Vol.193, No.2, p.375-381. [mouse] GenBank Accession Nos. A60185, Q08048, BAA01064 and CAA58865; Nature, 1990, Vol.346, No.6281, p.228; Proc. Natl. Acad. Sci. USA., 1990, Vol.195, No.1, p.34-43; Biochem. J., 1991, Vol.278, Pt.1, p.35-41; Biochem. Biophys. Acta, 1993, Vol.1216, No.2, p.299-303, 1993; Biochem. Biophys. Res. Commun. , 1994, Vol.199, No.2, p.772-779; J. Biol. Chem., 1995, Vol.270, No.2, p.830-836; Cell Adhes. Commun., 1993, Vol.1, No.2, p.101-111).

In the present invention, the term "MERMUC" refers to a novel mammalian mucin-like molecule described in International Application No. WO 01/05803. Herein, this mucin-like molecule is called c-Met Regulatory Mucin (MERMUC). A preferred embodiment of the present invention is human, mouse or rat MERMUC, and human MERMUC is particularly preferred.

The present inventors made every effort to study MERMUC, and identified that MERMUC has at least the structural characteristics described below, and in the Examples:
(1) the primary structure of mammalian MERMUC comprises extracellular region, a transmembrane region and cytoplasmic region;
(2) a mucin-specific domain known as a potential O-linked glycosylation site exists in the extracellular region. This domain comprises a repeat structure of particular amino acid sequence rich in serine/threonine residues that can be glycosylated (in human MERMUC, the 19 amino acid residues of SEQ ID NO: 8);
(3) the number of repeating units in the mucin-specific domain is variable, and polymorphism exists in MERMUC depending on the number of repeating units;
(4) a leucine repeat region exists in the C-terminal region, also the terminus of the cytoplasmic region, of MERMUC. This repeat structure is highly conserved among humans, mice and rats; and
(5) MERMUC may exist as a monomer of a molecule having the above-mentioned primary structure, or as a multimer (oligomer, such as dimer or trimer) formed by association or binding of two or more of the molecules.

Therefore, in the present invention, MERMUC refers to a molecule having structural characteristics as discussed above (including the characteristics previously reported in International Application No. WO 01/05803). The present invention encompasses all forms of any polymorphs, monomers and multimers (dimers, oligomers, etc.).

The primary structure of human MERMUC of this invention in which such polymorphs exist includes, for example, SEQ ID NO: 10 (extracellular region: amino acids 1-199, transmembrane region: amino acids 200 to 220 or 221, and cytoplasmic region: from 221 or 222 to 503; cDNA sequence: SEQ ID NO: 9). As mentioned above, while polymorphisms exist for human MERMUC that differ in the repeat structures of the mucin-specific domain of the extracellular region, the structures of the cytoplasmic region are the same.

The primary structure of mouse MERMUC is exemplified by SEQ ID NO: 12 (cDNA sequence: SEQ ID NO: 11). An example of the primary structure of rat MERMUC (lacking the N-terminus) is SEQ ID NO: 14 (cDNA sequence: SEQ ID NO: 13).

The phrase "C-terminal region of MERMUC" as employed herein refers to the amino acid sequence of the carboxyl terminus of the cytoplasmic region of MERMUC, and the region comprising a leucine repeat structure is preferred. In the interest of human MERMUC, the region comprising 53 amino acids of the C-terminus (SEQ ID NO: 15) can be mentioned as a specific example.

The terms "β-gal", "β-galΔα" and "β-galΔω" of this invention each have the meaning defined below:

The term "β-gal" is an abbreviation of β-galactosidase (lactase), and refers to an enzyme that degrades and synthesizes lactose (glucose-β-D-galactoside). This enzyme is widely distributed from bacteria to higher animals and plants, and the structure and optimum pH differ depending on the species.

β-gal of this invention encompasses β-gal of all kind of species ; however, β-gal of *E. coli* is particularly preferred.

The structural gene encoding β-gal of *E. coli* is called lacZ. The primary structure of the precursor of *E. coli* β-gal comprises 1024 amino acids (GenBank Accession Nos.P00722 and GBEC; J. Biol. Chem., 1978, Vol.253, No.15, p.5521-5525; Bioorg. Khim., 1980, Vol.6, p.1735-1736; Nature, 1980, Vol.285, No.5759, p.38-41; EMBO J., 1983, Vol.2, No.4, p.593-597; J. Mol. Biol., 1989, Vol.208, No.1, p.23-43; Gene, 1992, Vol.122, No.1, p.231-232; Nature, 1994, Vol.369, No.6483, p.761-766; Science, 1997, Vol.277, No.5331, p.1453-1474).

The biological activity of β-gal is expressed by a homotetramer formed via the association of 4 monomeric molecules having this primary structure (according to a three-dimensional structural analysis of *E. coli* β-gal, the primary structure of each monomer molecule has been reported to consist of 1021 amino acids lacking 3 amino acids of the N-terminus; GenBank Accession Nos. 13399708; Nature, 1994, Vol.369, No.6483, p.761-766; Protein Sci., 1999, Vol.8, No.1, p.122-136; Protein Sci., 2000, Vol.9, No.9, p.1685-1699).

On the other hand, in the interest of β-gal of *E*. *coli*, three mutants, β-galΔα, β-galΔµ and β-galΔω have been reported in a type of *E*. *coli* strain (Δ denotes deletion; Proc. Natl. Acad. Sci. USA., 1996, Vol.93, p.12423-12427; Proc. Natl. Acad. Sci. USA. , 1997, Vol. 94, p.8405-8410). β-galΔα lacks a portion (amino acids 12-42 of GenBank Accession No. P00722) of the N-terminal sequence which is the active site of wild-type β-gal. β-galΔµ lacks a portion (amino acids 50-602 of GenBank Accession No. P00722) of the central sequence of wild-type β-gal. Furthermore, β-galΔω lacks a portion (amino acids 790-1024 of GenBank Accession No. P00722) of the C-terminal sequence, a site for forming homotetramer in the wild-type β-gal monomers.

The term "β-galΔα" of the present invention refers to any mutant (including naturally occurring and artificially prepared mutants) lacking a portion of the N-terminal sequence which is the active site of wild-type β-gal (particularly preferred, β-gal of *E. coli),* and the previously reported mutant β-galΔα (SEQ ID NO: 16) is particularly preferred.

The term "β-galΔω" of the present invention refers to any mutant (including naturally occurring and artificially prepared mutants) lacking a portion of the C-terminal sequence which is the site for forming homotetramer in the wild-type β-gal monomer molecules, and the previously reported mutant β-galΔω (SEQ ID NO: 17) is particularly preferred.

"c-Met" in the definition of "fusion polypeptide represented by general formula W-X-Y", and "MERMUC" in the definition of "fusion polypeptide represented by general formula Z-Y" herein have the same meaning as the above-mentioned c-Met and MERMUC, respectively.

Y in the fusion polypeptide represented by the general formula W-X-Y denotes the full-length amino acid sequence of the entire polypeptide or a portion of a protein other than MERMUC. Herein, the term "protein other than" encompasses any protein; however, preferably, it refers to a protein having a characteristic that enables measurement of the presence or degree of the binding between c-Met and MERMUC, and particularly preferred examples include β-galΔα and β-galΔω mentioned above.

W in the fusion polypeptide, represented by the general formula W-X-Y, denotes the full-length amino acid sequence of the entire polypeptide or a portion of a protein other than MERMUC.

Herein, the term "protein other than" encompasses any protein. However, preferably, it refers to a protein having a characteristic that enables measurement of the presence or degree of multimerization of MERMUC, and particularly preferred examples include mouse, rat and human Fas (<mouse> GenBank Accession No. P25446 and NP032013; <human> GenBank Accession No. P25445 and NP 000034).

Y in the fusion peptide, represented by the general formula Z-Y, denotes the full-length amino acid sequence or a portion of the entire polypeptide of a protein other than c-Met. Herein, the term "protein other than" encompasses any protein; however, preferably, it refers to a protein having a characteristic that enables measurement of the presence or degree of the binding between c-Met and MERMUC, and particularly preferred examples include β-galΔα and β-galΔω mentioned above.

In the polypeptide or fusion polypeptide of this invention, a plurality of amino acids, preferably 1 to 10, and particularly preferably 1 to 5 amino acids within the amino acid sequence may be replaced, deleted and/or modified, or a plurality of amino acids, preferably 1 to 10, particularly preferably 1 to 5 amino acids may be added to the amino acid sequence as long as it has the desired property or accomplishes the desired objective.

The phrase "HGF drugs" of this invention refers to a drug for supplying hepatocyte growth factor (HGF) defined above, particularly preferably human HGF to an organism (particularly preferably human) , and includes "protein drugs" and "DNA drugs".

A protein drug is a pharmaceutical agent comprising an HGF protein (human HGF is particularly preferred; it may be purified HGF from a cell culture or a recombinant HGF) and a pharmaceutically acceptable carrier.

The DNA drug is a pharmaceutical agent comprising a "DNA structure" carrying a gene (cDNA or genomic DNA) encoding an HGF (human HGF is particularly preferred) and a pharmaceutically acceptable carrier, yet used for what is called gene therapy.

Herein, the "DNA structure" refers to a structure wherein a gene encoding HGF is inserted in an expressible manner in a cell (particularly preferred muscle cells) of a host (specifically, the organism to which this DNA drug is administered). For example, it includes plasmid vectors and virus vectors carrying the HGF gene. In the present invention, vectors include any arbitrary plasmid vectors and virus vectors used in the field of gene therapy.

The "substance" or "test substance" of the present invention refers to natural substances or any artificially prepared substances. Specific examples encompass chemically synthesized substances (low-molecular weight compounds and such) , polypeptides (natural form, recombinant form, cultured form, etc.), antibodies, DNAs and RNAs.

"Substances having activity to inhibit the binding between c-Met and MERMUC" of this invention includes any substance as long as it has activity to inhibit the binding between c-Met and MERMUC. Therefore, in the present invention, there is no limitation on the mechanism (inhibition mechanism) by which the substance exhibits the inhibitory activity on the binding between c-Met and MERMUC. Specifically, such substance includes those functioning through the following inhibition mechanism. However, the invention should not be construed as being limited to these examples:
(1) a substance that inhibits the binding between c-Met and MERMUC via direct binding to the MERMUC binding site or its vicinity (including three dimensional space) on c-Met;
(2) a substance that inhibits the binding between c-Met and MERMUC via direct binding to the c-Met binding site or its vicinity (including three dimensional space) on MERMUC;
(3) a substance that inhibits the binding between c-Met and MERMUC as a result of changing the three-dimensional structure of MERMUC or inhibiting the multimerization of MERMUC via the binding to an arbitrary region (e.g., the extracellular region) of MERMUC (for example, MERMUC-binding antibodies); and
(4) a substance that inhibits the binding between c-Met and MERMUC because of dissociating the bond of bound c-Met and MERMUC.

"A substance having activity to inhibit multimerization of MERMUC" of this invention includes any substance as long as it has activity to inhibit multimerization due to self-association and bonding of MERMUC molecules. Therefore, in the present invention, the mechanism (inhibition mechanism) by which the substance exhibits the activity of inhibiting multimerization of MERMUC molecules is not limited. Specifically, such substances include those functioning through the following inhibition mechanism. However, the present invention should not be construed as being limited to these examples:
(1) a substance that inhibits self-association (multimerization) of MERMUC via direct binding to the site at which 2 or more MERMUC molecules bind for self-association (specifically, the cytoplasmic region) or to its vicinity (including three dimensional space) ; and
(2) a substance that inhibits self-association (multimerization) of 2 or more MERMUC molecules as a result of changing the three-dimensional structure of MERMUC or by inhibiting multimerization of MERMUC via the binding to an arbitrary region (e.g. , the extracellular region) of MERMUC (for example, MERMUC-binding antibodies).

"Substances that suppress or inhibit the expression of MERMUC" of this invention include any substance as long as it has the activity of suppressing or inhibiting the expression of the MERMUC molecule on the cell membrane. Therefore, in the present invention, there are no limitations on the mechanism (inhibition mechanism) by which the substance exhibits this inhibitory activity. Specifically, such substances include those functioning through the following inhibition mechanism. However, the present invention should not be construed as being limited to these examples:
(1) a substance that suppresses or inhibits the transcription of a gene encoding MERMUC to mRNA; and
(2) a substance that inhibits or suppresses the translation of an mRNA encoding MERMUC to protein.

The above-mentioned (1) encompasses not only the following antisense DNA, but also substances having activity to modulate the activity of a promoter and/or a transcription factor that modulates the transcription of a gene encoding MERMUC to mRNA.

Each of the chemically synthesized substances (low-molecular weight compounds and such), polypeptides, antibodies, DNA and RNA encompassed by the "substances" composing the "pharmaceutical composition" of this invention is defined as follows.

Examples of the "antibodies" include MERMUC (particularly preferred, human MERMUC)-binding polyclonal antibodies, monoclonal antibodies and portions of the monoclonal antibodies. Monoclonal antibodies and portions thereof are preferred. The monoclonal antibodies include in addition to non-human mammal-derived monoclonal antibodies, recombinant chimeric monoclonal antibodies (Experimental Medicine (supplementary volume), 1988, Vol.1.6, No. 10; Examined Published Japanese Patent Application (JP-B) No. Hei 3-73280; etc.) , recombinant humanized monoclonal antibodies and human monoclonal antibodies (Nature Genetics, 1994, Vol.7, p.13-21; Nature Genetics, 1997, Vol.15, p.146-156; Published Japanese Translation of International Publications Nos. Hei 4-504365 and Hei 7-509137; Nikkei Science, June 1995, p.40-50; International Application No. WO 94/25585; Nature, 1994, Vol.368, p.856-859; Published Japanese Translation of International Publication No. Hei 6-500233; Nikkei Science, April 1997, p.78-84; etc.).

The term "polypeptide" (herein, it refers to polypeptides that are components of a pharmaceutical composition of this invention, and not to polypeptides themselves as another invention) encompasses oligopeptides, fusion polypeptides and chemically modified forms thereof. Examples of oligopeptides are peptides comprising 5 to 30 amino acids, preferably 5 to 20 amino acids. A chemical modification can be designed depending on various purposes, for example, to increase half-life in blood in the case of administering in vivo, or to increase tolerance against degradation or increase absorption in the digestive tract in oral administrations.

The term "DNA" means "DNA comprising a partial nucleotide sequence of an antisense DNA or a chemically modified DNA thereof", which DNA is designed based on the nucleotide sequence of the DNA (including cDNA and genomic DNA) encoding MERMUC, yet useful as an antisense DNA pharmaceutical. Specifically, the antisense DNA can inhibit the transcription of gene encoding MERMUC into mRNA or the translation of the mRNA into a protein by hybridizing to the gene or RNA encoding MERMUC.

The term "RNA" means "RNA comprising a partial nucleotide sequence of an antisense RNA or a chemically modified RNA thereof", which RNA is designed based on the nucleotide sequence of the RNA encoding the MERMUC, yet useful as an antisense RNA pharmaceutical. The antisense RNA can inhibit the transcription of the gene encoding MERMUC into mRNA or the translation of the mRNA into a protein by hybridizing to the gene or RNA encoding MERMUC.

The expression "partial nucleotide sequence" as employed herein, refers to a partial nucleotide sequence comprising an arbitrary number of nucleotides of an arbitrary region. A partial nucleotide sequence includes 5 to 100 consecutive nucleotides, preferably 5 to 70 consecutive nucleotides, more preferably 5 to 50 consecutive nucleotides, and even more preferably 5 to 30 consecutive nucleotides.

When the DNA or RNA is used as an antisense pharmaceutical, the DNA or RNA sequence can be chemically modified in part in order to extend the half-life (stability) in blood when the DNA or RNA is administered to patients, to increase the intracytoplasmic-membrane permeability of the DNA or RNA, or to increase the degradation resistance or the absorption of orally administered DNA or RNA in the digestive organs. Chemical modifications include, for example, the modification of a phosphate bond, a ribose, a nucleotide, the sugar moiety, and the 3' end and/or the 5' end in the structure of an oligonucleotide.

Modifications of phosphate bonds include, for example, the conversion of one or more bonds to phosphodiester bonds (D-oligo), phosphorothioate bonds, phosphorodithioate bonds (S-oligo), methyl phosphonate (MP-oligo) bonds, phosphoroamidate bonds, non-phosphate bonds or methyl phosphonothioate bonds, or combinations thereof. Modification of a ribose includes, for example, the conversion to 2'-fluororibose or 2'-0-methylribose. Modification of a nucleotide includes, for example, the conversion to 5-propynyluracil or 2-aminoadenine.

The phrase "chemically synthesized substance" or "low-molecular weight compound" refers to any compound except the above-mentioned antibodies, polypeptides, DNA and RNA, and examples include compounds having a molecular weight of approximately 100 to approximately 1000 or less, preferably approximately 100 to approximately 800, and more preferably approximately 100 to approximately 600.

The polypeptides, portions of the polypeptides (fragment) and fusion polypeptides mentioned above can be produced not only by recombinant DNA technology as mentioned below, but also by methods well known in the art, such as chemical synthetic methods or cell culture methods, or modified methods thereof.

The "antibodies that bind to MERMUC" of this invention include natural antibodies obtained by immunizing mammals such as mice, rats, hamsters, guinea pigs and rabbits, with an antigen, chimeric antibodies and humanized antibodies (CDR-grafted antibody) that may be produced using gene recombination techniques, and human antibodies that may be produced using human antibody-producing transgenic animals and such. The natural antibodies may be produced using cells (natural cells, established cell lines, tumor cells, etc.) that express MERMUC (particularly preferably human MERMUC) , transformants produced using gene recombination techniques so that MERMUC (particularly preferably human MERMUC) is overexpressed on the cell surface, polypeptides (particularly preferably polypeptides that compose the extracellular region) composing MERMUC (particularly preferably human MERMUC), or fusion polypeptides comprising all or a portion of the extracellular region of this MERMUC and all or a portion of another protein (human IgFc, glutathione-S-transferase, His tag, β-galactosidase, etc.) as antigens.

Monoclonal antibodies having any one isotype of IgG, IgM, IgA, IgD or IgE. IgG or IgM are preferred.

A polyclonal antibody (antisera) or monoclonal antibody can be produced by known methods. Specifically, a mammal, preferably, a mouse, rat, hamster, guinea pig, rabbit, cat, dog, pig, goat, horse or cow, or more preferably, a mouse, rat, hamster, guinea pig or rabbit is immunized, for example, with an antigen mentioned above with Freund's adjuvant, if necessary.

A polyclonal antibody can be obtained from the serum obtained from the animal so immunized. On the other hand, monoclonal antibodies are produced as follows. Hybridomas are prepared from the antibody-producing cells obtained from the animal so immunized and myeloma cells that are not capable of producing autoantibodies. The hybridomas are cloned, and clones producing the monoclonal antibodies showing a specific affinity to the antigen used for immunizing the mammal are screened.

Specifically, a monoclonal antibody can be produced as follows. Immunizations are performed by inj ecting or implanting once or several times an antigen mentioned above as an immunogen, if necessary, with Freund's adjuvant, subcutaneously, intramuscularly, intravenously, through the footpad, or intraperitoneally into a non-human mammal, specifically a mouse, rat, hamster, guinea pig or rabbit, preferably a mouse, rat or hamster (including a transgenic animal generated so as to produce antibodies derived from another animal, such as a transgenic mouse producing human antibody mentioned below). Usually, immunizations are performed once to four times every one to fourteen days after the first immunization. Antibody-producing cells are obtained from the mammal so immunized in about one to five days after the last immunization. The frequency and interval of immunizations can be appropriately arranged depending on, for example, the property of the immunogen used.

Hybridomas that secrete a monoclonal antibody can be prepared by the method of Köhler and Milstein (Nature, 1975, Vol.256, p.495-497), or by a modified method thereof. Specifically, hybridomas are prepared by fusing antibody-producing cells contained in a spleen, lymph node, bone marrow or tonsil, preferably a spleen, obtained from a non-human mammal immunized as mentioned above with myelomas without an autoantibody-producing ability, which are derived from, preferably, a mammal such as a mouse, rat, guinea pig, hamster, rabbit or human, or more preferably, a mouse, rat or human.

For example, a mouse-derived myeloma P3/X63-AG8.653 (653), P3/NSI/1-Ag4-1 (NS-1) , P3/X63-Ag8.U1 (P3U1) , SP2/0-Ag14 (Sp2/0, Sp2), PAI, F0 or BW5147, rat-derived myeloma 210RCY3-Ag.2.3., or human-derived myeloma U-266AR1, GM1500-6TG-A1-2, UC729-6, CEM-AGR, D1R11 or CEM-T15 can be used as a myeloma for cell fusion.

Hybridomas producing monoclonal antibodies can be screened by cultivating hybridomas, for example, in microtiter plates and by measuring the reactivity of the culture supernatant in wells in which hybridoma growth is observed, to the immunogen used for the immunization mentioned above, for example, by an enzyme immunoassay such as RIA and ELISA.

Monoclonal antibodies can be produced from hybridomas by cultivating the hybridomas *in vitro* or *in vivo* such as in the ascites fluid of a mouse, rat, guinea pig, hamster or rabbit, preferably a mouse or rat, more preferably mouse, and isolating the antibodies from the resulting culture supernatant or ascites fluid of a mammal.

*In vitro* cultivation of hybridomas can be performed depending on, e.g., the property of cells to be cultured, the object of the study and various conditions of the culture method, using known nutrient media or any nutrient media derived from known basal media for growing, maintaining and storing the hybridomas to produce monoclonal antibodies in the culture supernatant.

Examples of basal media are low calcium concentration media, such as Ham'F12 medium, MCDB153 medium or low calcium concentration MEM; and high calcium concentration media, such as MCDB104 medium, MEM, D-MEM, RPMI1640 medium, ASF104 medium or RD medium. The basal media can contain, for example, sera, hormones, cytokines, and/or various inorganic or organic substances depending on the objective.

Monoclonal antibodies can be isolated and purified from the culture supernatant or ascites fluid mentioned above by saturated ammonium sulfate precipitation, euglobulin precipitation method, caproic acid method, caprylic acid method, ion exchange chromatography (DEAE or DE52) and affinity chromatography using an anti-immunoglobulin column or a protein A column.

The phrase "a portion of an antibody" in this invention refers to a partial region of a monoclonal antibody such as those described above, and specifically include F(ab')₂, Fab', Fab, Fv (variable fragment of an antibody), sFv, dsFv (disulphide stabilized Fv) or dAb (single domain antibody) (Exp. Opine Ther. Patents, 1996, Vol.6, No.5, p.441-456).

The expression "pharmaceutically acceptable carrier" of this invention includes an excipient, a diluent, a filler, a disintegrating agent, a stabilizer, a preservative, a buffer, an emulsifier, an aromatic agent, a colorant, a sweetener, a thickening agent, a corrigent, a solubility-increasing agent or some other additive.

Using one or more of such carriers, pharmaceutical compositions of the present invention can be formulated into tablets, pills, powders, granules, injections, solutions, capsules, troches, elixirs, suspensions, emulsions, syrups, etc.

The pharmaceutical compositions of the present invention can be administered orally or parenterally. Other forms for parenteral administration include a solution for external application, suppository for rectal administration and pessary, prescribed by the usual method, which comprises one or more active ingredients.

The dosage of the pharmaceutical composition of the present invention can vary depending on the age, sex, weight and symptoms of a patient, effect of treatment, administration route, period of treatment, the kind of active ingredient (the "substance" according to the present invention, mentioned above) contained in the pharmaceutical composition, etc. Usually, the pharmaceutical composition can be administered to an adult at a dose of 10 µg to 1000 mg (or 10 µg to 500 mg) per one administration. Depending on various conditions, a dosage less than that mentioned above may be sufficient in some cases and a dosage more than that mentioned above may be necessary in others.

In the case of an injection, it can be produced by dissolving or suspending an antibody in a non-toxic, pharmaceutically acceptable carrier such as physiological saline or commercially available distilled water for injection adjusting the concentration in the range of 0.1 µg antibody/ml carrier to 10 mg antibody/ml carrier. The injection thus produced can be administered to a human patient in need of treatment in the dose range of 1 µg to 100 mg/kg body weight, preferably in the range of 50 µg to 50 mg/kg body weight, one or more times a day. Examples of administration routes are medically appropriate administration routes such as intravenous injection, subcutaneous injection, intradermal injection, intramuscular injection, intraperitoneal inj ection or such, preferably intravenous injection.

The injection can also be prepared into a non-aqueous diluent (for example, propylene glycol, polyethylene glycol, vegetable oil such as olive oil, and alcohol such as ethanol), suspension or emulsion.

The injection can be sterilized by filtration with a bacteria-filtering filter, by mixing a bactericide or by irradiation. The injection can be produced in such a manner that it is prepared at the time of use. Namely, it is freeze-dried to be a sterile solid composition that can be dissolved in sterile distilled water for injection or another solvent before use.

The pharmaceutical composition of the present invention can be used alone or in combination with the above-mentioned HGF drug for maintaining or promoting repair, regeneration or neogenesis of tissues and organs required for the prevention and/or treatment of various diseases. Therefore, the use of the pharmaceutical composition alone or in combination with the HGF drug enables treatment of various intractable diseases (for example, hepatic fibrosis, hepatic cirrhosis, fulminant hepatitis, viral hepatitis, acute hepatitis, chronic renal failure, renal fibrosis, pulmonary fibrosis, etc.) which treatment is considered difficult today.

In the combined use of the pharmaceutical composition (drug A) of this invention with the HGF drug (drug B) , drug A and drug B can be administered to a patient in any order and any dosage form depending on the purpose of treatment.

When the pharmaceutical composition of this invention is used alone, the biological activity originally possessed by endogenous HGF in the body of a patient is elicited by the active ingredient contained in the pharmaceutical composition, which removes the down-regulation of HGF activity that occurs due to the binding between the HGF receptor c-Met and MERMUC, to accomplish the therapeutic effect.

On the other hand, when the pharmaceutical composition of this invention is used in combination with the HGF drug, the dose of the HGF drug used in treating disorders such as those described above can be kept minimum by the active ingredient contained in the pharmaceutical composition, which removes the down-regulation of HGF activity that occurs due to the binding between HGF receptor c-Met and MERMUC.

In the present invention, "fibrosis" includes tissue fibrosis in any organ, and particularly preferable examples include pulmonary fibrosis, hepatic fibrosis or renal fibrosis.

In the present invention, hepatitis encompasses any inflammation of the liver elicited by any cause, and particularly preferable examples include fulminant hepatitis, acute hepatitis (inflammation elicited by liver toxins and such, due to viruses and pharmaceutical agents) or viral hepatitis (inflammation elicited by various viruses, in particular, hepatitis viruses such as type C hepatitis).

The pharmaceutical composition of the present invention can be used for treating (including preventing) any disorder for which regeneration or neogenesis of tissues is necessary for its treatment, and particularly preferred disorders include various hepatic disorders (e.g., hepatic fibrosis, hepatic cirrhosis, fulminant hepatitis, viral hepatitis, acute hepatitis, etc.), renal disorders (e.g., chronic kidney failure, renal fibrosis, etc.), and pulmonary disorders (e.g., pulmonary fibrosis). Such disorders are all representative intractable diseases, and thus, in other words, the pharmaceutical compositions of this invention are useful for treating or preventing such intractable diseases.

Furthermore, the present invention relates to a "method for identifying a substance having the activity to inhibit the binding between c-Met and MERMUC" (same as the definition indicated above) and a "method for testing the presence or extent of activity of a certain substance to inhibit the binding between c-Met and MERMUC". Such methods comprise, for example, the following steps of:
(a) preparing a cell that is designed to coexpress c-Met and MERMUC, and upon binding of c-Met and MERMUC, allow detection of their binding;
(b) culturing the cell obtained in step (a) in the presence of HGF while in the absence of test substance, and then measuring the extent of the binding between c-Met and MERMUC in the cell;
(c) culturing the cell obtained in step (b) in the presence of HGF and a test substance, and then measuring the extent of the binding between c-Met and MERMUC in the cell; and
(d) comparing the measurement result of step (b) with that of step (c).

Herein, host cells used for producing the cell of step (a) are not particularly limited as long as they are cells normally used for the production of recombinant proteins, and include any of the various cells of such as natural cells or artificially established recombinant cells (for example, bacteria (*Escherichia, Bacillus*, etc.), yeast (*Saccharomyces*, *Pichia,* etc.), animal cells, insect cells, etc.). More specifically, it may be any one of *E*. *coli* (DH5α, TB1, HB101, etc.) , mouse-derived cells (COP, L, C127, Sp2/0, NS-1, NIH3T3, etc.), rat-derived cells (PC12, PC12h), hamster-derived cells (BHK, CHO, etc.) , monkey-derived cells (COS1, COS3, COS7, CV1, Velo, etc.) and human-derived cells (HeLa, diploidfibroblast-derived cells, myeloma cells, HepG2, etc.). Animal cells such as those mentioned above are particularly preferred.

A favorable example of c-Met (full-length or a portion thereof) of step (a) includes a fusion polypeptide formed between all or a part of human c-Met defined above (a particularly preferred example includes those containing at least the binding site with human MERMUC) and β-galΔα or β-galΔω.

A favorable example of MERMUC (full-length or a portion thereof) of step (a) includes a fusion polypeptide formed between all or a part of human MERMUC defined above (a particularly preferred example includes those containing at least the binding site with human c-Met) and β-galΔα or β-galΔω.

By using the fusion polypeptide mentioned above, the presence, extent, or increase or decrease of the binding with MERMUC can be detected as β-galactosidase activity expressed within the cell sample (Proc. Natl. Acad. Sci. USA., 1997, Vol.94, p.8405-8410).

Identification or testing of compounds using the method of this invention can be performed manually. However, a quicker and more easy identification or testing can be achieved by using the so called high throughput screening, which is performed automatically using a machine (robot) (Saibo Baiyo Kogaku (Tissue Culture Engineering), 1997, Vol.23, No.13, p.521-524; US Patent No. 5,670,113).

Substances identified by the method of the present invention are used as active ingredients of the pharmaceutical composition of this invention.

### Brief Description of the Drawings

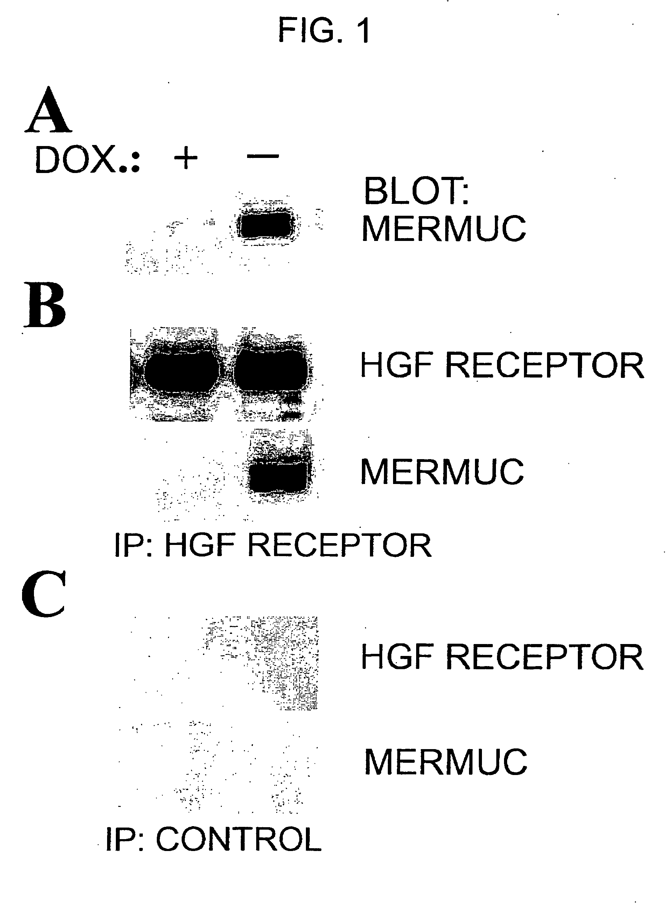

Fig. 1 depicts photographs showing the production of human MERMUC and/or human c-Met in recombinant human MERMUC-expressing HEK293 cells analyzed by Western blotting.
   (A) depicts a photograph showing the production of human MERMUC in recombinant cells cultured in the presence or absence of Dox analyzed by Western blotting using anti-MERMUC antibody.
   (B) depicts a photograph showing the production of human c-Met and MERMUC (coprecipitated with c-Met) in recombinant cells cultured in the presence or absence of Dox analyzed by Western blotting using anti-c-Met or anti-MERMUC antibody.
   (C) depicts a photograph showing the production of human c-Met and MERMUC (coprecipitated with c-Met) in recombinant cells cultured in the presence or absence of Dox analyzed by Western blotting using a control antibody.

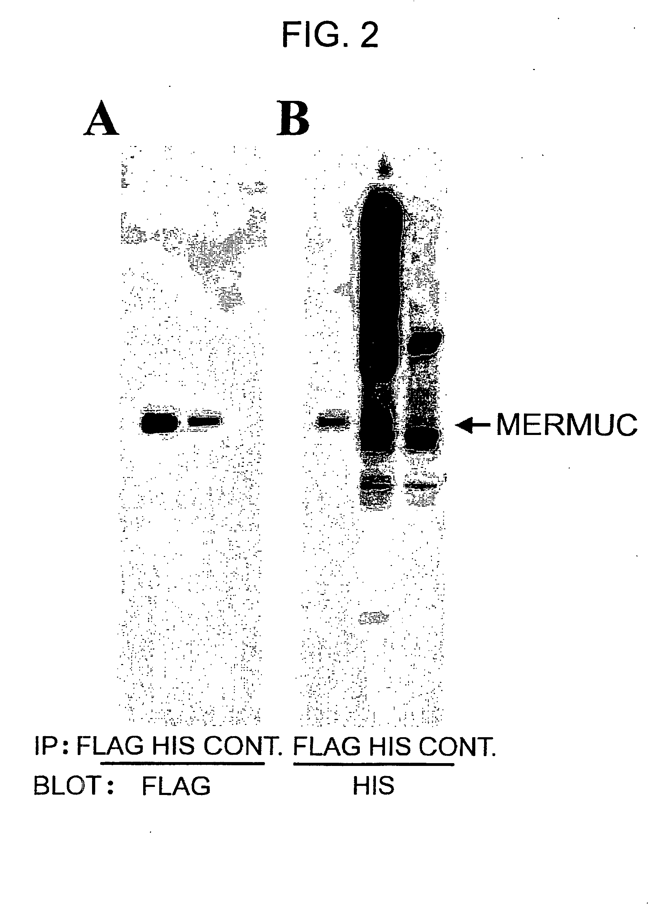

Fig. 2 depicts photographs showing the production of multimerized human MERMUC in recombinant human MERMUC-expressing HEK293 cells analyzed by Western blotting.
   (A) depicts a photograph showing the production of FLAG-tagged human MERMUC in various samples analyzed by Western blotting using anti-FLAG-tag antibody.
   (B) depicts a photograph showing the production of poly HIS-tagged human MERMUC in various samples analyzed by Western blotting using anti-poly HIS-tag antibody.
Fig. 3 depicts photographs showing the expression of human MERMUC mRNA in various human tissues analyzed by Northern blotting.
Fig. 4 depicts a photograph showing the expression of human MERMUC gene in various human tissues analyzed by PCR.
Fig. 5 depicts photographs showing the distribution of mouse MERMUC gene expression in various types of mouse kidney tissues analyzed by *in situ* hybridization. The letters G, PT and DT in the figure indicate glomerulus, proximal tubule and distal tubule, respectively.
   (a) indicates the expression distribution of MERMUC gene in kidney tissue of a MRL-lpr/lpr mouse (5 weeks old) analyzed using antisense cRNA probe.
   (b) indicates the expression distribution of MERMUC gene in kidney tissue of a MRL-lpr/lpr mouse (5 weeks old) analyzed using sense cRNA probe.
   (c) indicates the expression distribution of MERMUC gene in kidney tissue of a MRL-lpr/lpr mouse (23 weeks old) analyzed using antisense cRNA probe.
   (d) indicates the expression distribution of MERMUC gene in kidney tissue of a C57BL/6 mouse (23 weeks old) analyzed using antisense cRNA probe.
   (e) indicates the expression distribution of MERMUC gene in kidney tissue of a C57BL/6 mouse (23 weeks old) analyzed using sense cRNA probe.

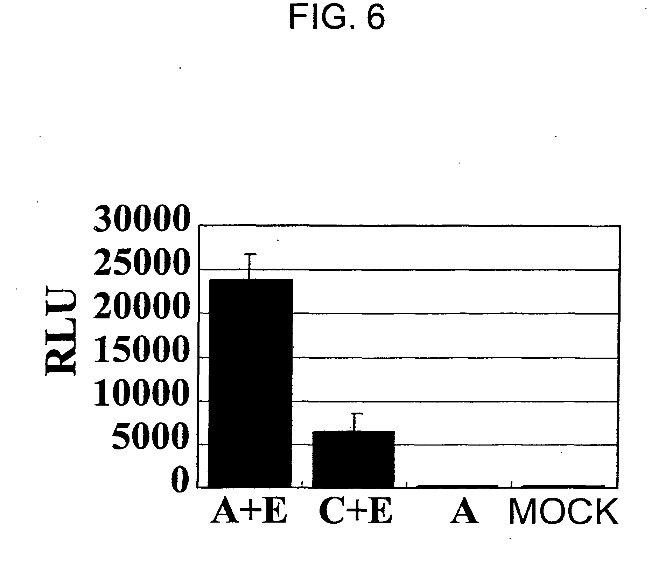

Fig. 6 shows the results of evaluating the binding of MERMUC with HGF receptor c-Met using the expression of β-gal activity as an indicator.

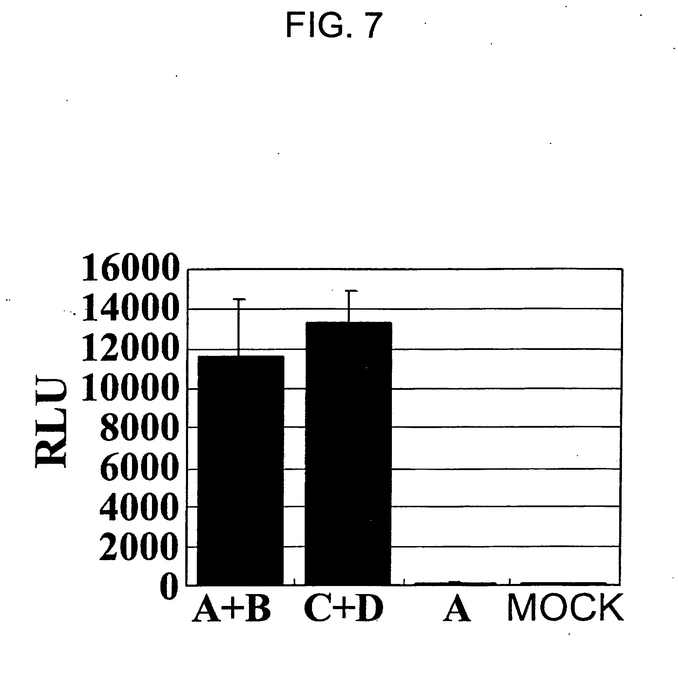

Fig. 7 shows the results of evaluating the intermolecular binding of MERMUC using the expression of β-gal activity as an indicator.

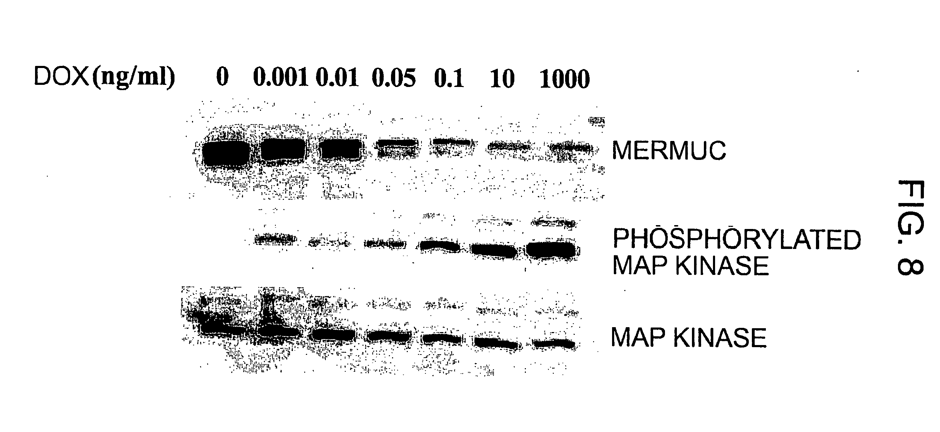

Fig. 8 depicts photographs showing the decrease in HGF-induced intracellular MAP kinase activity due to the increase in the production of MERMUC analyzed by Western blotting.

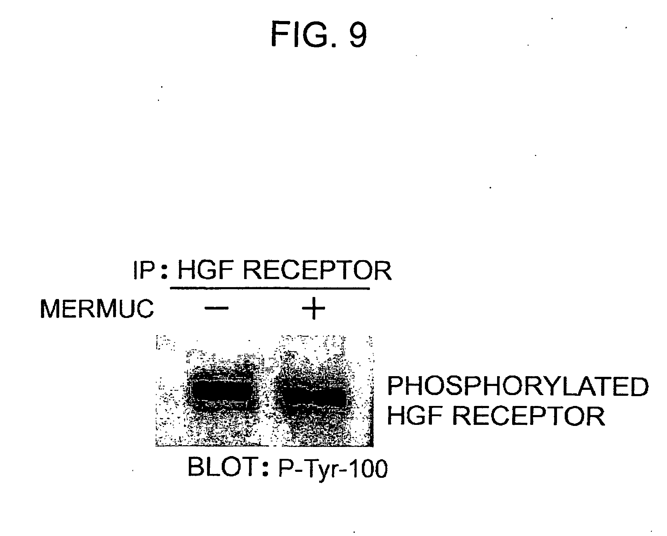

Fig. 9 depicts a photograph showing the difference in phosphorylated HGF receptor c-Met production between HGF-stimulated MERMUC producing cells and non-producing cells analyzed by Western blotting.

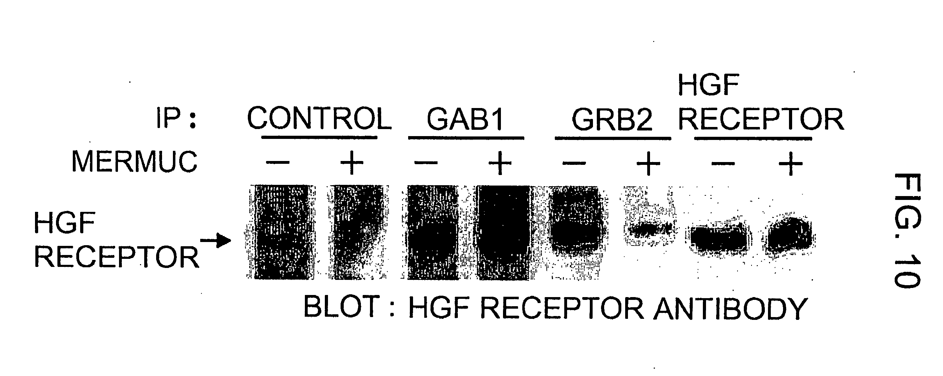

Fig. 10 depicts a photograph showing the difference in HGF receptor c-Met-bound Grb2 and Gab1 production between HGF-stimulated MERMUC producing cells and non-producing cells analyzed by Western blotting.

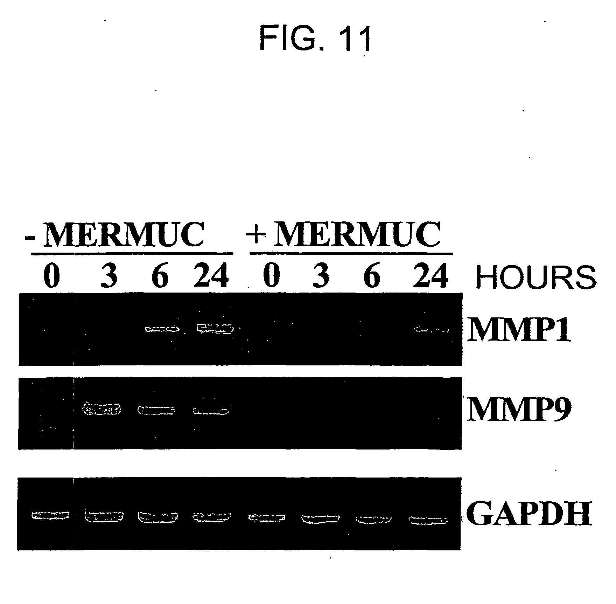

Fig. 11 depicts photographs showing the difference in MMP-1 and MMP-9 mRNA expressions between HGF stimulated MERMUC producing cells and non-producing cells analyzed by RT-PCR.

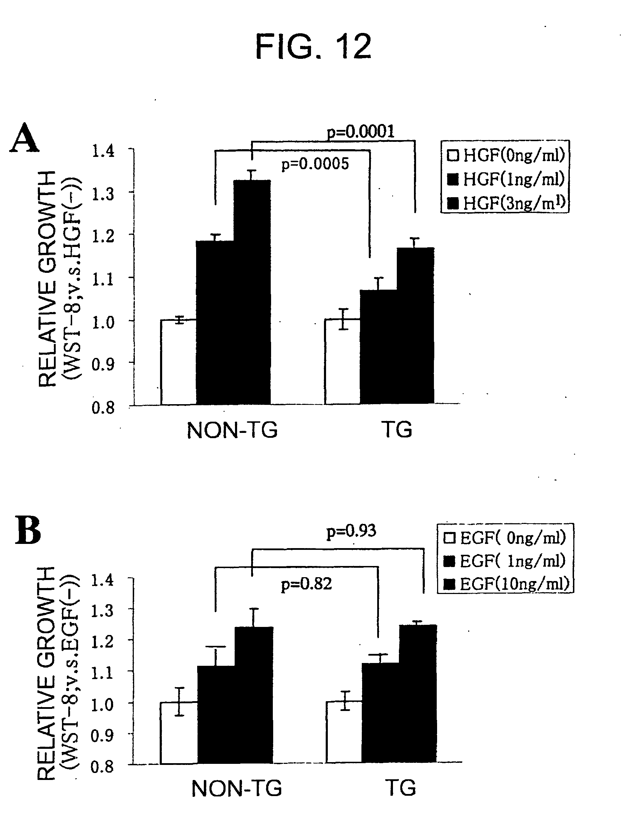

Fig. 12 shows the degree of cell proliferation in response to HGF or EGF in hepatocytes derived from MERMUC overexpressing transgenic mice and normal mice, respectively.
   (A) indicates the degree of cell proliferation in response to HGF.
   (B) indicates the degree of cell proliferation in response to EGF.

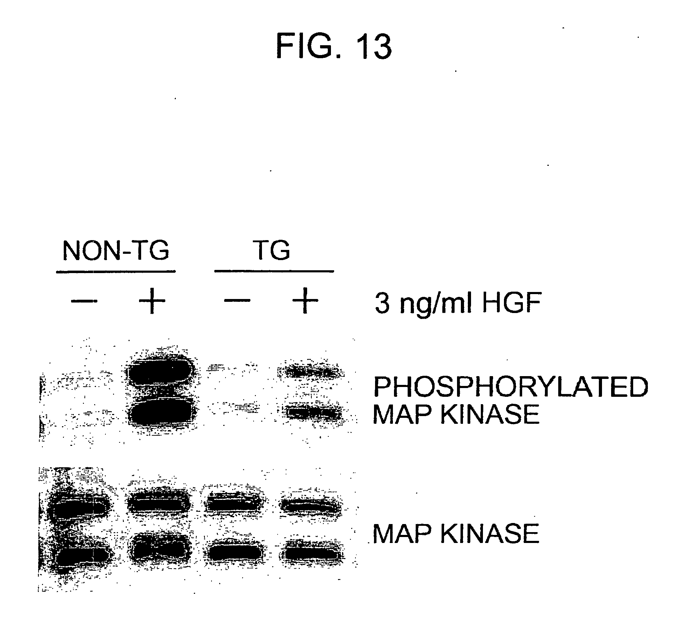

Fig. 13 depicts photographs showing the intensity of HGF-induced MAP kinase activity in hepatocytes of MERMUC overexpressing transgenic mice and normal mice, respectively, analyzed by Western blotting.

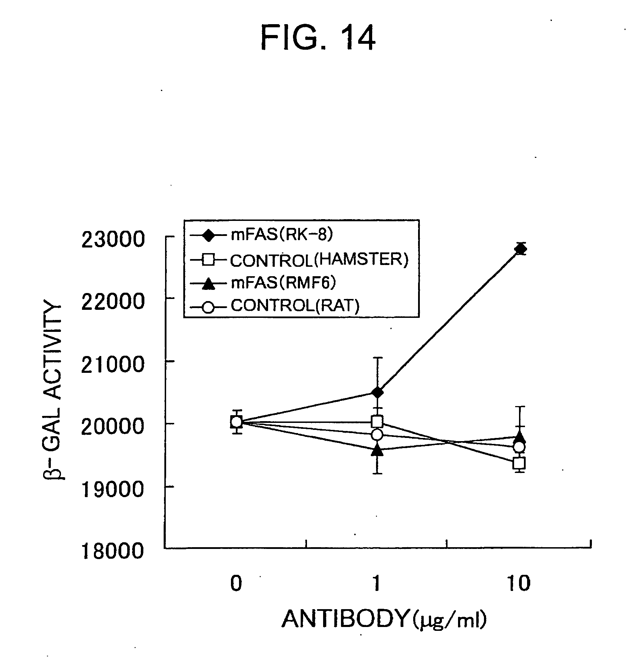

Fig. 14 shows the results of evaluating the presence of self-association of mFas-MERMUC fusion protein at MERMUC region using the expression of β-gal activity as an indicator.

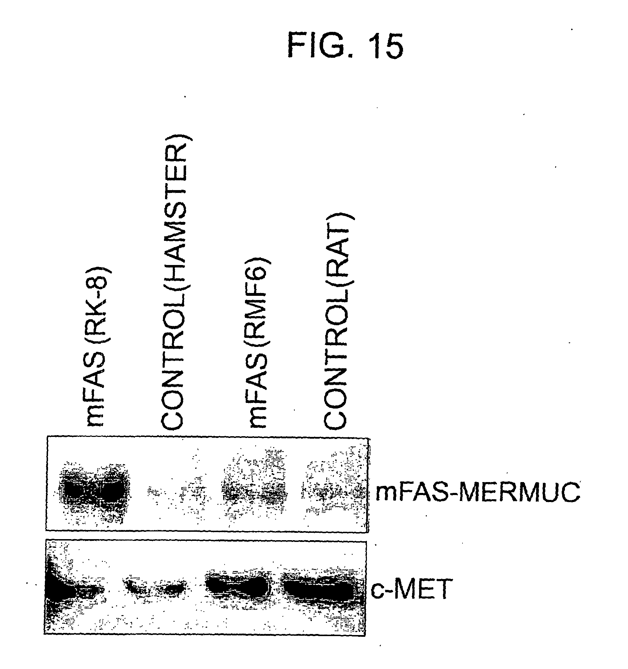

Fig. 15 depicts photographs showing the amount of self-associated MERMUC co-precipitating with the HGF receptor analyzed by Western blotting.

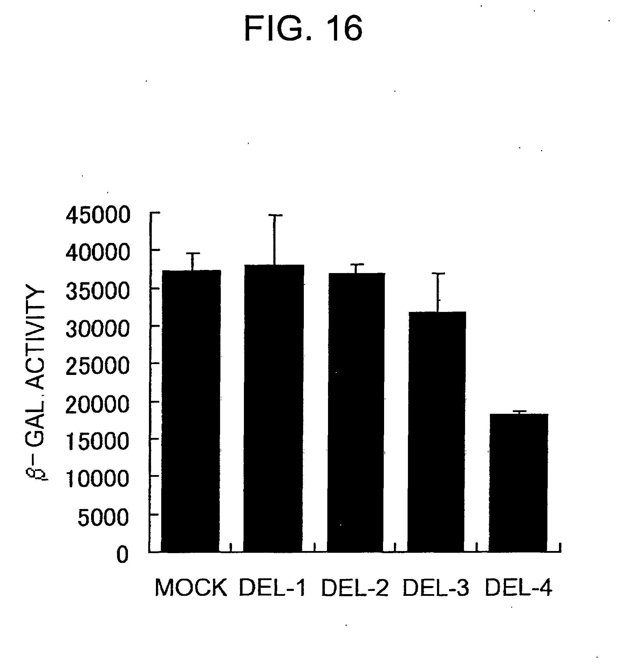

Fig. 16 shows the results of evaluating the degree of suppression of MERMUC self-association due to various MERMUC cytoplasmic region peptides using the expression of β-gal activity as an indicator.

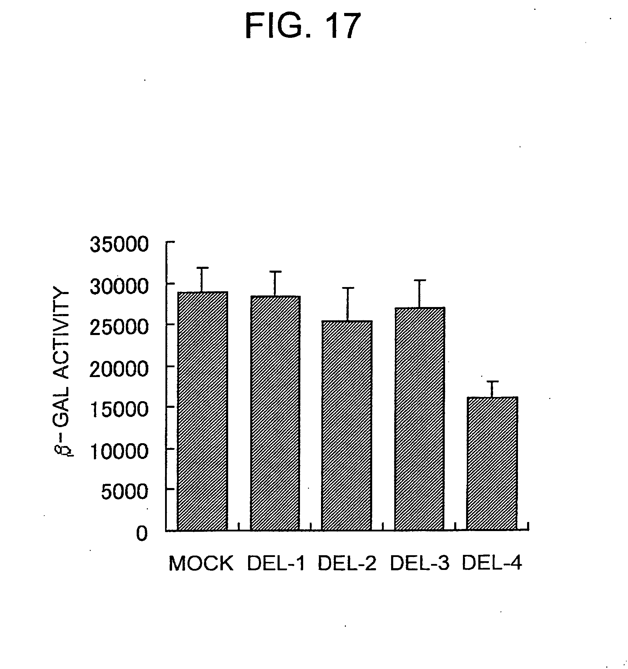

Fig. 17 shows the results of evaluating the degree of suppression of MERMUC binding to HGF receptor c-Met due to various MERMUC cytoplasmic region peptides using the expression of β-gal activity as an indicator.

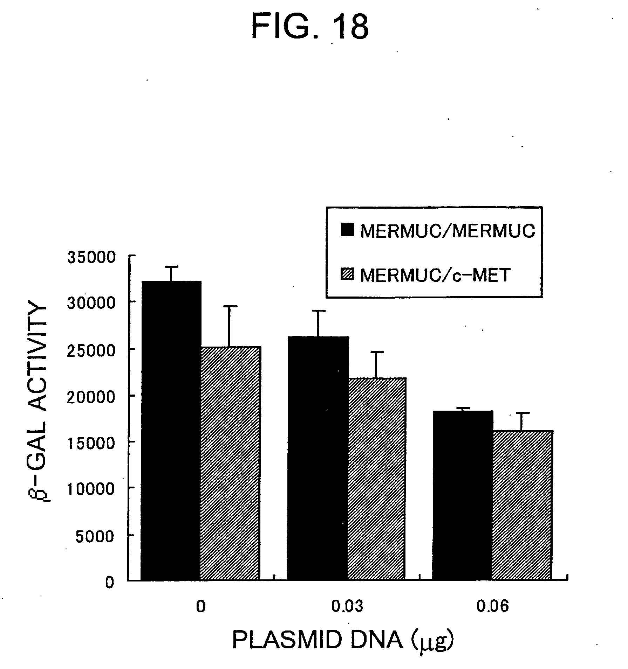

Fig. 18 shows the results of evaluating the degree of suppression of self-association of MERMUC and the binding of MERMUC to HGF receptor c-Met due to various concentrations of MERMUC cytoplasmic region peptide (del-4) using the expression of β-gal activity as an indicator.

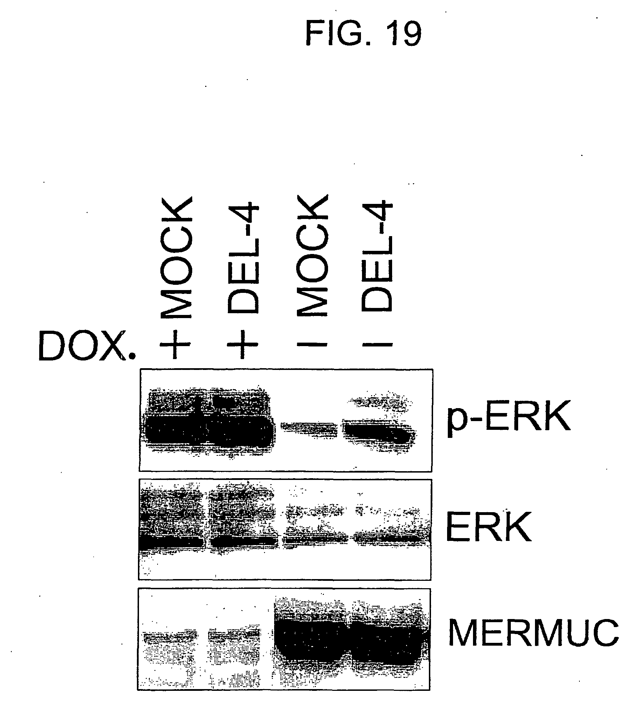

Fig. 19 depicts photographs showing the existence of MERMUC expression in the presence or absence of Dox, and the intensity of phosphorylated MAP kinase (p-Erk) production depending on the presence or absence of suppression of MERMUC self-association due to the MERMUC cytoplasmic region peptide (del-4) analyzed by Western blotting.

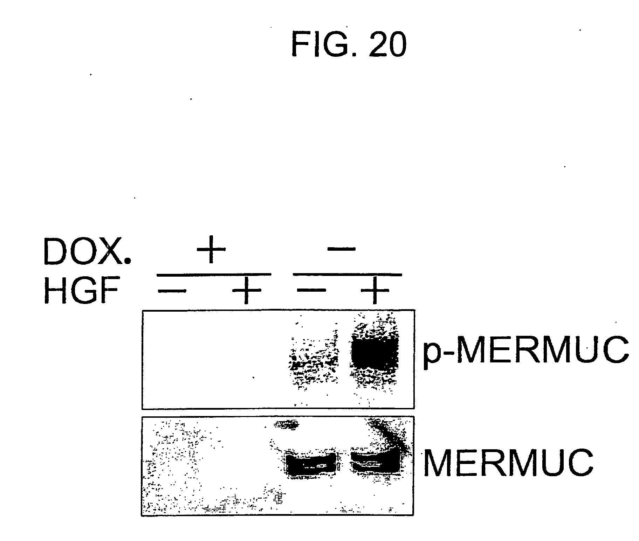

Fig. 20 depicts photographs showing the existence of MERMUC expression in the presence or absence of Dox and/or the existence of phosphorylated MERMUC production in the presence or absence of HGF stimulation.

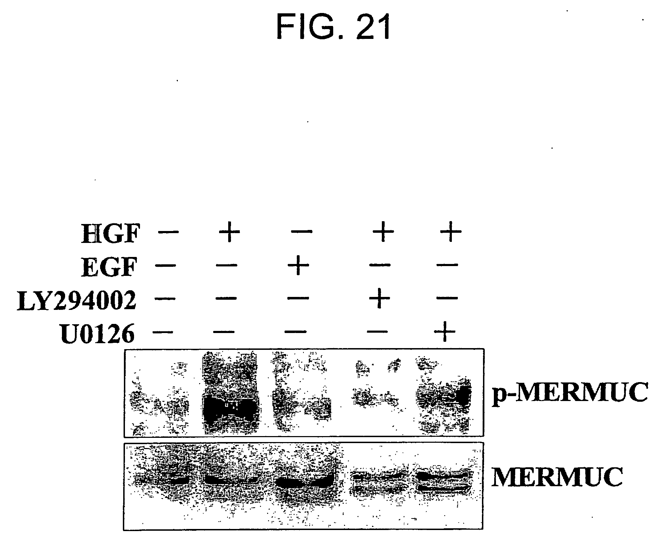

Fig. 21 depicts photographs showing the difference in phosphorylated MERMUC productions in the presence or absence of HGF and/or HGF signaling suppressor by immunoprecipitation.

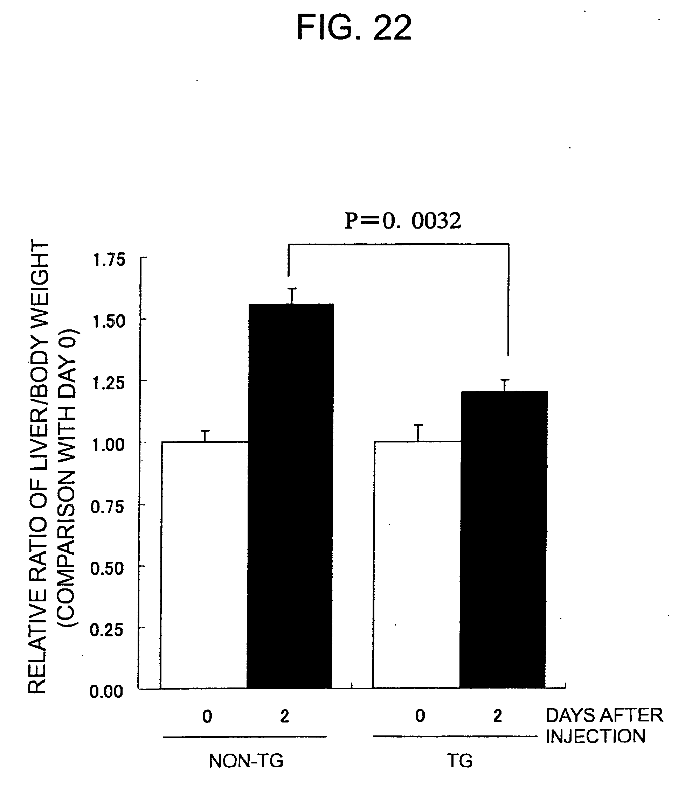

Fig. 22 shows the degree of HGF-dependent liver enlargement in MERMUC over expressing Tg and normal mice.

### Best Mode for Carrying out the Invention

The present invention will be described in detail below with reference to the Examples, but is not to be construed as being limited thereto.

### [Example 1] Identification of molecules binding to human MERMUC

Human genome analysis revealed that the gene encoding human MERMUC exists on chromosome 3 and comprises 3 exons (Ex.-1, Ex.-2 and Ex.-3) .

The primary structure (amino acid sequence) of every exon in human and mouse MERMUC polypeptides were compared. The homology between human and mouse at the region corresponding to Ex.-1 was low, of about 30%, however, that at the regions corresponding to Ex.-2 and Ex.-3 (each region comprises approximately 50 amino acids) were extremely high, i.e., 71% and 60%, respectively. Therefore, based on the analysis of the primary structure of the MERMUC peptides alone, these regions Ex.-2 and Ex.-3 corresponding to the C-terminal region of the MERMUC cytoplasmic region were suggested to be involved in some kind of important role for the expression of MERMUC function.

Thus, to reveal the function of MERMUC, the clarification of existence and identification of molecules that bind to the C-terminal region of MERMUC cytoplasmic region was performed.

Specifically, yeast two hybrid system (Proc. Natl. Acad. Sci. USA., 1991, Vol.88, p.9578-9582) was performed to screen a human kidney cDNA library using cDNA corresponding to the C-terminal region of the human MERMUC as the bait (that is, fishing bait).

Two kinds of bait (bait-2 and bait-3) were designed using each of Ex.-2 and Ex.-3 as the core. Bait-2 corresponds to a 123 amino acid portion of the C-terminus (bait-2) and bait-3 corresponds to the 65 C-terminal amino acids (bait-3).

Both baits were prepared by polymerase chain reaction (PCR) according to conventional methods using the full-length cDNA encoding human MERMUC as the template. Both of the two forward primers (<bait-2> SEQ ID NO: 18 and <bait-3> SEQ ID NO: 19) used for the PCR contain an *Eco*RI site at their 5' ends and a reverse primer (SEQ ID NO: 20) common to both baits contained a *Sal*I site at its 5' end.

Each bait vector was obtained by inserting each DNA fragment obtained by digesting each of the PCR products with the restriction enzymes *Eco*RI and *Sal*I into the *Eco*RI/*Sal*I site of pGBKT7 vector (leucine biosynthesis gene as a marker; CLONTECH). In yeast, the gene within the vector is produced as a fusion protein ([fusion protein A]) with the DNA binding region (GAL4-DB) of the yeast transcription factor GAL4.

On the other hand, each cDNA of the human kidney derived cDNA library (CLONTECH) that is used for the screening with the bait vector is inserted in pADK vector (tryptophan biosynthesis gene as a marker) . Therefore, proteins produced through the expression of each of these cDNAs in yeast are produced as a fusion protein ([fusion protein B]) with the GAL4 transcriptional activation region (GAL4-AD).

AH109 strain (leucine, tryptophan, histidine and adenine auxotrophic yeast; CLONTECH) was used as the yeast host. This yeast strain contains 3 types of genes (histidine biosynthesis gene, adenine biosynthesis gene and β-galactosidase gene) under the control of the GAL promoter on the chromosome.

The function as a transcription factor is recovered through the fusion of GAL4-DB and GAL4-AD by the binding of the bait protein (the fusion protein A) with the prey protein (the fusion protein B). As a result, the expression of the 3 genes is induced to supplement the histidine and adenine requirement, and exhibit β-galactosidase (β-gal) activity.

Each bait vector was introduced into yeast AH109 strain with the cDNA library plasmid mentioned above (50,000 clones per pool) by the polyethylene glycol/lithium acetate method for screening (J. Bacteriol., 1983, Vol. 153, p.163-16883; Curr. Genet., 1989, Vol. 16, p. 339-346; Nucleic Acids Res., 1991, Vol. 19, p. 5791; Nucleic Acids Res., 1992, Vol. 20, p.1425).

The marker for the first selection of yeast with the desired transformation in the present screening was acquired histidine non-requirement due to the expression of the histidine biosynthesis gene. Therefore, after introducing the plasmid, yeast was cultured for 5 days at 30°C in yeast minimum medium that lacks leucine, tryptophan and histidine (SD/-Leu,-Trp,-His) used for selecting transformants.

Transformed yeast that grew on the plate upon cultivation were picked with tooth pick and seeded on 3 kinds of plates described below, and then cultured for 5 days at 30°C for a second selection of the desired clones:
<A> plate containing yeast minimum medium lacking leucine, tryptophan and histidine (SD/-Leu, -Trp, -His);
<B> plate containing yeast minimum medium lacking leucine, tryptophan and adenine (SD/-Leu, -Trp, -Ade); and
<C> plate containing yeast minimum medium lacking leucine and tryptophan, and supplemented with α-X-gal (20ug/ml) (SD/-Leu, -Trp, +X-gal).

Positive clones were identified as colonies growing on both of the above-mentioned <A> and <B> plates (acquirement of histidine and adenine non-requirement) and showing blue color on plate <C> (α-X-gal addition) (expression of β-galactosidase activity). Four prey cDNA clones (clones No.3-309, No.3-312, No.2-22 and No. 2-211) were selected as positive. Plasmids possessing prey cDNA fragments carried by the positive yeast clones were transferred into *E. coli* from the yeast by the method described by Marcil and Higgins (Nucleic Acids Res., 1992, Vol. 20, p. 917).

The nucleotide sequences of the DNA fragments corresponding to the prey cDNAs were determined through nucleotide sequence determination procedure by the dideoxy method after recovering the plasmid DNAs from *E. coli*. Based on the determined nucleotide sequences of the prey cDNAs, searching and data analysis were performed using widely used gene databases.

As a result, the proteins derived from two clones (No.3-309 and 3-312) among the four positive clones had binding ability to both the proteins derived from bait-2 and bait-3. Moreover, the proteins corresponded to the C-terminal region of the β chain of c-Met that is the human HGF receptor. Clones No.3-309 and No.3-312 corresponded to the C-terminal 91 amino acids (amino acids 993-1083 of SEQ ID NO: 4) and the C-terminal 89 amino acids (amino acids 995-1083 of SEQ ID NO: 4) of the human c-Met β chain, respectively.

Moreover, proteins derived from two other positive clones (No.2-22 and 2-211) had binding ability to the protein derived from bait-2 and corresponded to the C-terminal region of human MERMUC itself. Clones No.2-22 and No.2-211 corresponded to the C-terminal 93 amino acids (amino acids 411-503 of SEQ ID NO: 10) and the C-terminal 56 amino acids (amino acids 448-503 of SEQ ID NO: 10) of human MERMUC, respectively.

### [Example 2] Binding of full-length MERMUC protein and full-length HGF receptor c-Met protein

### <2-1> Preparation of MERMUC expressing recombinant cells whose expression can be regulated by a drug

A recombinant cell that can regulate the expression of human MERMUC gene by Doxicycline (Dox) was prepared using the reported Tetracycline-controllable expression system (Tet system). Specifically, the recombinant cell possess a characteristic (Tet-off system) wherein the expression of the MERMUC gene is regulated depending on the concentration of Dox contained in the medium.

The cell was prepared using a commercially available pBI-L vector, pTK-Hyg vector and HEK293 Tet-Off cell (all from CLONTECH) and experimental procedures followed the experimental protocol provided with the experimental tool.

Specifically, a MERMUC expression vector was prepared by inserting cDNA (SEQ ID NO: 9) encoding the full-length human MERMUC into the multicloning site of the pBI-L vector. Next, HEK293 Tet-Off cell was cotransfected with the MERMUC expression vector and the pTK-Hyg vector (HEK293 cell strain derived from human kidney expresses the human HGF receptor c-Met). Cell strains stably trans fected with the MERMUC expression vector were selected using the presence of hygromycin resistance as an indicator. Then, a cell strain (dubbed MERMUC/203 cell) with high MERMUC gene expression efficiency and having a characteristic to enable precise regulation of the expression by DOX were further selected from the selected cell group. The selection was performed through the analysis of the expressed MERMUC protein by Western blotting using anti-human MERMUC polyclonal antibody.

### <2-2> Analysis of binding between human MERMUC and human HGF receptor c-Met by immunoprecipitation

Immunoprecipitation was performed by adding anti-c-Met antibody into the cell lysate prepared from MERMUC/293 cells as described above. The binding between full-length MERMUC protein with full-length HGF receptor c-Met protein was evaluated whether they coimmunoprecipitate or not.

Polyclonal antibody against human MERMUC used in the present assay was prepared according to a conventional method by immunizing a rabbit with a peptide corresponding to the 17 amino acids (SEQ ID NO.21) near the N-terminus. On the other hand, commercially available polyclonal antibody h-Met (C-28) (sc-161; Santa Cruz Biotechnology) was used as human c-Met antibody.

The anti-human c-Met polyclonal antibody was added to the cell lysate prepared from MERMUC/293 cells for reaction. The reaction solution was reacted with microbeads-bound protein-G Sepharose, and then centrifuged to immunoprecipitate the complex of c-Met and anti-c-Met polyclonal antibody.

The precipitate was then dissolved in electrophoresis buffer and the sample dissolved in the buffer was separated by SDS polyacrylamide gel electrophoresis (SDS-PAGE). Then, Western blotting was performed using anti-human MERMUC polyclonal antibody according to conventional method.

As a result, the existence of human MERMUC was detected (Fig.1) in the proteins immunoprecipitated with anti-human c-Met polyclonal antibody. This result reproduces the binding of full-length MERMUC with full-length HGF receptor c-Met in actual cells, and indicates the possibility that MERMUC modulates the signal transduction via the HGF/HGF receptor through the binding to c-Met.

### [Example 3] Analysis of intermolecular bindings (multimerization) of human MERMUC by immunocoprecipitation

An intermolecular binding activity between MERMUC molecules was predicted from the result of Example 1. Therefore, the existence of intermolecular binding of full-length MERMUC protein was analyzed.

### <3-1> Construction of full-length human MERMUC expression vector

Expression vectors expressing full-length human MERMUC with two different tags were constructed as follows. One vector expresses MERMUC added with a FLAG-tag at the C-terminus of MERMUC and the other expresses poly HIS-tagged MERMUC.

cDNAs encoding each of the tagged full-length MERMUC proteins were prepared by conventional PCR method using cDNA (SEQ ID NO: 9) encoding the full-length human MERMUC. Primer sets used for the PCR were as follows:

### <FLAG-tagged MERMUC>

forward primer: SEQ ID NO: 22, and
reverse primer: SEQ ID NO: 23; and

### <polyHIS-tagged MERMUC>

forward primer: SEQ ID NO: 22, and
reverse primer: SEQ ID NO: 24.

Each of the obtained PCR products was digested with restriction enzymes *Hind*III and *Xho*I, inserted into the *Hind*III and *Xho*I site of pcDNA3 vector, and then transformed into *E. coli.* Each plasmid for mammalian cell expression used in the present assay was prepared from the transformed *E*. *coli*.

A mixture containing equal amounts of each of the two expression vectors prepared above was added and cultured at 37°C for 48 hr to cotransfect HEK293 cells (human kidney derived cell strain) using GeneJammer Transfection Reagent (STRATEGENE) following the experimental protocol provided with the reagent.

The cell lysate prepared from the obtained transfected cells was treated for immunoprecipitation with anti-polyHIS polyclonal antibody (Santa Cruz Biotechnology), anti-FLAG monoclonal antibody (Anti-FLAG M2 monoclonal antibody; #F3165; Sigma) or control monoclonal antibody.

Next, the presence of each of the FLAG-tagged MERMUC and polyHIS-tagged MERMUC in the immunoprecipitated protein was analyzed by Western blotting using anti-FLAG monoclonal antibody and anti-polyHIS polyclonal antibody according to conventional method.

The results are shown in Fig. 2.

As a result, both of the FLAG-tagged MERMUC and polyHIS-tagged MERMUC were identified when anti-FLAG monoclonal antibody was used for immunoprecipitation. Furthermore, both of the tagged MERMUC were also identified when anti-poly HIS polyclonal antibody was used for immunoprecipitation.

These results indicate that MERMUC forms a multimer (multimerizes) via intermolecular binding of monomers on the cell membrane.

### [Example 4] Tissue distribution of human MERMUC expression

The expression of human HGF receptor c-Met is reported to be particularly high not only in liver, kidney and prostate, but also in digestive organs, such as small intestine and large intestine (Int. J. Cancer, 1991, Vol. 49, p. 323-328; Oncogene, 1991, Vol. 6, p. 1977-2003).

### <4-1> Analysis by Northern blot method

Thus, tissue distribution of MERMUC expression was analyzed by Northern blotting according to conventional method in order to analyze the physiological importance of the binding between MERMUC and HGF receptor c-Met.

Three commercially available membranes (Human 12-lane MTN Blot, Human MTN Blot II and Human Digestive MTN Blot; all from CLONTECH) blotted with human tissue derived mRNAs (2µg/lane) were used. Furthermore, a DNA fragment (about 1.5 kbp) comprising the cDNA region encoding the full-length human MERMUC that was prepared through the amplification by PCR was used as a probe. Hybridization was performed at 65°C over night. Membranes were washed with 4x SSC (0.1% SDS) for 15 min, and 2x SSC (0.1% SDS) for 15 min after the hybridization.

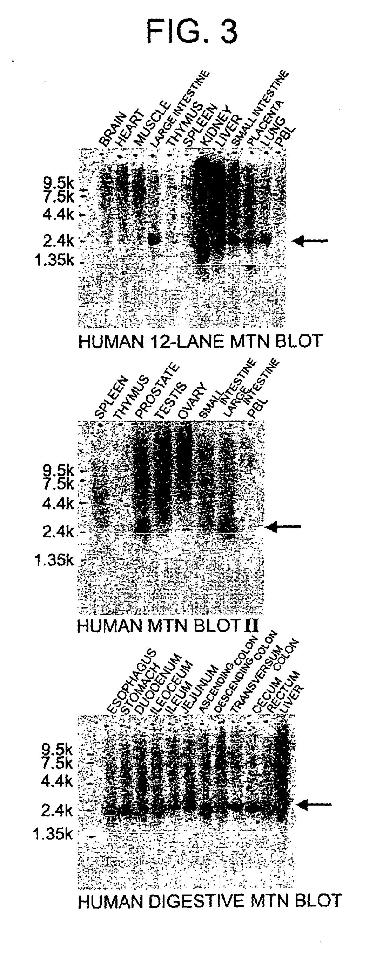

The results are shown in Fig. 3

A band of approximately 2.4 kbp, corresponding to MERMUC mRNA was detected in kidney, prostate, liver, lung, placenta, and digestive organs such as small intestine and large intestine. This expression distribution matched with that of the HGF receptor c-Met.

### <4-2> Analysis by PCR method

Furthermore, the expression of the MERMUC gene in some of the organs was analyzed by methods utilizing PCR.

Specifically, analyses were conducted by PCR (35 cycles at the annealing temperature 55°C) using cDNAs as a template (0.2ng each; Human Multiple Tissue cDNA Panel I; CLONTECH) that were derived from human brain, heart, liver, kidney, lung, pancreas, placenta and skeletal muscle, respectively and forward primer (SEQ ID NO: 25; corresponding to a part of Ex.-2) and reverse primer (SEQ ID NO: 26; corresponding to a part of Ex.-3).

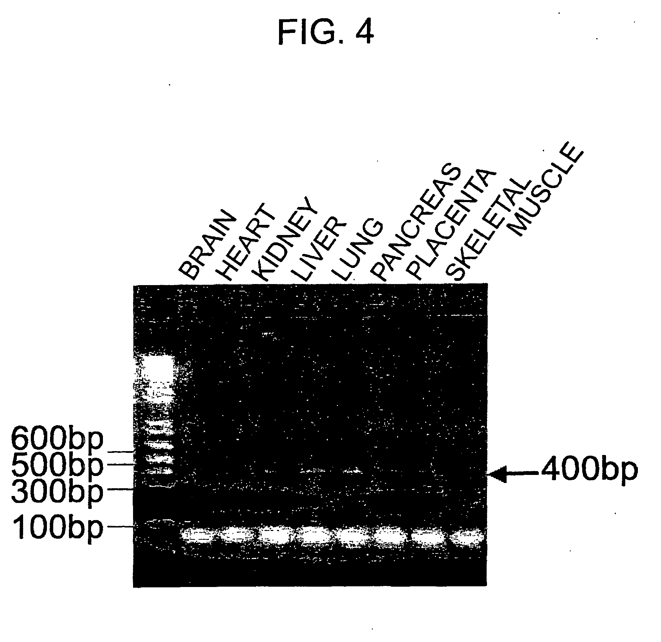

The results are shown in Fig. 4. As a result, an amplified DNA band of approximately 400bp that indicate the expression of MERMUC gene was detected in kidney, liver, lung, placenta and pancreas confirming significant expression of MERMUC gene in these organs.

### <4-3> Analysis by in situ hybridization

The expression of the HGF receptor c-Met gene in kidney tissue is localized in the epithelial cells of renal urinary tubule, and is involved in cell proliferation and lumen formation (Biochem. Biophys. Res. Commun., 1991, Vol. 174, p831-838; Cell, 1991, Vol. 67, p.901-908). Thus, the distribution of mouse MERMUC gene expression in kidney tissues was analyzed by *in situ* hybridization.

Tissue sections for RNA hybridization were prepared from kidney tissues of commercially available MRL-lpr/lpr mice (5 or 23 weeks old) and C57BL/6 mice (23 weeks old) according to a conventional method.

Anti-sense cRNA probe or sense cRNA probe synthesized using a fragment of about 500 bp encoding the N-terminus of the full-length mouse MERMUC (amino acid/ SEQ ID NO: 11; cDNA/SEQ ID NO: 12) as a template was used for the *in situ* hybridization.

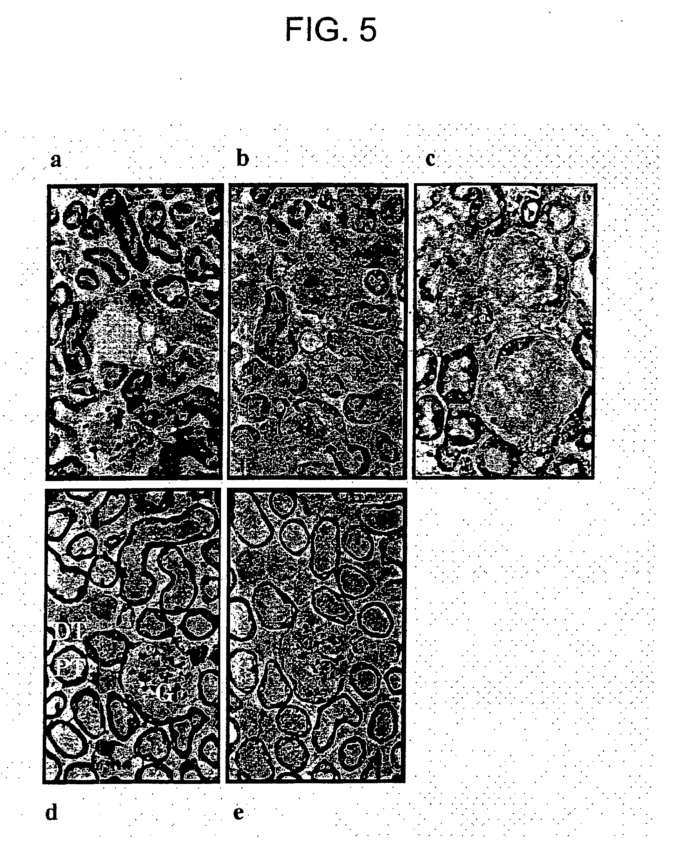

The results are shown in Fig. 5.

As a result, the expression of mouse MERMUC gene in kidney tissue was localized in the epithelial cells of the distal proximal tubules. The result agreed with the reported distribution of the HGF receptor c-Met expression in kidney cells.

### [Example 5] Identification of human HGF receptor c-Met intramolecular binding region to human MERMUC molecule

The present invention was performed using the reported yeast two-hybrid system (Proc. Natl. Acad. Sci. USA., 1991, Vol. 88, p.9578-9582) based on conventional method and the experimental protocols appended to the reagents.

PCR was performed using DNA (clone No. 3-309) corresponding to the 91 amino acids (amino acids 993-1083 of SEQ ID NO: 4) of the C-terminal region of the human HGF receptor c-Met β chain that was identified in the previous Example to have the binding ability to the cytoplasmic region of human MERMUC as a template. Seven DNA fragments shown below corresponding to the C-terminal region of the c-Met β chain having various lengths, wherein various length of N-terminal or C-terminal amino acids are deleted from said 91 amino acids were prepared via a conventional method by PCR:
A. DNA corresponding to a region (amino acids 1003-1083 of SEQ ID NO: 4) that lacks the 10 N-terminal amino acids of the 91 amino acids of the C-terminal human c-Met β chain;
B. DNA corresponding to a region (amino acids 1013-1083 of SEQ ID NO: 4) that lacks the 20 N-terminal amino acids of the 91 amino acids of the C-terminal human c-Met β chain;
C. DNA corresponding to a region (amino acids 1023-1083 of SEQ ID NO: 4) that lacks the 30 N-terminal amino acids of the 91 amino acids of the C-terminal human c-Met β chain;
D. DNA corresponding to a region (amino acids 993-1073 of SEQ ID NO: 4) that lacks the C-terminal 10 amino acids of the 91 amino acids of the C-terminal human c-Met β chain;
E. DNA corresponding to a region (amino acids 993-1063 of SEQ ID NO: 4) that lacks the 20 C-terminal amino acids of the 91 amino acids of the C-terminal human c-Met β chain;
F. DNA corresponding to a region (amino acids 993-1053 of SEQ ID NO: 4) that lacks the 30 C-terminal amino acids of the 91 amino acids of the C-terminal human c-Met β chain; and
G. DNA corresponding to a region (amino acids 993-1043 of SEQ ID NO: 4) that lacks the 40 C-terminal amino acids of the 91 amino acids of the C-terminal human c-Met β chain.

Furthermore, the forward and reverse primers used for the PCR were designed to contain an *Eco*RI site and *Sal*I site at their 5' end, respectively. Each of the DNA fragments prepared by PCR were inserted into the *Eco*RI/*Sal*I site of pADKT7 vector (a vector for a prey protein that contains a tryptophan biosynthesis gene as a marker; CLONTECH) in frame with GAL4-AD to prepare prey vectors wherein each of the 7 DNAs described above is inserted.

Each of the 7 prey vectors was cointroduced into yeast AH109 strain with the human MERMUC bait-2 vector prepared in Example 1.

The binding activity between the c-Met prey proteins derived from the 7 DNA fragments and the MERMUC bait protein was analyzed by the yeast two-hybrid system described in Example 1. Specifically, the binding activity was evaluated based on the growth of yeast transformed with the vector on 2 kinds of yeast minimum media, i.e., (1) SD/-Leu, -Trp, -His media and (2) SD/-Leu, -Trp, -Ade media.

As a result, the binding activity of each of the c-Met prey protein (DNA derived from A-G described above) was as follows:

| <prey DNA> | <binding activity with MERMUC bait protein> |
|---|---|
| A | + (bound) |
| B | + (bound) |
| C | - (no binding) |
| D | + (bound) |
| E | + (bound) |
| F | + (bound) |
| G | - (no binding). |

The 91 amino acids present at the C-terminus of the human c-Met β chain was revealed to bind to the C-terminal region of MERMUC (Example 1). The above results indicate that the deletion of 30 amino acid residues from the N-terminus or 40 amino acid residues from the C-terminus of the C-terminal 91 amino acids results in a loss of the binding ability to MERMUC.

The results revealed that the c-Met intramolecular region (binding site) exhibiting the binding ability to MERMUC is located within the common region of the above mentioned prey peptides. Namely, the binding site is located within the region consisting of about 40 amino acids on the N-terminal side from approximately the 30th amino acid from the C-terminus (from about amino acid 1013 to about amino acid 1052 of SEQ ID NO: 4.; corresponding to SEQ ID NO: 5).

This common region contains 2 tyrosine residues (amino acids 1349 and 1356 of SEQ ID NO: 2; amino acids 1042 and 1049 of SEQ ID NO.4) that are autophosphorylated upon activation of the HGF receptor c-Met via the binding with HGF.

### [Example 6] Identification of HGF receptor c-Met β chain binding region within human MERMUC molecule (Part 1)

The present examination was performed using the previously reported yeast two-hybrid system (references are described above) according to conventional method and the experimental protocols provided with the reagents.

Three bait vectors carrying DNAs corresponding to 3 MERMUC bait proteins described below were prepared by PCR based on the novel finding (Example 1) that the C-terminal region of human MERMUC cytoplasmi.c region binds to the C-terminal region of human c-Met:
A. The entire human MERMUC cytoplasmic region, i.e., DNA corresponding to the C-terminal 260 amino acids within the MERMUC cytoplasmic region (dubbed [C260]; amino acids 244-503 of SEQ ID NO: 10) ;
B. DNA corresponding to the region wherein 53 amino acids are deleted from the C-terminus of C260 (dubbed [C260 Δ53]; amino acids 244-450 of SEQ ID NO: 10); and
C. DNA corresponding to the C-terminal 53 amino acids of C260 (dubbed [C53]; amino acids 451-503 of SEQ ID NO: 10).

PCR was performed using cDNA encoding the full-length human MERMUC (SEQ ID NO: 10) as a template and primer set as follows:
primer for C260: DNAs described in SEQ ID NO: 27 and SEQ ID NO: 28, respectively;
primer for C260ΔC53: DNAs described in SEQ ID NO: 29 and SEQ ID NO: 30, respectively; and
primer for C53: DNAs described in SEQ ID NO: 31 and SEQ ID NO: 32, respectively.

Each of the obtained PCR product DNAs was inserted into plasmid pGBKT7 to produce a bait vector.

Yeast AH109 strain was cotransfected with each of the bait vectors and the prey vector carrying human HGF receptor c-Met (DNA corresponding to the C-terminal 91 amino acids of the human c-Met; clone No. 3-309) identified in Example 1.

The binding ability of MERMUC bait protein derived from each of the bait vectors to the HGF receptor c-Met β chain was analyzed by the yeast two-hybrid system described above.

As a result, both C260 (containing almost the entire MERMUC cytoplasmic region) and C53 (the C-terminal 53 amino acids of the MERMUC cytoplasmic region) possessed the binding activity to the HGF receptor c-MET β chain. On the other hand, C260ΔC53, wherein the C-terminal 53 amino acids of C260 were deleted, completely lost the binding activity to the HGF receptor c-Met β chain.

These results revealed that the C-terminal portion of MERMUC, particularly, the region comprising about 53 amino acids from the C-terminus (amino acids 451-503 of SEQ ID NO: 10) contribute to the binding of MERMUC to the HGF receptor c-Met.

Similar as above, the presence of binding between mouse MERMUC (amino acid/SEQ ID NO: 12; cDNA/SEQ ID NO:11) and mouse HGF receptor, as well as their binding sites were analyzed using the yeast two hybrid system.

cDNAs encoding each of the mouse MERMUC and mouse HGF receptor were prepared by cloning through the RT-PCR method from mouse kidney derived RNA.

As a result, the mouse MERMUC was revealed to bind at a region comprising about 53 amino acids of the C-terminus to the C-terminal region of the mouse HGF receptor β chain similar to the binding between human MERMUC and human c-Met. This observation explains the high amino acid sequence homology between human and mouse in the region comprising the C-terminal 53 amino acids of MERMUC. It also indicates that the C-terminal region is important for the binding to the HGF receptor regardless of species.

### [Example 7] Identification of HGF receptor c-Met β chain binding region within human MERMUC molecule (part 2)

The HGF receptor c-Met binding region revealed in the previous Example that exists in the human MERMUC C-terminal region (the region comprising the C-terminal 53 amino acids) was further analyzed in more detail. The analysis was performed by the yeast two-hybrid system similar to the previous Example.

Bait proteins corresponded to a part of the human MERMUC C-terminal region (randomly selected 10 continuous amino acids) as below:
A. amino acids 429-438 of SEQ ID NO.10 (overlaps 5 amino acids with B) ;
B. amino acids 434-443 of SEQ ID NO.10 (overlaps 5 amino acids with A and C, respectively);
C. amino acids 439-448 of SEQ ID NO.10 (overlaps 5 amino acids with B and D, respectively);
D. amino acids 444-453 of SEQ ID NO.10 (overlaps 5 amino acids with C and E, respectively);
E. amino acids 449-458 of SEQ ID NO.10 (overlaps 5 amino acids with D and F, respectively);
F. amino acids 454-463 of SEQ ID NO.10 (overlaps 5 amino acids with E and G, respectively);
G. amino acids 459-468 of SEQ ID NO.10 (overlaps 5 amino acids with F and H, respectively);
H. amino acids 464-473 of SEQ ID NO.10 (overlaps 5 amino acids with G and I, respectively);
I. amino acids 469-478 of SEQ ID NO.10 (overlaps 5 amino acids with H and J, respectively);
J. amino acids 474-483 of SEQ ID NO.10 (overlaps 5 amino acids with I and K, respectively);
K. amino acids 479-488 of SEQ ID NO.10 (overlaps 5 amino acids with J and L, respectively);
L. amino acids 484-493 of SEQ ID NO.10 (overlaps 5 amino acids with K and M, respectively);
M. amino acids 489-498 of SEQ ID NO.10 (overlaps 5 amino acids with L and N, respectively); and
N. amino acids 494-503 of SEQ ID NO.10 (overlaps 5 amino acids with M).

For each of the bait proteins described above (10 amino acids), sense strand DNA (an *Eco*RI site added at the 5' end) encoding the protein and antisense strand DNA (a *Sal*I added at the 5' end) were synthesized, annealed, and then inserted into the *Eco*RI/*Sal*I site of plasmid pGBKT7 to construct a bait vector.

Each of the bait vectors was cointroduced into yeast AH109 strain with human HGF receptor c-Met prey vector (comprising DNA encoding the C-terminal 91 amino acids of the c-Met; Example 1). The binding ability of each of the bait proteins (randomly selected 10 amino acids of the human MERMUC C-terminal region) to the C-terminal region of human c-Met β chain was analyzed by the yeast two-hybrid system similar to the aforementioned Example.

As a result, only those bait proteins relating to the C-terminal 53 amino acids that were identified to be important for the binding activity to the c-Met β chain in the previous Example possessed the binding activity to the C-terminal region of human c-Met β chain.

This test result further indicates that the C-terminal 53 amino acid region of human MERMUC has an extremely important role in the binding with the human HGF receptor c-Met.

Furthermore, the region conserved among human, mouse and rat within the amino acid sequence of the C-terminal region contains a leucine repeat structure wherein leucine appears at every 4 amino acid residues. This leucine repeat structure is suggested to have an important role for the binding of MERMUC and c-Met.

### [Example 8] Construction of assay system that enables evaluation of the binding of MERMUC and HGF receptor c-Met

β-Galactosidase (β-gal) is an enzyme widely distributed from bacteria to higher eukaryotes that degrades/synthesizes lactose. The biological activity of β-gal is exerted via a homotetramer wherein 4 monomer molecules of β-gal primary structure are associated together.

The β-gal of *E*. *coli* (lacZ gene) has been particularly studied well and applied as an experimental research tool.

Three mutants, β-galΔα, β-galΔµ and β-galΔω, have been reported in a certain *E. coli* strain (Δ indicates deletion; Proc. Natl. Acad. Sci. USA., 1996, Vol. 93, p.12423-12427; Proc. Natl. Acad. Sci. USA., 1997, Vol. 94, p.8405-8410).

β-galΔα lacks a part of the N-terminal sequence (amino acids 50-602 of GenBank Accession Nos. P00722) which is the active site of wild-type β-gal. β-galΔµ lacks a part of the sequence in the middle of wild-type β-gal (amino acids 50-602 of GenBank Accession Nos. P00722). β-galΔω lacks a part of the C-terminal sequence (amino acids 790-1024 of GenBank Accession Nos. P00722) which is necessary to form a homotetramer in wild-type β-gal monomers.

Therefore, β-galΔα or β-galΔω alone naturally do not exhibit enzyme activity. Furthermore, without binding to each other (including encounter) , even in the coexistence of β-galΔα and β-galΔω no β-gal activity is expressed.

However, when two proteins, [X] and [Y] , that have affinity to each other are fused with β-galΔα and β-galΔω, respectively, and the produced [X-β-galΔα] and [Y-β-galΔω] coexist, β-gal activity due to the encounter of these two fusion proteins by the affinity between X and Y appears (Proc. Natl. Acad. Sci. USA., 1997, Vol. 94, p. 8405-8410).

Applying this theory, the binding ability of MERMUC and HGF receptor c-Met was evaluated by establishing an assay system that enables evaluation of the binding between the two molecules.

Specifically, the entire human HGF receptor c-Met or the C-terminal region thereof and the entire human MERMUC or the C-terminal region thereof were used as X and Y of X-β-galΔα and Y-β-galΔω, respectively. The binding between MERMUC and c-Met is evaluated by coexpressing these two fusion proteins in animal cells, such as CHO cells, via genetic recombination techniques and using the β-galactosidase activity expressed in the recombinant cells as an indicator.

In order to construct the present evaluation system, expression vectors expressing each fusion polypeptide (fusion protein) described below were prepared. The method to prepare each of the vectors will be explained below:
A. human MERMUC (full-length; full)/ β-galΔω;
B. human MERMUC (full-length; full)/ β-galΔα;
C. human MERMUC (53 amino acid deletion at the C-terminus; ΔC53) / β-galΔω;
D. human MERMUC (53 amino acid deletion at the C-terminus; ΔC53) / β-galΔα; and
E. human c-Met (C-terminal 91 amino acids alone; C91) / β-galΔα.

### <8-1> Isolation of β-galΔα gene

β-galΔα lacks the N-terminal amino acids (amino acids 12-42 of GenBank Accession Nos. P00722) of the wild-type β-gal and *E. coli* DH5α strain is known to produce this mutant (Gene, 1992, Vol. 122, p. 231-232).

Thus, DNA encoding the target β-galΔα was cloned using DNA of *E*.*coli* DH5α strain as a template, and designing PCR forward primer (SEQ ID NO: 33; a *Xho*I site added at the 5' side) and reverse primer (SEQ ID NO: 34; a *Sal*I site added at the 5' side) were designed based on the nucleotide sequences corresponding to the regions comprising the N-terminal methionine and the C-terminal stop codon of the wild-type β-gal.

The β-galΔα gene fragment amplified by PCR was digested with restriction enzymes *Xho*I and *Sal*I, inserted into the *Xho*I and *Sal*I site of pBluescript KS, and then was introduced into *E*.*coli*. The plasmid containing β-galΔα used in the following experiments was prepared from this transformant.

The amino acid sequence of β-galΔα is shown in SEQ ID NO: 16.

### <8-2> Isolation of β-galΔω gene

β-GalΔω lacks a part of the C-terminal sequence (amino acids 790-1024 of GenBank Accession Nos. P00722) that is required for the formation of homotetramer in the wild-type β-gal monomer molecules (Proc. Natl. Sci. USA., 1996, Vol. 93, p. 12423-12427).

Thus, DNA encoding β-galΔω was prepared by PCR amplification of the region corresponding to the sequence from N-terminal methionine to the amino acid 789 using wild-type β-gal gene from *E. coli* DH5 strain as a template.

The following primers were used for the PCR:
1. forward primer: SEQ ID NO: 35 (a *Xho*I site added at the 5' side of ATG); and
2. reverse primer: SEQ ID NO: 36 (a *Sal*I site added at the 5' side of CGG).

The PCR product was digested with restriction enzymes *Xho*I and *Sal*I to prepare a β-GalΔω gene fragment. The fragment was inserted into the *Xho*I and *Sal*I sites of pBluescript KS and then introduced into *E*.*coli*. A plasmid containing β-galΔω gene used for following experiments was prepared from this transformant.

### <8-3> Preparation of DNAs encoding MERMUC (full) and MERMUC (ΔC53) used for fusion with β-gal mutant

In order to fuse the DNA encoding β-gal mutant to the DNA encoding human MERMUC (full) or human MERMUC (ΔC53) in-frame, both the stop codon of human MERMUC (full) DNA and the C-terminal codon of human MERMUC (ΔC53) were converted to a *Xho*I site by PCR using primers described below. DNA encoding the full-length human MERMUC was used as a template:

### <human MERMUC (full)> (amplification from N-terminal Met to amino acid 503)

forward primer: SEQ ID NO: 37 (a *Hind*III site added at the 5' side of ATG), and
reverse primer: SEQ ID NO: 38 (a *Xho*I site added at the 5' side of GCC); and

### <human MERMUC (ΔC53) > (amplification from N-terminal Met to amino acid 450)

forward primer: SEQ ID NO 39 (a *Hind*III site added at the 5' side of ATG), and
reverse primer: SEQ ID NO: 40 (a *Xho*I site added at the 5' side of TGC).

Each of the PCR products was digested with restriction enzymes *Hind*III and *Xho*I, inserted into the *Hind*III and *Xho*I site of pBluescript KS, and then introduced into *E. coli.* Each of the plasmids carrying the DNA encoding any one of the fusion proteins A to D described above was prepared from the transformed *E. coli* strain.

### <8-4> Preparation of DNA encoding human c-Met (C91)

In order to fuse DNA encoding β-galΔα to DNA encoding human c-Met (C91) in-frame, a DNA corresponding to the C-terminal 91 amino acids of the human HGF receptor c-Met (amino acids 993-1083 of SEQ ID NO: 4) was prepared by PCR using primer set described below:
forward primer: SEQ ID NO: 41 (ATG and *Hind*III site added at the 5' side of AGA); and
reverse primer: SEQ ID NO: 42 (*Xho*I site added at the 5' side of TCA).

Each of the obtained PCR product was digested with restriction enzymes *Hind*III and *Xho*I, inserted into the *Hind*III and *Xho*I site of pBluescript KS, and then introduced into *E. coli.* Plasmids carrying DNA encoding the fusion proteins described above were prepared from the transformed *E. coli* strain.

### <8-5> Preparation of expression vectors for fusion proteins (A, B, C, D and E)

### A) Preparation of human MERMUC (full)/β-galΔω expression vector

Each of the plasmids prepared above was digested with restriction enzymes *Hind*III and *Xho*I or *Xho*I and *Sal*I to cut out DNA fragments encoding human MERMUC (full) and β-galΔω, respectively. The obtained two DNA fragments were ligated at the *Xho*I site, and then inserted into the *Hind*III/*Sal*I site of pcDNA3 vector to construct the objective human MERMUC (full)/β-galΔω expression vector.

### B) Preparation of human MERMUC (full)/β-galΔα expression vector

Each of the plasmids produced above was digested with restriction enzymes *Hind*III and *Xho*I or *Xho*I and *Sal*I to cut out DNA fragments encoding human MERMUC (full) and β-galΔα, respectively. The obtained two DNA fragments were ligated at the *Xho*I site and then inserted into the *Hind*III/*Sal*I site of pcDNA3 vector to construct the objective human MERMUC (full)/β-galΔα expression vector.

### C) Preparation of human MERMUC (ΔC53)/β-galΔω expression vector

Each of the plasmids produced above was digested with restriction enzymes *Hind*III and *Xho*I or *Xho*I and *Sal*I to cut out DNA fragments encoding human MERMUC (ΔC53) and β-galΔω, respectively. The obtained two DNA fragments were ligated at the *Xho*I site and then inserted into the HindIII/SalI site of pcDNA3 vector to construct the objective human MERMUC (ΔC53)/β-galΔω expression vector.

### D) Preparation of human MERMUC (ΔC53)/β-galΔα expression vector

Each of the plasmids produced above was digested with restriction enzymes *Hind*III and *Xho*I or *Xho*I and *Sal*I to cut out DNA fragments encoding human MERMUC (ΔC53) and β-galΔα, respectively. The obtained two DNA fragments were ligated at the *Xho*I site and then inserted into the HindIII/SalI site of pcDNA3 vector to construct the objective human MERMUC (ΔC53)/β-galΔα expression vector.

### E) Preparation of human c-Met (C91)/β-galΔα expression vector

Each of the plasmids produced above was digested with restriction enzymes *Hind*III and *Xho*I or XhoI and *Sal*I to cut out DNA fragments encoding human c-Met (C91) and β-galΔα, respectively. The obtained two DNA fragments were ligated at the *Xho*I site and then inserted into the HindIII/SalI site of pcDNA3 vector to construct the objective human c-Met (C91)/β-galΔα expression vector.

### <8-6> Assay to evaluate the binding of MERMUC with HGF receptor c-Met

CHO cells were cotransfected with a mixture containing the above-described 5 fusion protein expression vectors in combination described below at equivalent amounts using commercially available transfection reagent (GeneJammer Transfection Reagent; STRATAGENE) according to the experimental protocol:
I) A + E, i.e., MERMUC (full)/β-galΔω + c-Met (C91)/β-galΔα;
II) A + B, i.e., MERMUC (full)/β-galΔω + MERMUC (full))/β-galΔα;
III) C + E, i.e., MERMUC (ΔC53)/β-galΔω + c-Met (C91)/β-galΔα; and
IV) C + D, i. e. , MERMUC (ΔC53)/β-galΔω + MERMUC (ΔC53) /β-) /β-galΔα.

After incubating the cells for 48 hr at 37°C, β-Galactosidase activity in each cell was measured according to the experimental protocol using GAL-SCREEN SYSTEM (Applied Biosystems).

The results are shown in Fig. 6 and Fig. 7.

As a result, the combinations of (I), (II) and (III) showed a significantly higher β-galactosidase activity compared with cells transformed with the control (mock-control) vector alone. On the other hand, the combination of (III) did not show any activity.

These results confirm the following findings that were clarified by the Examples described above:
1. MERMUC binds to HGF receptor c-Met;
2. the c-Met binding region of MERMUC exists in the region containing about 53 amino acids of the C-terminal cytoplasmic region of MERMUC; and
3. the MERMUC binding region of c-Met exists in the region containing about 91 amino acids of the C-terminal cytoplasmic region of c-Met β chain.

Furthermore, the test result of the combination (IV) described above indicated that the site contributing to the binding among MERMUC molecules exists much near to the N-terminus than the C-terminal 53 amino acids that is the c-Met binding region of MERMUC.

Moreover, the above results indicate that substrates inhibiting the binding of MERMUC and c-Met, and substances that inhibit the intermolecular binding of MERMUC can be identified by the assay system constructed in the present test. Similarly, the use of the assay system was indicated to enable measurement and evaluation of the existence or degree of activity of a substance to inhibit the binding between MERMUC and c-Met, or to suppress or inhibit the intermolecular binding of MERMUC.

### [Example 9] Biochemical analysis of binding of HGF receptor c-Met and MERMUC

The C-terminal region of the HGF receptor c-Met β-chain where MERMUC binds is known as the multifunctional docking site that contains autophosphorylated tyrosine residues. This region is extremely important for transmission of signals into the cell that are generated by the binding of HGF to HGF receptor c-Met. Therefore, HGF signaling was predicted to be physiologically modulated by the binding of MERMUC molecules to this region.

Thus, to examine the physiological importance of the binding of MERMUC and HGF receptor c-Met, following experiments were performed using the MERMUC expressing recombinant cells prepared in the above-described Example in which cells the gene expression is regulated by Dox.

### <9-1> Regulation of MAP kinase activation via HGF receptor

According to previous studies, the activation of MAP kinase caused by HGF signal transduction is revealed to be accomplished through a stepwise process as below (Cell. , 1994, Vol. 77, p. 261-271; Experimental Medicine, 1997, Vol. 15, p. 1033-1039; Nature, 1998, Vol. 391, p. 285-288):
(1) homodimerizaion of HGF receptor c-Met upon binding of HGF to the HGF receptor c-Met;
(2) autophosphorylation of tyrosine residues in the C-terminal region of C-Met β chain;
(3) recruitment of growth factor receptor-bound protein-2 (Grb2) to the phosphorylation site;
(4) Ras activation; and
(5) mitogen-activated protein (MAP) kinase activation.

Therefore, the existence of physiological effect of MERMUC expression on intracellular MAP kinase activation induced by HGF stimulation was analyzed.

Specifically, MERMUC non-producing system and MERMUC producing system were prepared by culturing the above-mentioned MERMUC/293 cell strain in the presence or absence of Dox, respectively. The degree of MAP kinase activation elicited immediately after the addition of HGF into each of the system was compared.

Each of the cells (MERMUC non-producing system) wherein MERMUC expression is suppressed by culturing in the presence of Dox and cells (MERMUC producing system) wherein MERMUC expression is induced by culturing in the absence of Dox was stimulated with human recombinant HGF (#375228; CALBIOCHEM). Immediately thereafter, cell lysate was prepared for the evaluation of MAP kinase activity.

The cell lysate was separated by SDS polyacrylamide gel electrophoresis (SDS-PAGE), and then subjected to western blotting according to conventional method using anti-MAP kinase polyclonal antibody (p44/42 MAP kinase antibody, #9102; Cell Signaling Technology) and anti-phosphorylated MAP kinase polyclonal antibody (Phospho-p44/42 MAP kinase (Thr202/Thr204) antibody, #9101L; Cell Signaling Technology).

The amount of MAP kinase protein in both of the samples was confirmed to be the same using the former antibody described above. Then, the signal intensities of phosphorylated MAP kinase that are detected with the latter antibody described above were compared.

The results are shown in Fig. 8.

As a result, the MAP kinase activity was significantly reduced in MERMUC producing cells compared with MERMUC non-producing cells.

Furthermore, MAP kinase activity gradually decreased in response to the increase of MERMUC production due to the lowering of the Dox concentration.

These results revealed that HGF signal transduction is suppressed depending on the amount of MERMUC production and that MERMUC down-regulates HGF signal transduction via the binding to the HGF receptor.

### <9-2> Regulation of autophosphorylation in cytoplasmic region of HGF receptor c-Met

The decrease in MERMUC production-dependent MAP kinase activity revealed in the above Example was suggested to be caused by a decrease in autophosphorylation of the cytoplasmic region of the HGF receptor c-Met during the HGF signal transduction process.

Therefore, the effect of MERMUC production on HGF signal transduction was examined by analyzing the level of autophosphorylation of HGF receptor that is induced by adding HGF to MERMUC overproducing cells and MERMUC non-producing cells.

Cell lysate was prepared from MERMUC overexpressing cells and MERMUC non-producing cells that were obtained by culturing MERMUC/293 cells in the absence or presence of Dox, respectively, as described in the previous Example.

Immunoprecipitation treatment was performed by adding anti-HGF receptor polyclonal antibody to each of the cell lysate. Then, after separating the precipitated proteins by SDS-PAGE, Western blotting was performed using anti-phosphorylated tyrosine monoclonal antibody (Phospho-Tyrosine monoclonal antibody (P-Tyr-100), #9411; Cell Signaling Technology).

The amount of HGF receptor protein in both of the cell lysates was confirmed to be identical using the former antibody described above. Then, autophosphorylation of tyrosine residues in the cytoplasmic region of the HGF receptor c-Met β-chain was analyzed by comparing the signal intensities detected with the latter antibody described above.

The results are shown in Fig. 9.

As a result, no difference in tyrosine residue phosphorylation could be observed between the two cells. Therefore, the results indicate that MERMUC production and the binding of MERMUC to the HGF receptor c-Met donot affect the autophosphorylation of tyrosine residues in the cytoplasmic region of the HGF receptor that is induced by HGF stimulation.

### <9-3> Regulation of Grb2 binding to phosphorylated tyrosine in cytoplasmic region of HGF receptor

The influence of MERMUC production (namely, the binding of MERMUC and c-Met) on a step of HGF signal transduction process, i.e., the binding of Grb2 molecule to phosphorylated tyrosine in the cytoplasmic region of the HGF receptor c-Met β chain was examined.

The amount of Grb2 bound to phosphorylated tyrosine was evaluated as the amount of coprecipitated HGF receptor protein in the immunoprecipitation of Grb2 with anti-Grb2 antibody.

Cell lysate was prepared from both the MERMUC over expressing cells and MERMUC non-producing cells that were obtained by incubating MERMUC/293 cells in the absence or presence of Dox, respectively, as described in the previous Example.

Grb2 protein was immunoprecipitated according to conventional method by adding anti-Grb2 polyclonal antibody (GRB2 (C-23) : sc-255; Santa Cruz Biotechnology) into each of the lysate. Next, after separating HGF receptor protein that coprecipitated with Grb2 protein by SDS-PAGE, Western blotting was performed using anti-HGF receptor polyclonal antibody.

The amount of HGF receptor protein coprecipitated with Grb2 was evaluated based on the detected signal intensity of HGF receptor.

Furthermore, as a control experiment, Gab1 that is also known to be recruited to the phosphorylated tyrosine site in the cytoplasmic region of the HGF receptor c-Met, similar to Grb2, was analyzed in the same way as described above. Anti-Gab1 polyclonal antibody (Gab1 (H-198) : sc-9049; Santa Cruz Biotechnology) was used for the control experiment.

The results are shown in Fig. 10.

As a result, the amount of HGF receptor c-Met that coprecipitated with Grb2 was significantly decreased in MERMUC producing cells compared with MERMUC non-producing cells. On the other hand, no significant difference could be observed in the amount of c-Met that coprecipitates with Gab1 between MERMUC non-producing cells and MERMUC producing cells.

These results indicate that the binding of Grb2 to phosphorylated tyrosine in the cytoplasmic region of the HGF receptor c-Met β chain is inhibited by MERMUC production.

### <9-4> Regulation of MMP-1 and MMP-9 gene expressions

Expression of matrixmetalloprotease-1 (MMP-1) and matrixmetalloprotease-9 (MMP-9) is known to be induced upon HGF stimulation in cultured cell strains and kidney tissues (Kidney Int. , 2000, Vol. 58, p.2028-2043; Carcinogenesis, 2000, Vol. 21, p. 1079-1085).

Therefore, human MERMUC expressing recombinant cells (MERMUC/293 cells; as described in the previous Example) were produced from HEK293 cells established from human kidney derived cells as a host, and the influence of MERMUC production on MMP-1 and MMP-9 expression induced through HGF stimulation in the cells was analyzed by RT-PCR.

MERMUC overexpressing cells and MERMUC non-producing cells were prepared by incubating MERMUC/293 cells in the absence or presence of Dox, respectively, similarly as described in the previous Example.

Human HGF was added to each of the cells and RNAs were prepared from each of the cells immediately after HGF addition (time 0) and 3, 6 and 24 hr after HGF addition according to conventional method. Each of the RNA sample was used as a template and RT-PCR was performed according to conventional method using a primer set described below to determine the mRNA expression level of MMP-1, MMP-9 and GADH (control) in each of the samples:
primers for MMP-1: SEQ ID NO: 43 and SEQ ID NO: 44;
primers for MMP-9: SEQ ID NO: 45 and SEQ ID NO: 46; and
primers for GAPDH: SEQ ID NO: 47 and SEQ ID NO: 47.

The results are shown in Fig. 11.

Increase over time in MMP-1 mRNA expression was observed in MERMUC non-producing cells. However, the expression induction was significantly suppressed in MERMUC producing cells.

Moreover, a peak was observed in the expression induction of MMP-9 mRNA 3 hr after HGF stimulation in MERMUC non-producing cells. However, almost no expression induction of MMP-9 mRNA was observed in MERMUC producing cells.

These results indicate that the expression of both MMP-1 and MMP-9 genes induced by HGF stimulation is decreased due to the overexpression of MERMUC protein, and that the decrease in the expression of both of the genes result from the decreased MAP kinase activation due to the down-regulation of HGF signaling by MERMUC protein.

The findings described below were identified in relation to the MERMUC function and the down-regulation mechanism of HGF signaling from the test results mentioned above:
1) MERMUC binds in the vicinity of autophosphorylated tyrosine in the cytoplasmic region of the HGF receptor c-Met β chain, and thereby, inhibits the binding of Grb2 protein that is recruited to this site;
2) as a result, MAP kinase activity that should be elicited through the signal transduction due to the binding of HGF/HGF receptor c-Met is decreased;
3) as a result of the decrease in MAP kinase activity, biological reactions thereafter (for example, the expression of MMP-1, MMP-9 and such) are inhibited; and
4) finally, HGF/HGF receptor c-Met signaling is down-regulated.

### [Example 10] Production and analysis of MERMUC overexpressing transgenic mice

### <10-1> Production of MERMUC-Tg mouse

The proliferation of primary cultured cells prepared from liver is reported to be enhanced by HGF stimulation (Proc. Natl. Acad. Sci. USA., 1986, Vol. 83, p. 6489-6493). Utilizing this phenomenon, the effect of the increase in MERMUC protein production in liver cells on HGF stimulation-dependent cell proliferation was analyzed using liver cells of MERMUC overexpressing transgenic (Tg) mouse (hereafter, simply referred to as MERMUC-Tg mouse, MERMUC-Tg or Tg).

MERMUC-Tg mouse was produced according to conventional method ("Manipulating the mouse embryo, a laboratory manual", 1989, Chap. 2-4, p.77-203, Kindai Shuppan; "The latest Laboratory Manual for Animal Cells", Chap 7, p361-408, 1990; LIC Press).

cDNA (SEQ ID NO: 11) encoding the full-length mouse MERMUC was inserted into expression vector pCAGGS (Gene, 1991, Vol. 108, p193-200) carrying the avian β-actin promoter using DNA blunt end kit (TAKARA) to obtain a mouse MERMUC expression vector. The vector was linearized by digestion with restriction enzyme and was used as the mouse MERMUC gene for Tg production.

A white ICR mouse (female; Charles River Japan) and a vasoligated white ICR mouse (male; Charles River Japan) were crossed to obtain a female ICR mouse possessing a plug (or a vaginal gland) as the surrogate mouse. Furthermore, mouse for harvesting fertilized eggs to introduce the mouse MERMUC expression vector was produced by crossing an overovulation ICR mouse (female; Charles River Japan) prepared by administration of PMS (5 unit; Sigma) and hCG (5 unit; Sigma) with an ICR mouse (male; Charles River Japan). After mating, the oviduct was extirpated from the ICR mouse (female) , treated with hyaluronidase to obtain only fertilized eggs, and the eggs were preserved in culture media.

The introduction of mouse MERMUC gene into a fertilized egg was performed according to conventional method using a manipulator under a microscope. The fertilized egg was fixed with a retention needle, and a solution containing the mouse MERMUC gene diluted with Tris-EDTA buffer was injected into the male pronucleus of the fertilized egg using a DNA injection needle at 37°C.

After gene transfer, fertilized eggs keeping normal conditions were selected as the mouse MERMUC gene transferred eggs and inserted into the fimbriae of uterine tube in the ovary of the surrogate mouse (white ICR mouse). Mouse off springs (chimera mice) born from the surrogate mother were obtained as MERMUC-Tg mice.

Northern blot analysis was performed according to conventional method using mRNA extracted from each of various organs (liver, kidney, heart, lung, brain, etc.) of the produced MERMUC-Tg and normal mice (non-Tg mice; normal mice born from the above-mentioned surrogate mother) to analyze the mRNA expression level of mouse MERMUC.

As a result, mRNA expression of mouse MERMUC in Tg mice was significantly higher in all of the organs compared to the non-Tg mice. Thus, the produced MERMUC-Tg mice were confirmed to be mouse MERMUC overexpressing mice.

### <10-2> Analysis of the effect of increase in MERMUC production on HGF stimulation-dependent liver cell proliferation

Primary cell culture of hepatocytes from liver cells obtained from the liver of each of the MERMUC-Tg mice (8 weeks old, 4 mice) and non-Tg mice (8 weeks old, 4 mice) was prepared by collagenase treatment according to the method of Berry and Friend, and Seglen *et al*. (Berry & Friend , J. Cell Biol., 1969, Vol. 43, p. 506-520; Seglen *et al*., Methods in Cell Biol., 1976, Vol. 13, p. 29-83).

Each of the obtained hepatocytes (2 x 10³ cells) was cultured in serum-free media for 15 hr, HGF (0, 1 or 3 ng/ml) was added to the media to induce HGF-dependent cell proliferation, and was further cultured. Similarly, cultivation was performed by adding epidermal growth factor (EGF; 0, 1, or 10 ng/ml) in place of HGF to the culture as a control.

After 24 hr from HGF addition, cell numbers were counted using Cell Count Reagent SF (Nacalai tesque) according to the experimental protocol provided with the reagent to calculate the relative cell proliferation rate after HGF (or EGF) addition. Tests were conducted in triplicate.

The results are shown in Fig 12.

There was no difference between MERMUC-Tg and non-Tg in the control EGF stimulated hepatocyte proliferation, and both showed dosage-dependent cell proliferation (Fig. 12-B).

On the other hand, the proliferation due to HGF stimulation of hepatocytes derived from MERMUC-Tg mice showed a significant decrease in the proliferation (reactivity) compared with the cells from non-Tg mice (Fig. 12-A).

### <10-3> Analysis of the effect of increase in MERMUC production on activation of intracellular MAP kinase

The changes in intracellular MAP kinase activation after HGF stimulation in hepatocytes derived from each of the MERMUC-Tg mice and non-Tg mice were analyzed similarly as in Example 9.

The results are shown in Fig 13.

The results showed a significant decrease in MAP kinase activation in MERMUC-Tg derived cells.

These results indicate that the proliferation of various cells, including liver cells, is suppressed through the suppression of functional expression of HGF/HGF receptor by the binding of MERMUC to the HGF receptor. They also indicate down-regulation of cell proliferation, and regeneration and neogenesis of tissues, organs, blood vessels and such via the suppression of functional expression of HGF/HGF receptor by MERMUC.

### [Example 11] Analysis of increase in MERMUC binding affinity with HGF receptor due to MERMUC self-association

The yeast two-hybrid system and the immunoprecipitation method described above showed that MERMUC forms multimers by self-association.

In the present experiment, the relationship between MERMUC self-association and the binding between MERMUC and HGF receptor was analyzed by establishing an evaluation system that reproduces the stimulation-dependent MERMUC self-association.

### <11-1> Establishment of evaluation system reproducing stimulation-dependent MERMUC self-association

The following three expression vectors were constructed by the method described below:
(1) plasmid (hereafter, referred to as "mFas-MERMUC") expressing a fusion chimeric protein consisting of a part of mouse Fas (CD95) (consisting of the extracellular region and transmembrane region) and the cytoplasmic region of human MERMUC;
(2) plasmid (hereafter, referred to as "'mFas-MERMUC-β-galΔω"). expressing a fusion chimeric protein consisting of a part of mouse Fas (consisting of the extracellular region and transmembrane region) , the cytoplasmic region of human MERMUC and β-galΔω described above; and
(3) plasmid (hereafter, referred to as "mFas-MERMUC-β-galΔα") expressing a fusion chimeric protein consisting of a part of mouse Fas (consisting of the extracellular region and transmembrane region) , the cytoplasmic region of human MERMUC and β-galΔα described above.

The mouse Fas described above was as reported (GenBank Accession Nos. P 25446 and NP 032013; J. Immunol., 1992, Vol. 148, No. 4, p. 1274-1279; etc.).

cDNA encoding the part of mouse Fas (the extracellular region and transmembrane region) was prepared by PCR using mouse spleen cDNA library (CLONTECH) as a template and a primer set as follows:
forward primer: SEQ ID NO: 49; and
reverse primer: SEQ ID NO: 50.

cDNA encoding the cytoplasmic region of human MERMUC was prepared by PCR using full-length human MERMUC cDNA (SEQ ID NO: 9) described above as a template and a primer set as follows:
forward primer: SEQ ID NO: 51; and
reverse primer: SEQ ID NO: 52.

Each of the obtained cDNA was subcloned into pTargeT vector (Promega) and then both fragments were obtained by redigestion with restriction enzymes *Xba*I and NotI. The digested fragments were ligated at the *Xba*I site and inserted into pcDNA3 vector to obtain mFas-MERMUC expression plasmid.

Expression plasmids for mFas-MERMUC-β-galΔω and mFas-MERMUC-β-galΔα were constructed as follows.

Both of the above-prepared human MERMUC (full-length) /β-galΔα and human MERMUC (full-length)/β-galΔω) were digested with *Apa*I to prepare cDNAs encoding MERMUC (cytoplasmic region)-β-galΔα and MERMUC (cytoplasmic region)-β-galΔω. Expression plasmids for mFas-MERMUC-β-galΔω and mFas-MERMUC-β-galΔα were prepared by inserting the obtained respective cDNAs downstream of the *Apa*I site of the previously constructed mFas-MERMUC.

Next, expression plasmids for mFas-MERMUC-β-galΔω and mFas-MERMUC-β-galΔα were added to HEK293 cells and cultured at 37°C for cotransfection using GeneJammer Transfection Reagent (STRATAGENE) according to the attached protocol. Recombinant cells coexpressing mFas-MERMUC-β-galΔω and mFas-MERMUC-β-galΔα on the cell membrane were obtained.

Both the bound and unbound states of the extracellular Fas region can be created by treating this recombinant cell with anti-Fas antibody that either possess or lacks the activity to induce the association of Fas molecules. Furthermore, the association (binding) of MERMUC protein portion within the cells induced by the association of the Fas region can be evaluated by measuring β-galactosidase activity.

Forty eight hr after the transfection of both of the expression plasmids into the recombinant cells, the cells were treated with anti-Fas antibody and negative control antibody (1 or 10 µg/ml each) for 3 hr, respectively, and then, β-galactsidase activity in each of the cells was measured using Gal-Screen System (Applied Biosystems) according to the accompanying protocol.

Hamster anti-mouse Fas antibody (PK-8; Medical and Biological Laboratories) that has the activity to induce Fas molecule association or rat anti-mouse Fas antibody (RMF6; Medical and Biological Laboratories) that does not have the activity was used as the anti-Fas antibody. On the other hand, hamster antibody or rat antibody that does not react with mFas was used as the negative control antibody.

The results are shown in Fig. 14

As a result, no change in β-galactosidase activity was detected when recombinant cells that coexpress mFas-MERMUC-β-galΔω and mFas-MERMUC-β-galΔα were treated with anti-Fas antibody lacking Fas molecule association inducing activity. However, the treatment with anti-Fas antibody that has Fas molecule association inducing ability increased the β-galactosidase activity in a concentration-dependent manner.

These results indicate that, using the recombinant cells that coexpress mFas-MERMUC-β-galΔω and mFas-MERMUC-β-galΔα produced in the present Example, the intracellular association of MERMUC region can be freely regulated by regulating the association of extracellular Fas protein region with anti-Fas antibody.

### <11-2> Analysis of the effect of MERMUC self-association on binding affinity of MERMUC and HGF receptor

The effect of self-association of MERMUC molecules on the binding affinity of MERMUC and HGF receptor c-Met was analyzed by immunoprecipitation of MERMUC and HGF receptor (immunocoprecipitation) using the recombinant cells produced above.

mFas-MERMUC expressing recombinant cells were prepared by transfecting HEK293 cells with mFas-MERMUC expression plasmid produced above. The cells were treated with each of the above-described anti-Fas antibody and then cell lysate was prepared by lysing cells according to conventional method. Commercially available anti-HGF receptor c-Met polyclonal antibody h-Met (C-28) (sc-161; Santa Cruz Biotechnology) described above was added to the cell lysate, reacted with protein-G Sepharose, and then HGF receptor protein/anti-c-Met antibody complex and protein components bound to the complex were precipitated by centrifugation.

The precipitate was dissolved in electrophoresis buffer, separated by SDS-PAGE, and then subjected to Western blotting according to convention method using anti-mouse Fas antibody (R&D) to evaluate the amount of coprecipitated mFas-MERMUC protein.

The results are shown in Fig. 15.

The results indicate significant increase of coimmunoprecipitated mFas-MERUMUC in cells treated with anti-Fas antibody (RK-8) that possesses Fas molecule association inducing activity but not in cells treated with anti-Fas antibody (RMF6) lacking the association inducing activity or control antibody (non-reactive to mouse Fas; described above).

These results showed that the self-association (within the cytoplasmic region) of MERMUC promoted the binding ability of MERMUC with HGF receptor.

### [Example 12] Analysis of decrease in binding of MERMUC with HGF receptor by suppression of MERMUC self-association

As described in the previous Example, the self-association ability of MERMUC is maintained even by the deletion of the C-terminal 53 amino acids. Therefore, the responsible region for MERMUC self-association was predicted to exist in the N-terminal side of the HGF receptor-binding region (the C-terminal 53 amino acid residues of MERMUC) of MERMUC.

To validate this prediction and at the same time synthesize a MERMUCMERMUC peptide that is able to suppress MERMUC self-association through the binding to the MERMUC self-association region, the following 4 types of peptides each corresponding to a part of MERMUC cytoplasmic region were synthesized by the method described later:
del-1: amino acid sequence from amino acids 244 to 345 of SEQ ID NO: 10;
del-2: amino acid sequence from amino acids 244 to 380 of SEQ ID NO: 10;
del-3: amino acid sequence from amino acids 244 to 415 of SEQ ID NO: 10; and
del-4: amino acid sequence from amino acids 244 to 450 of SEQ ID NO: 10.

cDNAs encoding each of the human MERMUC cytoplasmic region fragments (del-1 to del-4) were prepared using full-length human MERMUC cDNA (SEQ ID NO: 9) as a template and a primer set as follows:
(1) del-1
   forward primer: SEQ ID NO: 53 and
   reverse primer: SEQ ID NO: 54;
(2) del-2
   forward primer: SEQ ID NO: 53 and
   reverse primer: SEQ ID NO: 55;
(3) del-3
   forward primer: SEQ ID NO: 53 and
   reverse primer: SEQ ID NO: 56; and
(4) del-4
   forward primer: SEQ ID NO: 53 and
   reverse primer: SEQ ID NO: 57.

Furthermore, ATG corresponding to a methionine codon and a HindIII site to the 5' side thereto was added at the 5' side of the forward primer (SEQ ID NO: 53). Moreover, TTA corresponding to the antisense of a stop codon and a *Xho*I site to the 5' side thereto was added at the 5' side of each reverse primer.

Each of the cDNA obtained by PCR was digested with *Hind*III and *Xho*I and inserted into the *Hind*III/*Xho*I site of pcDNA3 vector to obtain an expression vector expressing each of the human MERMUC cytoplasmic region fragments (del-1 to del-4).

Next, the following combination of recombinant protein expression vectors were added to CHO-K1 cells and cultured at 37°C for 48 hr for cotransfection using GeneJammer Transfection Reagent (STRATAGENE):
1) vector expressing MERMUC (full)/β-galΔω (Example 8)),
   vector expressing c-Met (C91)/β-galΔα (Example 8) , and
   vector expressing any one of del-1 to del-4 (described above); or
2) vector expressing MERMUC (full)/β-galΔω (Example 8)),
   vector expressing MERMUC (full)/β-galΔα (Example 8), and
   vector expressing any one of del-1 to del-4 (described above).

Intracellular β-Galactosidase activity of the recombinant cells was measured using Gal-Screen System (Applied Biosystems) according to the accompanying protocol 48 hr after plasmid transfection.

As a control, recombinant cells transfected with pcDNA3 vector (called "mock") instead of vectors expressing one of del-1 to del-4 were used in above (1) and (2), respectively.

The results are shown in Fig. 16 to Fig. 18.

As shown in Fig 16, del-4 showed the strongest suppression on MERMUC self-association. Furthermore, the greatest decrease in the binding of MERMUC and HGF receptor c-Met was achieved by del-4 as shown in Fig. 17. Moreover, the suppression of MERMUC self-association and the suppression of the binding of MERMUC and HGF receptor c-Met by del-4 both increased in del-4 dosage-dependent manner.

### [Example 13] Analysis of recovery of MAP kinase activation due to suppression of binding of MERMUC and HGF receptor

As described in the previous Example, HGF receptor mediated MAP kinase activation due to HGF stimulation was decreased by the binding of MERMUC to the HGF receptor.

Therefore, it was examined whether the decrease in MAP kinase activity is suppressed by the MERMUC cytoplasmic region fragment del-4 described above.

The del-4 protein expression vector produced above was added to Tet-Off/MERMUC expressing recombinant cells (MERMUC/293 cells) (produced in Example 2) and cultured at 37°C for 48 hr for transfection using GeneJammer Transfection Reagent (STRATAGENE) according to the accompanying protocol.

Cells were similarly transfected with pcDNA3 vector [mock] that lack the cDNA encoding del-4 instead of the del-4 expression vector as a control.

The cells were stimulated with human recombinant HGF (CALBIOCHEM) , and cell lysate was prepared according to conventional method immediately after the stimulation to evaluate the activation level of MAP kinase (also referred to as Erk).

The cell lysate was separated by SDS-PAGE, and then subjected to Western blotting according to conventional method using anti-phosphorylated MAP kinase polyclonal antibody (Phospho-p44/42 MAP kinase (Thr202/Thr204) antibody; Cell Signaling Technology) to determine the MAP kinase activation level based on the obtained signal intensity.

The results are shown in Fig. 19.

The results showed, as expected, decrease in the phosphorylated MAP kinase level after HGF stimulation due to the expression of MERMUC protein in control cells transfected with the mock vector (pcDNA3). However, the suppression was removed in the del-4 expressing cells and a significant level of phosphorylated MAP kinase was observed.

These results indicate a close relationship between MERMUC self-association (multimerization) and the binding ability of MERMUC with the HGF receptor. Furthermore, it was suggested that the increase in MERMUC self-association enhances the binding ability (binding affinity) of MERMUC with the HGF receptor, and as a result, enhances the ability of HGF to suppress the HGF receptor mediated MAP kinase activity.

### [Example 14] Analysis of induction of MERMUC protein phosphorylation by HGF stimulation

The intracellular signal transduction via the HGF receptor after HGF stimulation is modulated through the binding of MERMUC to the HGF receptor c-Met. Therefore, it was predicted that MERMUC itself was also receive some kind of modification/regulation (for example, feedback regulation) downstream of HGF/HGF receptor signaling.

The human MERMUC cytoplasmic region contains many amino acid residues, i.e., 45, 37 and 1 threonine, serine and tyrosine residues, respectively, that may be phosphorylated. Therefore, actual phosphorylation of the residues was analyzed as described below.

After labeling the Tet-Off/MERMUC expressing recombinant cells (MERMUC/293 cells) produced above with [³²P] orthophosphate, the cells were stimulated with human recombinant HGF (20ng/ml; 5 min). Next, cell lysate was prepared by lysing cells according to conventional method. Anti-human MERMUC polyclonal antibody prepared as above (Example 2) was added to and reacted with the obtained cell lysate, bound to protein-G Sepharose, and then MERMUC protein and anti-MERMUC polyclonal antibody complex was precipitated by centrifugation.

The precipitate was extracted with 1% SDS and similar immunoprecipitation procedure was performed to reduce the background. The sample prepared by this procedure was separated by SDS-PAGE and analyzed by autoradiography.

Furthermore, MERMUC expression in the above mentioned recombinant cells was confirmed to be constant regardless of the presence or absence of HGF by Western blotting according to conventional method using anti-human MERMUC polyclonal antibody.

The results are shown in Fig 20.

As a result, a strong signal band indicating phosphorylated MERMUC protein was detected in cells wherein MERMUC was overexpressed by cultivation without Dox via HGF stimulation.

The following experiments were conducted similarly as described above:
1) epidermal growth factor (EGF) was used in place of HGF;
2) MERMUC expressing cells were treated with HGF signaling suppressor, PI3 kinase inhibitor (LY294002; 10 µM; CALBIOCHEM), before HGF stimulation;
3) MERMUC expressing cells were treated with HGF signaling suppressor, MEK 1/2 inhibitor (U0126; 10 µM; CALBIOCHEM) , before HGF stimulation;
4) MERMUC expressing cells were treated with PI3 kinase inhibitor (LY294002; 10 µM) before EGF stimulation; and
5) MERMUC expressing cells were treated with MEK 1/2 inhibitor (U0126; 10 µM) before EGF stimulation.

The results are shown in Fig. 21.

As a result, other growth factors such as EGF did not show effect to increase phosphorylation of MERMUC. Furthermore, the treatment of cells with PI3 kinase inhibitor and MEK 1/2 inhibitor revealed that the enhancement of MERMUC phosphorylation is suppressed only in the cells treated with PI3 kinase inhibitor.

Thus, MERMUC phosphorylation was supposed to be mediated by the PI3 kinase and Akt kinase pathway.

### [Example 13] Analysis of suppression on HGF-dependent liver enlargement in MERMUC overexpressing transgenic mice

It has been reported that HGF is transiently overexpressed *in vivo* by the injection of HGF expression plasmid DNA into the tail vein of normal mice, causing liver enlargement due to the enhancement of hepatocyte proliferation (Hepatology, 2001, Vol. 33, p848-859).

Therefore, to validate the down-regulation activity of MERMUC on HGF receptor mediated signaling, the method in the above mentioned report was applied to MERMUC overexpressing transgenic mice (MERMUC-Tg; 7 weeks old; male; 23-33 g) produced in the previous Example, and the effect due to the expression of MERMUC on liver enlargement induced by HGF overexpression was examined.

Human HGF expression plasmid (human HGF-cDNA in pcDNA3; Invitrogen) or plasmid pcDNA3 (mock) was injected into the tail vein of each of MERMUC-Tg mouse and littermate non-Tg mouse at a concentration of 28 µg/mouse (2.3 ml saline) within 5 to 10 seconds. The HGF level in serum of each of the mice was measured using a human HGF ELISA kit (Genzyme Techne) two days after the plasmid injection. The level of HGF production due to the administered plasmid in each animal was evaluated together with the changes in liver weight upon laparotomy.

The results are shown in Fig 22.

As a result, the ratio of liver weight to body weight increased about 1.6 times due to the transient overexpression of HGF in non-Tg mice. However, the increase was suppressed to about 1.2 times in MERMUC-Tg mice. That is, significant difference in HGF dependent-liver enlargement was observed between Tg/non-Tg mice.

The findings hitherto forecast HGF-dependent liver enlargement to result from overproliferation of hepatocytes in liver tissue. Big contribution of the activation of the MAP kinase pathway among the HGF-signaling pathways is predicted. The results of the present examination indicate that, also *in vivo*, MERMUC binds to the HGF receptor causing a decrease in MAP kinase activation after HGF stimulation.

### Industrial Applicability

The pharmaceutical compositions of the present invention eliminate down-regulation of HGF activity caused by the binding of c-Met and MERMUC (including multimerization of MERMUC molecules). Thus, the maximum potential biological activity of endogenous HGF or HGF (as HGF protein drug or by HGF gene therapy) exogenously supplied to the patient can be triggered.

Therefore, the use of the pharmaceutical compositions of the present invention alone or in combination with an HGF drug in disease treatment attains repair, regeneration or neogenesis of tissues and organs that is required for the prevention and treatment of various diseases. And such effect can be achieved with the required minimum dose of HGF drug (HGF protein, HGF gene, etc.). More specifically, various intractable diseases (for example, hepatic fibrosis, hepatic cirrhosis, fulminant hepatitis, viral hepatitis, acute hepatitis, chronic renal failure, renal fibrosis, pulmonary fibrosis, etc.) that are hard to treat can be treated with the present pharmaceutical compositions.

Moreover, when the pharmaceutical compositions of the present invention are applied together with an HGF drug, the dose, frequency of administration, administration period and such of the HGF drug administrated to a patient can be reduced. Accordingly, the patient's physical and mental pain can be reduced while reducing various expenses (costs borne by each of patients, medical institutes, drug makers and government agencies).

The present invention further provides methods for identifying active ingredients of the pharmaceutical compositions: (1) substances having activity to inhibit the binding between c-Met and MERMUC; (2) substances having activity to inhibit multimerization (self-association) of MERMUC molecules; or (3) substances that suppress or inhibit the expression of MERMUC. Furthermore, the present invention provides methods of testing an arbitrary substance for various activities (activity to inhibit the binding between c-Met and MERMUC, activity to inhibit multimerization of MERMUC, activity to suppress or inhibit the expression of MERMUC, and such). In addition, the present invention provides various tools (various polypeptides, fusion polypeptides, etc.) used for these methods. Using these methods and tools, the pharmaceutical compositions of the present invention can be readily discovered.

## Claims

1. A pharmaceutical composition used for maintaining or enhancing cell proliferation, or formation, repair, regeneration or neogenesis of tissues, organs, blood vessels, mucosa, skeleton or nerves, which comprises a substance having activity to inhibit the binding between c-Met and MERMUC or multimerization of MERMUC, and a pharmaceutically acceptable carrier.

2. A pharmaceutical composition used for maintaining or enhancing cell proliferation, or formation, repair, regeneration or neogenesis of tissues, organs, blood vessels, mucosa, skeleton or nerves due to HGF, which comprises a substance having activity to inhibit the binding between c-Met and MERMUC or multimerization of MERMUC, and a pharmaceutically acceptable carrier.

3. The pharmaceutical composition of claim 2, wherein said HGF is an endogenous HGF of an organism.

4. The pharmaceutical composition of claim 2, wherein said HGF is an HGF drug that exogenously provides HGF to an organism.

5. The pharmaceutical composition of claim 4, wherein the HGF drug is a protein drug comprising an HGF protein.

6. The pharmaceutical composition of claim 4, wherein the HGF drug is a DNA drug comprising DNA encoding an HGF protein.

7. The pharmaceutical composition of claim 1 or 2 , which is used for preventing or treating fibrosis.

8. The pharmaceutical composition of claim 7, wherein the fibrosis is hepatic, renal or pulmonary fibrosis.

9. The pharmaceutical composition of claim 1 or 2, which is used for preventing or treating hepatic cirrhosis, hepatic fibrosis or hepatitis.

10. the pharmaceutical composition of claim 1 or 2, wherein hepatitis is used for preventing or treating fulminant hepatitis, acute hepatitis or viral hepatitis.

11. The pharmaceutical composition of claim 1 or 2, which is used for preventing or treating chronic renal failure.

12. The pharmaceutical composition of claim 1 or 2, which is used for preventing or treating arteriosclerosis obliterans.

13. The pharmaceutical composition of claim 1 or 2, wherein the substance has activity to inhibit the binding between c-Met and MERMUC.

14. The pharmaceutical composition of claim 13, wherein the substance has activity to inhibit the binding between the C-terminal region of the c-Met β chain and the C-terminal region of MERMUC.

15. The pharmaceutical composition of claim 1 or 2, wherein the substance has activity to inhibit multimerization of MERMUC.

16. The pharmaceutical composition of claim 1 or 2, wherein the substance binds to MERMUC.

17. A pharmaceutical composition used for maintaining or enhancing cell proliferation, or formation, repair, regeneration or neogenesis of tissues, organs, blood vessels, mucosa, skeleton or nerves, which comprises a substance that suppresses or inhibits the expression of MERUMUC and a pharmaceutically acceptable carrier.

18. The pharmaceutical composition of claim 17, wherein the substance inhibits the transcription of a gene encoding MERMUC to mRNA.

19. The pharmaceutical composition of claim 17, wherein the substance inhibits the translation of an mRNA encoding MERMUC to protein.

20. A pharmaceutical composition used for maintaining or enhancing cell proliferation, or formation, repair, regeneration or neogenesis of tissues, organs, blood vessels, mucosa, skeleton or nerves due to HGF, which comprises a substance that suppresses or inhibits the expression of MERUMUC and a pharmaceutically acceptable carrier.

21. The pharmaceutical composition of claim 20, wherein the substance inhibits the transcription of a gene encoding MERMUC to mRNA.

22. The pharmaceutical composition of claim 20, wherein the substance inhibits the translation of an mRNA encoding MERMUC to protein.

23. The pharmaceutical composition of claim 20, wherein said HGF is an endogenous HGF of an organism.

24. The pharmaceutical composition of claim 20, wherein said HGF is an HGF drug that can exogenously provide HGF to an organism.

25. The pharmaceutical composition of claim 24, wherein the HGF drug is a protein drug comprising an HGF protein.

26. The pharmaceutical composition of claim 24, wherein the HGF drug is a DNA drug comprising DNA encoding an HGF protein.

27. The pharmaceutical composition of claim 17 or 20, which is used for preventing or treating fibrosis.

28. The pharmaceutical composition of claim 27, wherein the fibrosis is hepatic, renal or pulmonary fibrosis.

29. The pharmaceutical composition of claim 17 or 20, which is used for preventing or treating hepatic cirrhosis, hepatic fibrosis or hepatitis.

30. the pharmaceutical composition of claim 17 or 20, wherein hepatitis is used for preventing or treating fulminant hepatitis, acute hepatitis or viral hepatitis.

31. The pharmaceutical composition of claim 17 or 20, which is used for preventing or treating chronic renal failure.

32. The pharmaceutical composition of claim 17 or 20, which is used for preventing or treating arteriosclerosis obliterans.

33. A polypeptide comprising a partial amino acid sequence of the full-length amino acid sequence of MERMUC, and having activity to bind c-Met.

34. The polypeptide of claim 33, wherein said partial amino acid sequence corresponds to whole or a portion of a cytoplasmic region of MERMUC.

35. The polypeptide of claim 34, wherein said whole or a portion of a cytoplasmic region comprises whole or a portion of a leucine repeat region in the C-terminal region of MERMUC.

36. The polypeptide of claim 33, wherein said MERMUC is human MERMUC.

37. The polypeptide of claim 33, which comprises the amino acid sequence of SEQ ID NO: 15.

38. A polypeptide comprising a partial amino acid sequence of the full-length amino acid sequence of c-Met, and having activity to bind MERMUC.

39. The polypeptide of claim 38, wherein said partial amino acid sequence corresponds to whole or a portion of a cytoplasmic region of the c-Metβ chain.

40. The polypeptide of claim 39, wherein said whole or a portion of a cytoplasmic region comprises whole or a portion of a C-terminal region of c-Met comprising one or more tyrosine residues that undergo self-phosphorylation.

41. The polypeptide of claim 38, wherein said c-Met is human c-Met.

42. The polypeptide of claim 38, which comprises the amino acid sequence of SEQ ID NO: 5.

43. A polypeptide comprising a partial amino acid sequence of the full-length amino acid sequence of MERMUC, and comprising the activity to bind MERMUC.

44. The polypeptide of claim 43, wherein said partial amino acid sequence corresponds to whole or a portion of a cytoplasmic region of MERMUC.

45. The polypeptide of claim 33, wherein said MERMUC is human MERMUC.

46. A fusion polypeptide represented by the general formula W-X-Y (wherein, W is absent, or the full-length amino acid sequence or a portion of a protein other than MERMUC; X is the full-length amino acid sequence of MERMUC or a portion thereof; and Y is the full-length amino acid sequence or a portion of a protein other than MERMUC).

47. The fusion polypeptide of claim 46, wherein the protein Y is β-galΔα or β-galΔω.

48. The fusion polypeptide of claim 47, wherein said β-galΔα comprises the amino acid sequence of SEQ ID NO: 16.

49. The fusion polypeptide of claim 47, wherein said β-galΔω comprises the amino acid sequence of SEQ ID NO: 17.

50. The fusion polypeptide of claim 46, wherein said portion of the full-length amino acid sequence of MERMUC has activity to bind c-Met.

51. The fusion polypeptide of claim 46, wherein said portion of the full-length amino acid sequence of MERMUC comprises whole or a portion of a cytoplasmic region of MERMUC.

52. The fusion polypeptide of claim 51, wherein said portion of the full-length amino acid sequence of MERMUC comprises whole or a portion of a leucine repeat region of the C-terminal region of MERMUC.

53. The fusion polypeptide of claim 46, wherein said MERMUC is human MERMUC.

54. The polypeptide of claim 46, wherein said X comprises the amino acid sequence of SEQ ID NO: 15.

55. The polypeptide of claim 46, wherein the protein W comprises the extracellular region of Fas.

56. A fusion polypeptide represented by the general formula Z-Y (wherein Z is the full-length amino acid sequence of c-Met or a portion thereof; and Y is the full-length amino acid sequence or a portion of a protein other than c-Met).

57. The fusion polypeptide of claim 52, wherein said protein is β-galΔα or β-galΔω.

58. The fusion polypeptide of claim 57, wherein said β-galΔα comprises the amino acid sequence of SEQ ID NO: 16.

59. The fusion polypeptide of claim 57, wherein said β-galΔω comprises the amino acid sequence of SEQ ID NO: 17.

60. The fusion polypeptide of claim 56, wherein said portion of the full-length amino acid sequence of c-Met has activity to bind MERMUC.

61. The fusion polypeptide of claim 56, wherein said portion of the full-length amino acid sequence of c-Met comprises whole or a portion of a cytoplasmic region of the c-Met β chain.

62. The fusion polypeptide of claim 56, wherein said portion of the full-length amino acid sequence of c-Met comprises whole or a portion of a C-terminal region of c-Met comprising one or more tyrosine residues that undergo self-phosphorylation.

63. The fusion polypeptide of claim 56, wherein said c-Met is human c-Met.

64. The fusion polypeptide of claim 56, wherein said Z comprises the amino acid sequence of SEQ ID NO: 5.

65. A method of testing a substance for the presence or extent of activity to inhibit the binding between c-Met and MERMUC, which comprises the step of comparing the presence or extent of the binding between c-Met and MERMUC in the presence of a test substance to the extent of the binding between c-Met and MERMUC in the absence of the test substance.

66. A method of identifying a substance that has activity to inhibit the binding between c-Met and MERMUC, which comprises the step of comparing the presence or extent of the binding between c-Met and MERMUC in the presence of a test substance to the extent of the binding between c-Met and MERMUC in the absence of the test substance.

67. A method of testing for the presence or extent of activity of a substance to inhibit the binding between c-Met and MERMUC, which comprises the steps of:
(a) preparing a cell that is designed to coexpress c-Met and MERMUC, and allow detection of the binding between said c-Met and MERMUC;
(b) culturing the cell obtained in step (a) in the presence of HGF while in the absence of test substance, and then measuring the extent of the binding between c-Met and MERMUC in the cell;
(c) culturing the cell obtained in step (b) in the presence of HGF and a test substance, and then measuring the extent of the binding between c-Met and MERMUC in the cell; and
(d) comparing the measurement result of step (b) with that of step (c).

68. The method of claim 67, wherein said MERMUC is the fusion polypeptide of any one of claims 46 to 54.

69. The method of claim 67, wherein said MERMUC is the fusion polypeptide of any one of claims 47 to 49.

70. The method of claim 67, wherein said c-Met is the fusion polypeptide of any one of claims 56 to 64.

71. The method of claim 67, wherein said c-Met is the fusion polypeptide of any one of claims 57 to 59.

72. A method of identifying a substance that has activity to inhibit the binding between c-Met and MERMUC, which comprises the steps of:
(a) preparing a cell that is designed to coexpress c-Met and MERMUC, and allow detection of the binding between said c-Met and MERMUC bind;
(b) culturing the cell obtained in step (a) in the presence of HGF while in the absence of test substance, and then measuring the extent of the binding between c-Met and MERMUC in the cell;
(c) culturing the cell obtained in step (b) in the presence of HGF and a test substance, and then measuring the extent of the binding between c-Met and MERMUC in the cell; and
(d) comparing the measurement result of step (b) with that of step (c).

73. The method of claim 72, wherein said MERMUC is the fusion polypeptide of any one of claims 46 to 54.

74. The method of claim 72, wherein said MERMUC is the fusion polypeptide of any one of claims 47 to 49.

75. The method of claim 72, wherein said c-Met is the fusion polypeptide of any one of claims 56 to 64.

76. The method of claim 72, wherein said c-Met is the fusion polypeptide of any one of claims 57 to 59.

77. A method of testing a substance for the presence or extent of activity to inhibit multimerization of MERMUC, which comprises the step of comparing the presence or extent of multimerization of MERMUC in the presence of a test substance to the extent of multimerization of MERMUC in the absence of the test substance.

78. A method for identifying a substance having activity to inhibit multimerization of MERMUC, which comprises the step of comparing the presence or extent of multimerization of MERMUC in the presence of a test compound to the extent of multimerization of MERMUC in the absence of the test substance.
